# EUROPEAN PATENT SPECIFICATION

(11) **EP 1 277 763 B1**
(45) Date of publication and mention of the grant of the patent: **20.12.2006**
(21) Application number: 02014868.0
(22) Date of filing: 04.07.2002
(51) Int. Cl.: C07K 14/32, C12N 15/31, C12N 1/21, A01N 63/00, C12R 1/07

(54) **Polypeptides having larvae growth inhibiting or insecticidal effect on scarabaeidae insects and polynucleotides encoding the same**
Polypeptide die das larvale Wachstum hemmen oder insektizider Wirkung auf scarabaeidae Insekten haben und dafür kodierende Polynukleotide
Polypeptides inhibitant la croissance larvale ou ayant un effet insecticide sur les insectes scarabaeidae et polynucléotides codant lesdites

(30) Priority: 04.07.2001 JP 2001203463
(43) Date of publication of application: 22.01.2003
(73) Proprietor: DAINIPPON INK AND CHEMICALS, INC., Itabashi-ku, Tokyo 174-8520 (JP)
(72) Inventor: Tanaka, Masao, Chiba-shi, Chiba (JP); Yokoyama, Tomoko, Chiba-shi, Chiba (JP); Aoyagi, Moriichi, Katori-gun, Chiba (JP); Hasegawa, Makoto, Chiba-shi, Chiba (JP); Ehara, Gaku, Sakura-shi, Chiba (JP); Kimura, Masaharu, Ichihara-shi, Chiba (JP); Nishihashi, Hideji, Sakura-shi, Chiba (JP)
(74) Representative: Hiebl, Inge Elisabeth

(56) References cited:
- WO-A-97/14798
- JP-A- 11 332 556
- US-A- 4 824 671
- US-A- 5 554 534
- DATABASE EMBL28 January 1997 (1997-01-28) FONCERRADA L. ET AL.: "Antiscarab pest toxin 50C(a)" Database accession no. AAW06418 XP002199539
- DATABASE EMBL28 January 1997 (1997-01-28) FONCERRADA L. ET AL.: "Antiscarab pest toxin 50C(a)" Database accession no. AAT43222 XP002201220
- DATABASE EMBL28 January 1997 (1997-01-28) FONCERRADA L. ET AL.: "Antiscarab pest toxin 50C(b)" Database accession no. AAW06417 XP002199540
- DATABASE EMBL28 January 1997 (1997-01-28) FONCERRADA L. ET AL.: "Antiscarab pest toxin 50C(b)" Database accession no. AAT43221 XP002201221

## Description

### Background of the Invention

### Field of the Invention

The present invention relates to polypeptides having larvae growth inhibiting or insecticidal effect on Scarabaeidae insects, to polynucleotides encoding the same, to controlling agents for controlling Scarabaeidae insects containing the polypeptides as active ingredients, and to a method of controlling Scarabaeidae insects using the polypeptides.

### Description of the Related Art

Larvae of Scarabaeidae insects eat roots of a wide variety of plants such as turf, agricultural crops, horticultural crops and trees and bushes and cause serious damage thereto. A large number of reports have been made on damages caused by, especially, *Anomala cuprea, Blitopertha orientalis,* and *Popillia japonica* to a lawn of golf courses, a sweet potato field, and a peanut field. In particular, *Anomala cuprea,* which is particularly large in size, does considerable harm thereto due to its eager appetite.

However, conventional ground spreading of chemical pesticides is difficult to give a controlling effect since these larvae inhabit in the ground and are difficult to locate them, and thus it was necessary to spread a large amount of chemical pesticides over a wide range of the surface of the ground and penetrate them into the ground. Therefore, there were concerns about adverse influences of the chemical pesticides on the natural environment and human bodies, and there has been a keen desire for a biological controlling method that can replace the chemical pesticides.

As one of the biological controlling methods for controlling Scarabaeidae insects, attempts to utilize bacteria belonging to *Bacillus popilliae* have been tried over a long period of time. The bacteria are parasitic to larvae of Scarabaeidae insects as hosts and infect to them per os usually in a form of sporangia, propagate in large amounts in hemolymph to cause milky disease and finally lead the larvae to death.

Recently, an amino acid sequence of a polypeptide constituting a parasporal body of a strain belonging to *Bacillus popilliae*, i.e., *Bacillus popilliae* subsp. *melolonthae* H1, and a nucleotide sequence of the gene encoding the polypeptide were partly clarified (J. Bacteriol. Vol. 179, p.4336-4341 (1997)), and it has been reported that the polypeptide constituting the parasporal body of *Bacillus popilliae* subsp. *melolonthae* H1 has a controlling effect on *Melolontha melolontha* (WO97/14798). However, it was not clarified whether the bacterial strain and a polypeptide thereof have controlling effects on *Anomala cuprea*, *Blitopertha orientalis* and *Popillia japonica,* each of which belongs to different species.

In addition, US patent 5,554,534 relates to an isolated polynucleotide encoding a toxin which is active against scarab pests, said polynucleotide encoding the amino acid sequence of SEQ ID NO: 4 thereof or a fragment thereof sufficient to retain anti-scarab activity.

### Summary of the Invention

An object to be solved according to the present invention is to provide polypeptides having a larvae growth inhibiting effect or an insecticidal effect on Scarabaeidae insects, in particular *Anomala cuprea, Blitopertha orientalis* and *Popillia japonica*, transformants having introduced therein a polynucleotide encoding the polypeptide, controlling agents for Scarabaeidae insects containing the polypeptides as active ingredients, controlling agents for Scarabaeidae insects using the polypeptide and sporangia of *Bacillus popilliae,* and a method of controlling Scarabaeidae insects using the controlling agents.

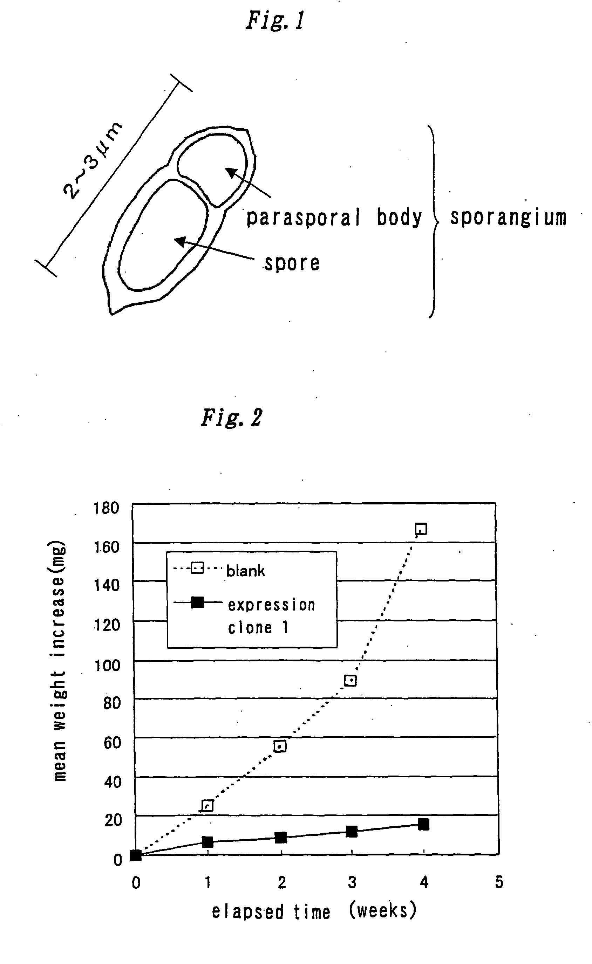

The inventors of the present invention have found that the sporangia of certain strains belonging to *Bacillus popilliae* have a larvae growth inhibiting effect or an insecticidal effect. Subsequently, they have taken out the parasporal body from sporangia of *Bacillus popilliae* (Fig. 1) containing a spore and a parasporal body, performed screening using an antibody to the polypeptide constituting the parasporal body to thereby isolate a polynucleotide encoding the polypeptide and determined its nucleotide sequence and amino acid sequence. A transformant having introduced therein the polynucleotide by using a vector enabled mass production of the above-mentioned polypeptide. Further, the inventors of the present invention have made it clear that the polypeptide produced by the transformant has a controlling effect on *Anomala cuprea* and further that use of the polypeptide in combination with the sporangia increases the controlling effect. Thus, the present invention was accomplished.

Therefore, according to the present invention, there is provided (1) a polypeptide having the amino acid sequence of SEQ ID NO:2, or the amino acid sequence including substitution, deletion or insertion of 9 or less amino acid residues and having a larvae growth inhibiting or insecticidal effect on a Scarabaeidae insect.

According to the present invention, there is provided (2) a polypeptide having the amino acid sequence of SEQ ID NO:4, 6, 18 or 20, or the amino acid sequence including substitution, deletion or insertion of 50 or less amino acid residues and having a larvae growth inhibiting or insecticidal effect on a Scarabaeidae insect.

Further, the present invention provides polynucleotides encoding the polypeptides.

Still further, the present invention provides vectors comprising the polynucleotides and transformants which have introduced the polynucleotides.

Further, the present invention provides controlling agents for controlling a Scarabaeidae insect containing the polypeptides as active ingredients.

Further, the present invention provides controlling agents for controlling a Scarabaeidae insect comprising the polypeptides and sporangia of bacteria belonging to *Bacillus popilliae* as active ingredients.

Still further, the present invention provides a method of controlling a Scarabaeidae insect, in which the Scarabaeidae insect is subjected to an action of the controlling agent for controlling a Scarabaeidae insect.

In the present invention, the term "polypeptide" includes peptides consisting of a plurality of amino acids linked through peptide bonds and polypeptides to which other molecules such as glycoproteins are bonded as well as proteins in a form of multimers, etc.

According to the present invention, polypeptides having a larvae growth inhibiting or an insecticidal effect on Scarabaeidae insects, in particular *Anomala cuprea, Blitopertha orientalis* and *Popilliae japonica,* transformants having introduced therein a polynucleotide encoding the polypeptide, a controlling agent for controlling a Scarabaeidae insect comprising the polypeptide as an active ingredient, and a method of controlling a Scarabaeidae insect using the polypeptide and sporangia of *Bacillus popilliae* as active ingredients.

### Brief Explanation of the Drawings

Fig. 1 is a schematic diagram of a sporangium containing a spore and a parasporal body;
Fig. 2 is a diagram illustrating a larvae growth inhibiting effect of a polypeptide produced by the polynucleotide on a Scarabaeidae insect (larva of *Anomala cuprea*) performed in Example 4;
Fig. 3 is a diagram illustrating an insecticidal effect of biological tests performed in Example 5;
Fig. 4 is a diagram illustrating the larvae growth inhibiting effect of the biological tests performed in Example 5;
Fig. 5 shows alignment of amino acid sequences in Example 11;
Fig. 6 shows alignment of amino acid sequences in Example 11;
Fig. 7 shows alignment of amino acid sequences in Example 11;
Fig. 8 is a diagram illustrating a larvae growth inhibiting effect on a Scarabaeidae insect of a polypeptide produced by a polynucleotide in Example 13 of the present invention (larvae of *Anomala cuprea*).

### Detailed Description of the Invention

Hereinafter, the present invention will be described in detail.

A parasporal body contained in sporangia of a bacterium belonging to *Bacillus popilliae* is a proteinaceous aggregate (Hukuhara, 'Toshihiko, "Insect Pathology", p. 57, 1979), which comprises one kind or plural kinds of polypeptides. The polypeptide of the present invention is one or more polypeptides that constitute the parasporal body in the sporangium of *Bacillus popilliae samadara* or the like that will be described below. However, the polypeptide of the present invention is different in the amino acid sequence from a polypeptide that constitutes a parasporal body described in WO97/14798, and hence the polypeptide and polynucleotide of the present invention are considered to be novel.

According to a first aspect of the present invention, there is provided a polypeptide having the amino acid sequence of SEQ ID NO: 2 and having a larvae growth inhibiting effect or an insecticidal effect on a Scarabaeidae insect.

According to a second aspect of the present invention, there is provided a polypeptide having the amino acid sequence of SEQ ID NO: 4, 6, 18 or 20 and having a larvae growth inhibiting effect or an insecticidal effect on a Scarabaeidae insect.

Besides, there is disclosed a polypeptide having the amino acid sequence of SEQ ID NO: 8 or 10 and having a larvae growth inhibiting effect or an insecticidal effect on a Scarabaeidae insect.

Note that the amino acid sequence of SEQ ID NO: 2 and the nucleotide sequence of SEQ ID NO: 1 are sequences found in the amino acid sequences of SEQ ID NO: 4, 6, 18 or 20 and the nucleotide sequences of SEQ ID NO: 3, 5, 17 or 19 with homology of 95% or more and is considered to be specific to the parasporal body of *Bacillus popilliae* (cf. Example 11).

The above-mentioned polypeptide of the present invention may include polypeptides containing substitution, deletion or insertion of one or several numbers of amino acid residues as far as the polypeptides have a larvae growth inhibiting effect or an insecticidal effect on a Scarabaeidae insect. Here, the term "several numbers of" means, specifically, a number of 9 or less as regards a polypeptide having the amino acid sequence of SEQ ID NO:2, and 50 or less as regards a polypeptide having the amino acid sequence of SEQ ID NO: 4, 6, 18 or 20, although it may vary depending on the position and kinds of amino acid residues in a three-dimensional structure of the polypeptide. These polypeptides including substitution, deletion or insertion of one or several numbers of amino acid residues can be obtained, for example, by introducing a mutation to a polynucleotide encoding the polypeptide by site-specific mutatagenesis and performing transcription and translation of the polynucleotide. These polypeptides of the present invention may be fused polypeptides with other polypeptides. The polypeptides of the present invention may be polypeptides that have the amino acid sequence having homology of 50% or more, preferably 70% or more, and more preferably 90% or more, with the above-mentioned amino acid sequence as far as the polypeptides have a larvae growth inhibiting effect or an insecticidal effect on a Scarabaeidae insect. Further, the polypeptides of the present invention may be polypeptides that have a part of the amino acid sequence of SEQ ID NO: 4, 6, 18 or 20 as far as the polypeptides have a larvae growth inhibiting effect or an insecticidal effect on a Scarabaeidae insect.

The polynucleotides of the present invention are polynucleotides that encode the above-mentioned polypeptides of the present invention. Codons of each of amino acid residues are not particularly limited as far as the amino acid sequence encoded is the same. Specific examples of the polynucleotide of the present invention include a polynucleotide comprising the nucleotide sequence of SEQ ID NO: 1, a polynucleotide having the nucleotide sequence of the coding region of SEQ ID NO: 3, 5, 17 or 19. Also disclosed is a polynucleotide having the nucleotide sequence of SEQ ID NO: 7 or 9.

The polynucleotide of the present invention may be a polynucleotide that is hybridizable under the stringent conditions to each of the above-mentioned polynucleotide or a probe that can be prepared from the above-mentioned polynucleotide as far as they encode polypeptides having a larvae growth inhibiting effect or an insecticidal effect on a Scarabaeidae insect. The term "the stringent conditions" used herein refers to a condition under which a so-called specific hybrid is formed and nonspecific hybrid is not formed. Such a condition is a condition in which DNAs having high homology, for example, DNAs having homology of 50% or more, preferably 70% or more, and more preferably 90% or more, are hybridized and DNAs having homology that is lower than the above are not hybridized. Specifically, it includes a condition of 40°C, 1×SSC (0.15 M NaCl, 15 mM Sodium Citrate, pH 7.0) and 0.1% SDS (Sodium Dodecyl Sulfate).

The polynucleotides of the present invention can be obtained from, for example, bacteria belonging to *Bacillus popilliae,* more specifically from *Bacillus popilliae semadara,* FERM P-16818, *Bacillus popilliae var. Mame,* FERM P-17661, *Bacillus popilliae var. popilliae Hime,* FERM P-17660, *Bacillus popilliae var. popilliae Sakura,* FERM P-17662, and *Bacillus popilliae* Dutky, American Type Culture Collection No. 14706 by the following methods.

*Bacillus popilliae semadara,* FERM P-16818 has been deposited by Chiba-ken (1-1 Ichiba-cho, Chuo-ku, Chiba-shi, Chiba-ken, Japan) since May 21, 1998 in National Institute of Bioscience and Human Technology of Agency of Industrial Science and Technology of Ministry of International Trade and Industry (zip code: 305-8566, 1-3 Higashi 1-Chome, Tsukuba-shi, Ibaraki-ken, Japan) (currently, National Institute of Advanced Industrial Science and Technology, International Patent Organism Depositary, Tsukuba Central 6, 1-1, Higashi 1-Chome, Tsukuba-shi, Ibaraki-ken, 305-8566, Japan), and the original deposit was converted to international deposit based on Budapest Treaty on June 10, 2002, and assigned the deposition number of FERM BP-8068.

*Bacillus popilliae var. Mame,* FERM P-17661 has been deposited by the applicant of the application since November 25, 1999 in National Institute of Bioscience and Human Technology of Agency of Industrial Science and Technology of Ministry of International Trade and Industry (zip code: 305-8566, 1-3 Higashi 1-Chome, Tsukuba-shi, Ibaraki-ken, Japan) (currently, National Institute of Advanced Industrial Science and Technology, International Patent Organism Depositary, Tsukuba Central 6, 1-1, Higashi 1-Chome, Tsukuba-shi, Ibaraki-ken, 305-8566, Japan), and the original deposit was converted to international deposit based on Budapest Treaty on June 10, 2002, and assigned the deposition number of FERM BP-8069.

One method of obtaining them includes a screening method by utilizing an antibody. That is, an antibody to a purified parasporal body is prepared in advance. On the other hand, a chromosomal DNA of the above-mentioned bacterial strain is collected, cleaved with an appropriate restriction enzyme and inserted into an expression vector to prepare a chromosomal DNA library. Then, the library is expanded in *Escherichia coli* or the like and screened by Western blotting utilizing the above-mentioned antibody.

Another method of obtaining the polynucleotides of the present invention includes a PCR method. An appropriate nucleotide sequence is selected from nucleotide sequences of SEQ ID NOs: 11 to 16, 21 and 22 and nucleotide sequences obtained by adding modifications thereto and partial nucleotide sequences derived from the nucleotide sequences of SEQ ID NOs: 1, 3, 5, 7, 9, 17 and 19, and two kinds of polynucleotides directing opposite to each other are provided. PCR is performed using the polynucleotides as primers and a chromosomal DNA as a template. By cloning an amplified DNA fragment, the polynucleotides of the present invention can be obtained.

Note that PCR using oligonucleotides having respective nucleotide sequences of SEQ ID NOS: 11 and 13 as primers and a chromosomal DNA of *Bacillus popilliae semadara* as a template can give rise to polynucleotide amplified fragments of about 4.2 kb having the nucleotide sequences of SEQ ID NOS: 17 and 19, respectively. The nucleotide sequences of these polynucleotides exist adjacent to each other on the chromosomal DNA of *Bacillus popilliae semadara* and the amino acid sequences (SEQ ID NOS: 18 and 20) of the polypeptides encoded by the polynucleotides have homology of about 73% to each other, which strongly suggests that both the polynucleotides have similar functions.

Also, the polynucleotides having the nucleotide sequences of SEQ ID NOS: 3, 5, 7, and 9 obtained by PCR using a chromosomal DNA of *Bacillus popilliae var. popilliae Mame* as a template and polypeptides having amino acid sequences of SEQ ID NOS: 4, 6, 8 and 10, which are translation products thereof, have high homology to the nucleotide sequence of the polynucleotide of *Bacillus popilliae semadara* and the polypeptide encoded by the polynucleotide (homology of 61 to 88% for the polynucleotide and 59 to 98% for the polypeptide). This suggests that they also have similar functions.

Further, another method of obtaining the polynucleotides of the present invention includes a hybridization method. After digesting the chromosomal DNA with an appropriate restriction enzyme, a genome library is prepared by utilizing a plasmid vector or a phage vector or the like. On the other hand, appropriate polynucleotide probes having the nucleotide sequences of SEQ ID NOS: 11 to 16, 21 and 22 or nucleotide sequences obtained by adding modifications thereto, or partial nucleotide sequences derived from the nucleotide sequences of SEQ ID NOS: 1, 3, 5, 7, 9, 17 and 19 are provided. By colony hybridization or plaque hybridization in which a library prepared in *Escherichia coli* or the like by using the vector is screened with a labeled probe, the polynucleotide of the present invention can be obtained.

The vectors that can be used in the present invention include plasmid vectors, Ti plasmid vectors, phage vectors, phagemid vectors, YAC vectors, virus vectors and the like. The vectors may include, in addition to the polynucleotides of the present invention, sequences that regulate or assist expression of polypeptides (for example, a promoter, an operator, a terminator, an enhancer, and an SD sequence) or marker gene for selecting transformant hosts (for example, an ampicillin resistance gene, a tetracycline resistance gene, a kanamycin resistance gene, and a neomycin resistance gene) and the like. The vectors may comprise plural kinds of polynucleotides of the present invention or a plurality of molecules of the same polynucleotide.

The hosts that can be used in the present invention include microbes (for example, bacteria such as *Escherichia coli, Pseudomonad,* and *Bacillus,* actinomycetes such as *Streptomyces,* Fungi such as molds and yeasts), plants (for example, turf, sweet potatoes, tobaccos, rice plants, and corn), cultured cells (for example, plant cultured cells such as a tobacco cultured cell, insect cultured cells such as a silkworm moth cultured cell and animal cultured cells such as mouse cultured cell) and the like.

In the present invention, conventional methods used in genetic engineering can be used in the procedure of constructing a vector and transformation operation of hosts with the vector. For example, the transformation method includes a calcium chloride method, an electroporation method, a PEG method and the like for microbe hosts, an electroporation method, a particle gun method, an agrobacterium method and the like for cultured cells and plant hosts.

In a case where the host is a microbe or a cultured cell, the method of producing a polypeptide of the present invention can be performed by culturing the microbe or the cultured cell in an appropriate medium and recover the produced polypeptide from the culture. The culture method of culturing a transformed host may be substantially the same as the culture method for the host to be used. Extraction of the polypeptide from the culture and purification thereof, if necessary, may be performed by usually employed methods. On the other hand, in a case where the host is a plant, the transformed plant is grown by the same methods as the methods usually used, and a polypeptide can be extracted from the plant body. From a different standpoint, the transformed plant and its seeds may be provided as is as insect resistant cultigen species. In addition, there is a production method of a polypeptide without using any transformant. It includes, for example, an in vitro cell-free transcription/translation system utilizing a wheat germ extract or an *Escherichia coli* extract. A polypeptide can be produced by adding a DNA fragment or an RNA fragment, or a vector containing the polynucleotide of the present invention to the transcription/translation system, thereby performing a conventional method for producing a polypeptide.

The polypeptides of the present invention have a larvae growth inhibiting or insecticidal activity on Scarabaeidae insects and exhibit controlling effects thereon. For this reason, the polypeptides can be applied to turf, agricultural crops or trees as a controlling agent or an active ingredient thereof for the purpose of controlling Scarabaeidae insects. Use of the polypeptides of the present invention in combination with spores or sporangia containing spores and parasporal bodies of bacteria belonging to *Bacillus popilliae* can further increase the larvae growth inhibiting or insecticidal activity on Scarabaeidae insects.

The Scarabaeidae insects that are the target of control with the polypeptides of the present invention include *Anomala cuprea, Blitopertha orientalis, Popillia japonica, Phyllopertha diversa, Adoretus tenuimaculatus, Anomala rufocuprea* and the like. The polypeptides of the present invention are particularly effective on *Anomala cuprea* and *Blitopertha orientalis.*

As the controlling agent of the present invention, the polypeptides produced by the above-mentioned production method may be used as they are in the form of the transformants, as a purified product after isolation or as mixtures of these with substances used for culture/reaction systems of these. Alternatively, they may be used by adding excipients, carriers, nutritive agents, stabilizers and the like known and commonly used in agricultural chemicals to convert them into an optional preparation form such as dust, wettable powder, or a flowable agent.

The above-mentioned optional component includes: mineral fine powders such as kaolin clay, bentonite, montmorillonite, diatomaceous earth, acid clay, talcs, pearlite, and vermiculite, inorganic salts such as ammonium sulfate, urea, ammonium chloride, and ammonium nitrate, organic fine powders such as wheat bran, chitin, polysaccharides, rice bran, and flour, as solid carriers; fixing agents and dispersants such as casein, gelatin, gum Arabic, alginic acid, saccharides, synthetic polymers (polyvinyl alcohol, polyacrylic acids, etc.) as adjutants; and cryoprotectants such as propylene glycol and ethylene glycol, natural polysaccharides such as xanthan gum, thickeners such as polyacrylic acids as other components.

The ratio of the polypeptides of the present invention contained in the controlling agent is not particularly limited as far as they exhibit a controlling effect on Scarabaeidae insects, but it is preferably 0.0001 to 100 mass%.

The method of application of the controlling agent is selected as appropriate depending on the use form such as the preparation form, crops and the like and includes, for example, liquid dispersion on the ground, solid dispersion on the ground, liquid dispersion in the air, solid dispersion in the air, application in installations, application for mixing with soil, application for perfusion into soil, and the like methods.

Also, it is possible to apply the controlling agent of the present invention after mixing with other agents, i.e., pesticides, nematicides, acaricides, herbicides, microbicides, plant growth regulators, fertilizers, soil conditioning materials (peat, humic acid materials, polyvinyl alcohol-based materials, etc.) and the like, to apply it with alternately applying the other agents without mixing, or to apply it together with the other agents simultaneously.

The application amount of the controlling agent of the present invention cannot be uniquely defined since it may vary depending on the kind of Scarabaeidae insect, kind of plant to which the agent is applied, preparation form of the agent and the like. For example, the application amount of the polypeptide may be 0.01 to 100 g/m², preferably about 0.1 to 50 g/m² for dispersion on the ground.

Further, it is possible to use mixtures of the controlling agent of the present invention with other biocontrol agents or polypeptides derived from microbes (for example, the parasporal bodies of another strain of *Bacillus popilliae* or the parasporal bodies of *Bacillus thuringiensis*) or microbial spores (for example, spores of *Bacillus popilliae, Bacillus thuringiensis,* or *Bacillus subtilis*).

### Best Mode for Carrying out the Invention

Hereinafter, the present invention will be illustrated more specifically. However, the scope of the present invention is not limited thereto.

### Example 1

A suspension of sporangia of *Bacillus popilliae* prepared from larvae of *Anomala cuprea* infected with milky disease was subjected to treatment by a French press to take out spores and parasporal bodies from the sporangia. Only the parasporal bodies were recovered by a two-phase separation method by using sodium dextran sulfate (produced by Sigma Co., Ltd.), polyethylene glycol 6000 (produced by Wako Pure Chemical Industry Co., Ltd.) and NaCl and solubilized with 0.1 M NaOH and then subjected to SDS-PAGE. An about 135 kD band was cut out and pulverized to small pieces, which then were mixed with an adjuvant. The mixture was intradermally injected to a rabbit to immunize it.

To decrease the background upon screening a polypeptide gene clone that constitutes the parasporal body by using an antibody, absorption operation of the obtained antiserum with *Escherichia coli* fragments was repeated three times to remove the antibody that adsorbs on *Escherichia coli* itself and collect only the antibody that adsorbs on the polypeptide constituting the target parasporal body.

### Example 2

A chromosomal DNA was collected from the bacterial cells of *Bacillus popilliae semadara* by an ordinary method and digested with restriction enzyme *Eco*RI. This was ligated to phosphatase-treated pBluescript SK(+) and *Escherichia coli* DH5α was transformed with the ligation product. The transformants were spread over the LB agar plate so that 2,000 to 3,000 colonies could appear thereon.

A nitrocellulose membrane was closely contacted on the agar plate to transfer colonies on the membrane and the membrane was placed on a fresh agar plate so that the colonies-attached side of the membrane directs upward. The plate was incubated at 37°C for 2 hours. For bacteriolysis of the bacterial cells on the membrane, the following operation was performed. That is, the membrane was transferred on a filter paper impregnated with 10% SDS and left to stand for 5 minutes. Then, the membrane was transferred on a filter paper impregnated with 0.2 M NaOH, 0.15 M NaCl and 1% β-mercaptoethanol and left to stand for 5 minutes and finally it was transferred on a filter paper impregnated with 0.15 M NaCl, 0.5 M Tris-HCl, and pH 7.5, and left to stand for 10 minutes. The membrane was transferred to a buffer solution (0.1 M Tris-HCl, pH 7.8, 0.15 M NaCl, 5 mM MgCl₂, 40 µg/ml lysozyme, and 5% skimmed milk) and shaken for 2 hours to remove the bacterial cells.

After washing the membrane with TBST (0.02 M Tris-HCl, pH 7.2, 0.5 M NaCl, and 0.05%. Tween 20) for 5 minutes, the membrane was shaken for 1.5 hours in an anti-parasporal body antiserum solution diluted 8,000 folds with an antibody diluting liquid (TBST containing 5% skimmed milk). After repeating 10 minutes' washing with TBST three times, the membrane was transferred into a solution of 2,000-fold diluted Anti-Rabbit IgG (H&L), HRP-Linked (produced by New England Biolabs, Inc.) and incubated until color development was observed in an HRP coloring substrate (4CN, produced by NEN Life Science Products Co.).

Colonies near a site where strong coloring was observed, that is, to which the anti-parasporal body antiserum was adsorbed, were collected one by one from the master plate and the collected colonies were subjected to secondary screening performed in the same manner as described above to obtain a positive clone.

Plasmid was collected from the positive clone and the nucleotide sequence of the inserted portion was determined by an ordinary method. As a result, it revealed that the plasmid contained two polypeptide genes, which constitute the parasporal body of *Bacillus popilliae,* (SEQ ID NOS: 17 and 19, respective translation products being of SEQ ID NOS: 18 and 20) in a single cloned fragment. The amino acid sequence of SEQ ID NO: 18 and that of SEQ ID NO: 20 have about 73% of homology to each other. In this case, gene information processing software GENETYX-WIN (Software Development Co., Ltd.) is used for retrieval of the homology.

### Example 3

PCR was performed by using the plasmid of the above-mentioned positive clone as a template and polynucleotides having nucleotide sequences corresponding to parts of a polynucleotide of SEQ ID NO: 19 and a complementary strand thereof (SEQ ID NOS: 21 and 22, respectively) as primers. After electrophoresing the reaction solution, about 4 kb of amplified fragments were recovered and purified and digested with restriction enzymes *Eco*RI and *PstI.* The digested products were ligated to a vector pTrc99A (produced by Amersham Pharmacia Biotech Co., Ltd.) that had been digested with EcoRI and PstI in advance, and *Escherichia coli* BL21(DE3) was transformed with the reaction solution. The transformants obtained were shake-cultured overnight in a 2xYT liquid medium containing 50 mg/l of ampicillin and then IPTG (isopropyl β-D-thiogalactopyranoside) was added so as to bring the transformants to a final concentration of 1 mM, followed by further culture for 2 hours. Observation with a microscope indicated formation of an inclusion body in the cells. Therefore, the clone was named as an expression clone (1).

### Example 4

In a 2-liter baffled Erlenmeyer flask was charged 750 ml of a 2xYT liquid medium containing 50 mg/l of ampicillin and the expression clone (1) obtained in Example 3 was inoculated therein. The medium was shake-cultured at 37°C. 16 hours afterwards, IPTG is added to the medium so as to bring it to a final concentration of 1 mM, and the medium was further cultured for 2 hours. The cultured medium was centrifuged to recover from the culture solution bacterial cells, which were washed with a 10 mM potassium phosphate buffer solution, pH 7.0. Again the suspension was centrifuged to recover the bacterial cells, which were suspended in the above-mentioned buffer solution to finally make it into 120 ml.

10 g per cup of leaf mold was measured out and charged in 40 plastic-made food cups of 6 cm in diameter. In 20 cups out of them was dispersed 1 ml per cup of the suspension of the expression clone (1) and 1 ml per cup of water as blank was dispersed in the remaining 20 cups, followed by well mixing. In each of the food cups, one first instar larva of *Anomala cuprea* was released and fed under a condition of 25°C. Mortality of the larvae and mean body weight of the surviving larvae were measured with a lapse of time to verify the insecticidal activity and larvae growth inhibiting activity of the expression clone (1).

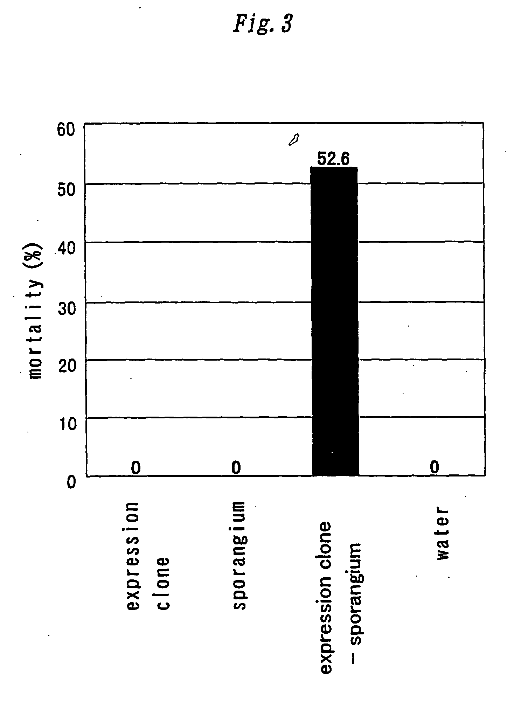

The results are shown in Table 1 and Fig. 2. A lot where the expression clone (1) was dispersed showed an increase in mortality, and on 5^{th} week, high mortality of 95% was obtained. Further, the mean body weight of surviving larvae in the lot where the expression clone (1) was dispersed did not substantially increase but remained at a low level as compared with the blank. These results confirmed that the expression clone (1), that is, the polypeptide of the present invention has a larvae growth inhibiting or insecticidal activity.

**Table 1**

| test lot | Cumulative Mortality (%) | | | | |
|---|---|---|---|---|---|
| | 1^{st} week | 2^{nd} week | 3^{rd} week | 4^{th} week | 5^{th} week |
| Expression clone | 5.0 | 45.0 | 65.0 | 80.0 | 95.0 |
| Blank | 5.0 | 5.0 | 5.0 | 5.0 | 5.0 |

### Example 5

In a 2-liter baffled Erlenmeyer flask was charged 750 ml of a 2xYT liquid medium containing 50 mg/l of ampicillin and the expression clone (1) obtained in Example 3 was inoculated therein. The medium was shake-cultured at 37°C. After 16 hours, IPTG was added so as to bring the medium to a final concentration of 1 mM and the medium was further cultured for 2 hours. The culture medium was centrifuged to recover from the culture solution bacterial cells, which were washed with a 10 mM potassium phosphate buffer solution, pH 7.0. Again the suspension was centrifuged to recover the bacterial cells, which were suspended in the above-mentioned buffer solution to finally make it into 75 ml, thus making it as an expression clone suspension.

On the other hand, the sporangia of *Bacillus popilliae samadara* were collected from larvae of *Anomala cuprea* infected with milky disease and washed with water. They were suspended again in water to a density of 8×10⁷ sporangia/ml to make it as a sporangia suspension.

80 plastic-made food cups of 6 cm in diameter, each of which was charged with 25 g of leaf mold that was measured out, were prepared. In 20 cups out of them was dispersed 1 ml per cup of the suspension of the expression clone (1), in other 20 cups was dispersed 0.5 ml per cup of the sporangia suspension, in still other 20 cups was dispersed 1 ml of the expression clone suspension together with 0.5 ml of the sporangia suspension, and in the remaining 20 cups was dispersed 1 ml per cup of water, followed by well mixing. In each of the food cups, a second instar larva of *Anomala cuprea* was released one by one and fed under a condition of 25°C for 21 days. Mortality of the larvae and mean body weight of the surviving larvae were measured with a lapse of time to verify synergistic effects in the insecticidal activity and the larvae growth inhibiting activity of the expression clone (1) used in combination with the sporangia.

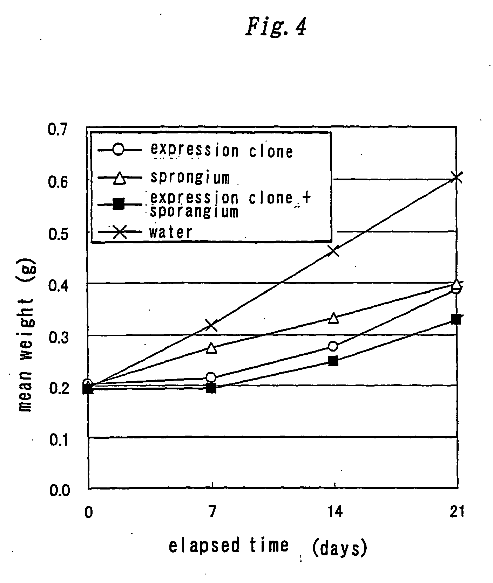

The results are shown in Figs. 3 and 4. Dispersion of the suspension of the expression clone (1) or the sporangia alone exhibited a larvae growth inhibiting effect but did not exhibit a sufficient insecticidal effect. In contrast, when the expression clone (1) and the sporangia were dispersed in combination, an abrupt increase in mortality was observed, so that synergistic effect was confirmed.

### Example 6

Polynucleotides having the nucleotide sequences of SEQ ID NO: 11 and SEQ ID NO: 13 corresponding to parts of complementary strands of SEQ ID NO: 17 and SEQ ID NO: 19, respectively, were prepared by chemical synthesis. PCR performed by using the polynucleotides as primers and a chromosomal DNA of *Bacillus popilliae semadara* as a template indicated amplification of about 4.2 kb fragments. TA cloning of the fragments gave rise to a number of clones, some of which were subjected to determination of the nucleotide sequence of the insert portion by sequencing. As a result, polynucleotides , having the nucleotide sequences of SEQ ID NO: 17 and SEQ ID NO: 19 were obtained. Therefore, it revealed that the polynucleotide of the present invention can also be obtained by PCR using the polynucleotides having the nucleotide sequences of SEQ ID NO: 11 and SEQ ID NO: 13, respectively.

### Example 7

A chromosomal DNA was collected from the bacterial cells of *Bacillus popilliae var. popilliae Mame* by an ordinary method. On the other hand, based on the nucleotide sequence of the gene encoding the polypeptide constituting the parasporal body of *Bacillus popilllae semadara* as a reference, polynucleotides having the nucleotide sequences of SEQ ID NO: 11 and SEQ ID NO: 14, respectively, were chemically synthesized. PCR performed using the polynucleotides as primers and the chromosomal DNA as a template indicated amplification of about 4.3 kb fragments. TA cloning of the fragments gave rise to a number of clones, one of which was subjected to determination of the nucleotide sequence of the insert by sequencing. As a result, a polynucleotide having the nucleotide sequence of SEQ ID NO: 3 was obtained. This was further translated to obtain the amino acid sequence of SEQ ID NO: 4.

### Example 8

Polynucleotides having nucleotide sequences of SEQ ID NO: 12 and SEQ ID NO: 13, respectively, were prepared by chemical synthesis. PCR performed by using the polynucleotides as primers and the chromosomal DNA prepared as described above as a template indicated amplification of about 4.2 kb fragments. TA cloning of the fragments gave rise to a large number of clones, one of which was subjected to determination of the nucleotide sequence of the insert portion by sequencing. As a result, a polynucleotide having the nucleotide sequence of SEQ ID NO: 5 was obtained. This was further translated to obtain the amino acid sequence of SEQ ID NO: 6.

### Example 9 (Referential Example)

Polynucleotides having the nucleotide sequence of SEQ ID NO: 15 corresponding to a part of a complementary strand of SEQ ID NO: 3 and the nucleotide sequence of SEQ ID NO: 11, respectively, were prepared by chemical synthesis. PCR performed by using the polynucleotides as primers and the chromosomal DNA prepared as described above as a template indicated amplification of about 1.2 kb fragments. TA cloning of the fragments gave rise to a large number of clones, one of which was subjected to determination of the nucleotide sequence of the insert portion by sequencing. As a result, a polynucleotide having the nucleotide sequence of SEQ ID NO: 7 was obtained. This was further translated to obtain the amino acid sequence of SEQ ID NO: 8.

### Example 10

The chromosomal DNA obtained as described above was degested with restriction enzyme *Xho*I and the fragments were ligated with *Sal*I Cassette contained in a LA PCR in vitro Cloning Kit produced by Takara Shuzo Co., Ltd. On the other hand, a polynucleotide having the nucleotide sequence (SEQ ID NO.: 16) corresponding to a part of a sequence of SEQ ID NO: 3 was prepared by chemical synthesis. Then, PCR was performed by using the polynucleotide and a Cassette primer C1 attached to the Kit as primers and the ligated chromosomal DNA fragment as a template. As a result, amplification of about 3 kb fragments was observed. TA cloning of the fragments gave rise to a large number of clones, one of which was subjected to determination of the nucleotide sequence of the insert portion by sequencing. As a result, a clone having a polynucleotide having the nucleotide sequence of SEQ ID NO: 9 coupled in tandem to downstream of the latter half of the nucleotide sequence of SEQ ID NO: 3 was obtained. The nucleotide sequence of SEQ ID NO: 9 was further translated to obtain the amino acid sequence of SEQ ID NO: 10.

### Example 11

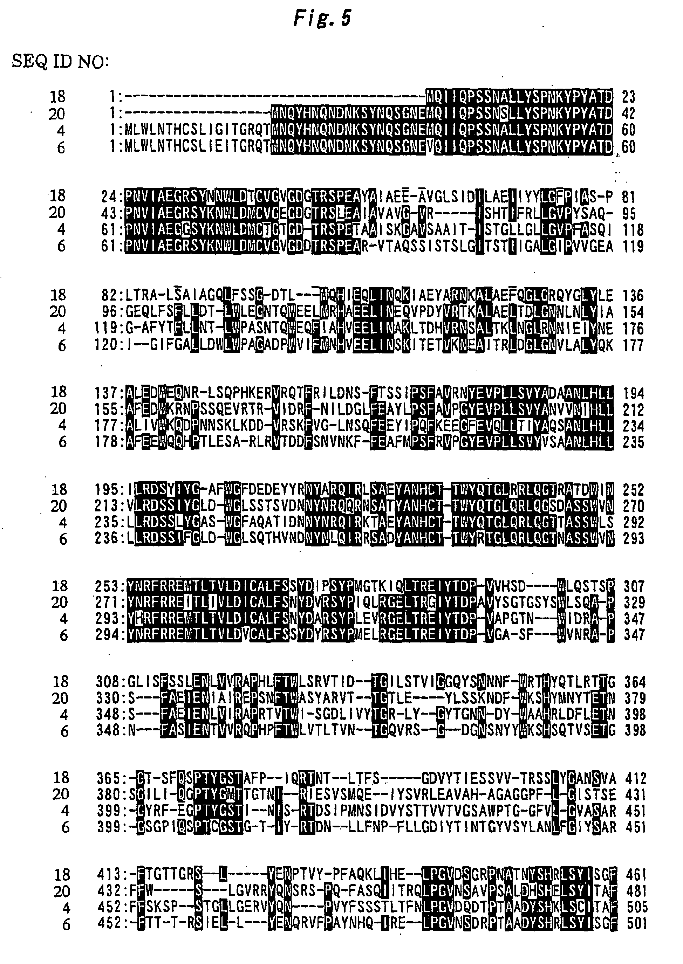

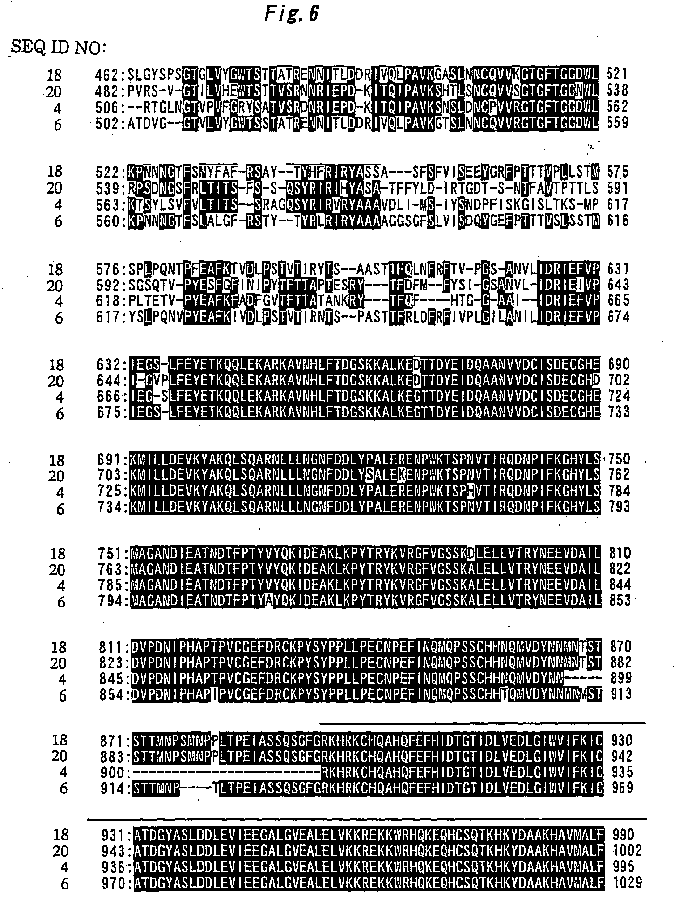

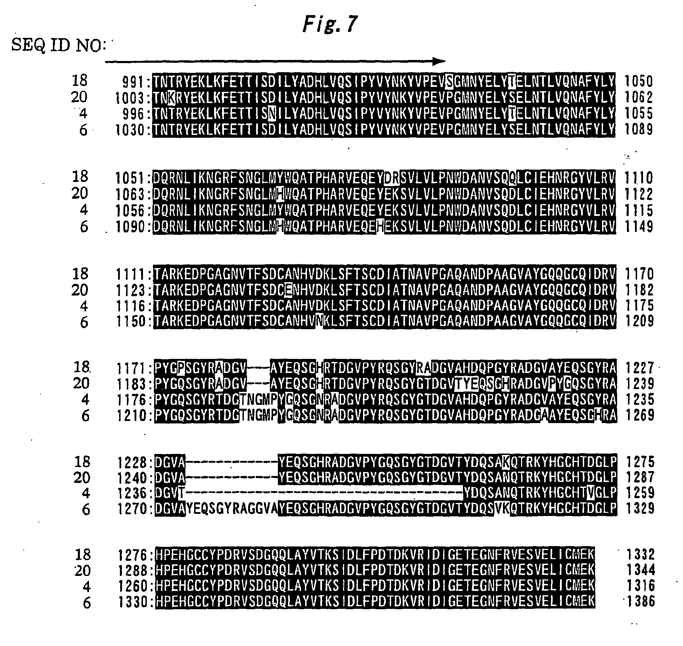

The amino acid sequences of the polypeptides of the parasporal bodies existing in *Bacillus popilliae* obtained in Examples 2, 7 and 8 and the nucleotide sequences of the polynucleotides encoding them were comparatively studied by using gene information processing software GENETYX-WIN (Software Development Co., Ltd.). As a result, sequences that have very high homology of 98% or more in the amino acid sequence and are considered to be specific to the parasporal body of *Bacillus popilliae,* i.e., the amino acid sequence of SEQ ID NO: 2 and the corresponding nucleotide sequence of SEQ ID NO: 1 were found. The portions indicated by an arrow in Figs. 5 to 7 correspond to the amino acid sequence of SEQ ID NO: 2.

Note that these sequences have homology of 9% or less in the amino acid sequence to the sequence isolated from *Bacillus popilliae* subsp. *Melolonthae* H1 described in WO97/14798, and hence is completely different therefrom.

### Example 12

PCR was performed by using a chromosomal DNA of *Bacillus popilliae var. popilliae Name* as a template and polynucleotides of SEQ ID NO: 12 and SEQ ID NO: 14 as primers to amplify about 4 kb fragments, which were purified and recovered by electrophoresis and subsequent cutting out. The fragments were ligated to a pT7Blue vector (Novagen, Inc.) and *Escherichia coli* DH5α was transformed with the ligation product. Among the obtained clones, a clone harboring the pT7Blue with the 4 kb insert downstream of lac promoter in a direction of sense to form a fusion polypeptide with an N-terminal portion of a lacZ polypeptide was selected. This was named as an expression clone (2) and used in the following tests of production of a polypeptide and effects thereof.

### Example 13

Ten 300 ml-capacity Erlenmeyer flasks containing 200 ml of a 2xYT medium were prepared. In 5 flasks out of them was inoculated the expression clone (2) obtained in Example 12 and shake-cultured at 37°C overnight. As a comparative control clone, *Escherichia coli* DH5α having a pT7Blue vector having inserted therein no 4 kb fragment was inoculated in the remaining 5 flasks and cultured in the same manner as the above. After completion of the culture, bacterial cells were observed with a microscope. As a result, formation of inclusion body in the bacterial cells was observed only in the case of the expression clone (2). The bacterial cells were once recovered from each flask by centrifugation and after washing with water, the bacterial cells were centrifuged again to recover them and then suspended in water to finally make it into 23 ml.

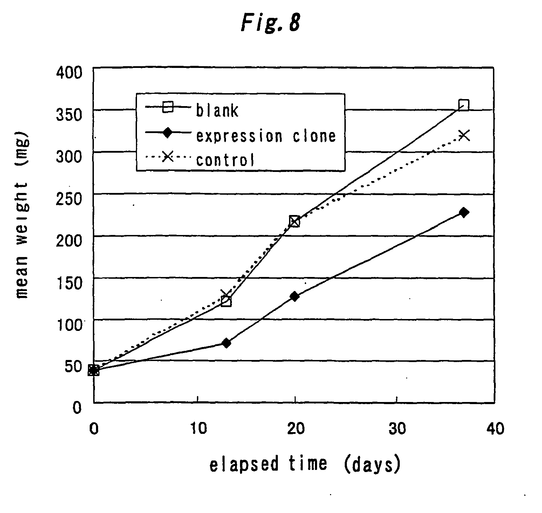

20 g per cup of leaf mold was measured out and charged in 15 plastic-made food cups of 6 cm in diameter. In 5 cups out of them was added a suspension of the expression clone and in other 5 cups out of them was added a suspension of the comparative control clone, followed by well mixing. In the remaining 5 cups was dedicated to blank tests of no addition. In each of the food cups, one first instar larva of *Anomala cuprea* was released and fed under a condition of 25°C. The body weights of larvae were measured with a lapse of time and compared to verify the larvae growth inhibiting activity of the expression clone (2). In contrast to the blank test and the control lot, a lot where the expression clone (2) was added apparently suppressed increase in mean body weight (Fig. 8), which confirmed that the expression clone (2), that is, the polypeptide of the present invention has a larvae growth inhibiting effect.

## Claims

1. A polypeptide having the amino acid sequence of SEQ ID NO: 2, or the amino acid sequence including substitution, deletion or insertion of 9 or less amino acid residues and having a larvae growth inhibiting or insecticidal effect on a Scarabaeidae insect.

2. A polypeptide having the amino acid sequence of SEQ ID NO: 4, 6, 18 or 20, or the amino acid sequence including substitution, deletion or insertion of 50 or less amino acid residues and having a larvae growth inhibiting or insecticidal effect on a Scarabaeidae insect.

3. A polynucleotide encoding the polypeptide of claim 1 or 2.

4. The polynucleotide according to claim 3, wherein the polynucleotide comprises the nucleotide sequence of SEQ ID NO: 1, 3, 5, 17 or 19.

5. A vector comprising the polynucleotide of claim 3 or 4.

6. A transformant of a microbe, plant or cultured cell which has introduced the polynucleotide of claim 3 or 4, or a vector of claim 5.

7. A Scarabaeidae insect controlling agent comprising as an active ingredient the polypeptide of claim 1 or 2.

8. The Scarabaeidae insect controlling agent according to claim 7, further comprising sporangia of a bacterium belonging to *Bacillus popilliae.*

9. A method of controlling a Scarabaeidae insect comprising using the Scarabaeidae insect controlling agent of claim 7 or 8.

## Patentansprüche

1. Polypeptid, das die Aminosäuresequenz von SEQ ID NO: 2 oder die Aminosäuresequenz, die Substitution, Deletion oder Insertion von 9 oder weniger Aminosäureresten einschließt, aufweist, und einen Larvenwachstum-inhibierenden oder insektiziden Effekt auf ein Scarabaeidae-Insekt aufweist.

2. Polypeptid, das die Aminosäuresequenz von SEQ ID NO: 4, 6, 18 oder 20 oder die Aminosäuresequenz, die Substitution, Deletion oder Insertion von 50 oder weniger Aminosäureresten einschließt, aufweist, und einen Larvenwachstum-inhibierenden oder insektiziden Effekt auf ein Scarabaeidae-Insekt aufweist.

3. Polynucleotid, codierend das Polypeptid nach Anspruch 1 oder 2.

4. Polynucleotid nach Anspruch 3, wobei das Polynucleotid die Nucleotidsequenz von SEQ ID NO: 1, 3, 5, 17 oder 19 umfasst.

5. Vektor, umfassend das Polynucleotid nach Anspruch 3 oder 4.

6. Transformante einer Mikrobe, Pflanze oder kultivierten Zelle, welche eingeführt das Polynucleotid nach Anspruch 3 oder 4 oder einen Vektor nach Anspruch 5 aufweist.

7. Scarabaeidae-Insekt-Bekämpfungsmittel, umfassend als einen aktiven Bestandteil das Polypeptid nach Anspruch 1 oder 2.

8. Scarabaeidae-Insekt-Bekämpfungsmittel nach Anspruch 7, ferner umfassend Sporangien eines Bakteriums, das zu *Bacillus popilliae* gehört.

9. Verfahren zur Bekämpfung eines Scarabaeidae-Insekts, umfassend das Verwenden des Scarabaeidae-Insekt-Bekämpfungsmittels nach Anspruch 7 oder 8.

## Revendications

1. Polypeptide présentant la séquence d'acides aminés SEQ ID NO : 2 ou la séquence d'acides aminés comprenant la substitution, la délétion ou l'insertion de 9 résidus d'acides aminés ou moins, et ayant un effet inhibant la croissance larvaire ou un effet insecticide sur un insecte scarabéidé.

2. Polypeptide présentant la séquence d'acides aminés SEQ ID NO : 4, 6, 18 ou 20, ou la séquence d'acides aminés comprenant la substitution, la délétion ou l'insertion de 50 résidus d'acides aminés ou moins, et ayant un effet inhibant la croissance larvaire ou un effet insecticide sur un insecte scarabéidé.

3. Polynucléotide codant pour le polypeptide de la revendication 1 ou 2.

4. Polynucléotide selon la revendication 3, dans lequel le polynucléotide comprend la séquence de nucléotides SED ID N° : 1, 3, 5, 17 ou 19.

5. Vecteur comprenant le polynucléotide de la revendication 3 ou 4.

6. Transformant d'un microorganisme, d'une plante ou d'une cellule cultivée qui a introduit le polynucléotide de la revendication 3 ou 4, ou un vecteur de la revendication 5.

7. Agent de contrôle d'un insecte scarabéidé comprenant comme ingrédient actif, le polypeptide de la revendication 1 ou 2.

8. Agent de contrôle d'un insecte scarabéidé selon la revendication 7, comprenant en outre un sporange d'une bactérie appartenant à *Bacillus popilliae.*

9. Procédé de contrôle d'un insecte scarabéidé comprenant l'utilisation de l'agent de contrôle de l'insecte scarabéidé de la revendication 7 ou 8.


### SEQUENCE LISTING

<110> Dainippon Ink and Chemicals, Inc.
<120> Polypeptide having larvae growth inhibiting or insecticidal effect on scarabaeidae insects and polynucleotide encoding the same
<130> 12290EP
<140>
   <141>
<150> JP 2001-115754
   <151> 2001-04-13
   <150> JP 2001-203463
   <151> 2001-07-04
<160> 22
<170> Patent In Ver. 2.0
<210> 1
   <211> 402
   <212> DNA
   <213> Bacillus popilliae
<220>
   <221> CDS
   <222> (1) .. (402)
<400> 1
<210> 2
   <211> 134
   <212> PRT
   <213> Bacillus popilliae
<400> 2
<210> 3
   <211> 4359
   <212> DNA
   <213> Bacillus popilliae
<220>
   <221> CDS
   <222> (282)..(4229)
<400> 3
<210> 4
   <211> 1316
   <212> PRT
   <213> Bacillus popilliae
<400> 4
   Met Leu Trp Leu Asn Thr His Cys Ser Leu Ile Gly Ile Thr Gly Arg
    1               5                  10                  15
   Gln Thr Met Asn Gln Tyr His Asn Gln Asn Asp Asn Lys Ser Tyr Asn
                   20                  25                  30
   Gln Ser Gly Asn Glu Met Gln Ile Ile Gln Pro Ser Ser Asn Ala Leu
               35                  40                  45
   Leu Tyr Ser Pro Asn Lys Tyr Pro Tyr Ala Thr Asp Pro Asn Val Ile
           50                  55                  60
   Ala Glu Gly Gly Ser Tyr Lys Asn Trp Leu Asp Met Cys Thr Gly Thr
    65                  70                  75                  80
   Gly Asp Thr Arg Ser Pro Glu Thr Ala Ala Ile Ser Lys Gly Ala Val
                   85                  90                  95
   Ser Ala Ala Ile Thr Ile Ser Thr Gly Leu Leu Gly Leu Leu Gly Val
               100                 105                 110
   Pro Phe Ala Ser Gln Ile Gly Ala Phe Tyr Thr Phe Leu Leu Asn Thr
           115                 120                 125
   Leu Trp Pro Ala Ser Asn Thr Gln Trp Glu Gln Phe Ile Ala His Val
       130                 135                 140
   Glu Glu Leu Ile Asn Ala Lys Leu Thr Asp His Val Arg Asn Ser Ala
   145                 150                 155                 160
   Leu Thr Lys Leu Asn Gly Leu Arg Asn Asn Ile Glu Ile Tyr Asn Glu
                   165                 170                 175
   Ala Leu Ile Val Trp Lys Gln Asp Pro Asn Asn Ser Lys Leu Lys Asp
               180                 185                 190
   Asp Val Arg Ser Lys Phe Val Gly Leu Asn Ser Gln Phe Glu Glu Tyr
           195                 200                 205
   Ile Pro Gln Phe Lys Glu Glu Gly Phe Glu Val Gln Leu Leu Thr Ile
       210                 215                 220
   Tyr Ala Gln Ser Ala Asn Leu His Leu Leu Leu Leu Arg Asp Ser Ser
   225                 230                 235                 240
   Leu Tyr Gly Ala Ser Trp Gly Phe Ala Gln Ala Thr Ile Asp Asn Asn
                   245                 250                 255
   Tyr Asn Arg Gln Ile Arg Lys Thr Ala Glu Tyr Ala Asn His Cys Thr
               260                 265                 270
   Thr Trp Tyr Gln Thr Gly Leu Gln Arg Leu Gln Gly Thr Thr Ala Ser
           275                 280                 285
   Ser Trp Leu Ser Tyr His Arg Phe Arg Arg Glu Met Thr Leu Thr Val
       290                 295                 300
   Leu Asp Ile Cys Ala Leu Phe Ser Asn Tyr Asp Ala Arg Ser Tyr Pro
   305                 310                 315                 320
   Leu Glu Val Arg Gly Glu Leu Thr Arg Glu Ile Tyr Thr Asp Pro Val
                   325                 330                 335
   Ala Pro Gly Thr Asn Trp Ile Asp Arg Ala Pro Ser Phe Ala Glu Ile
               340                 345                 350
   Glu Asn Leu Val Ile Arg Ala Pro Arg Thr Val Thr Trp Ile Ser Gly
           355                 360                 365
   Asp Leu Ile Val Tyr Thr Gly Arg Leu Tyr Gly Tyr Thr Gly Asn Asn
       370                 375                 380
   Asp Tyr Trp Ala Ala His Arg Leu Asp Phe Leu Glu Thr Asn Gly Tyr
   385                 390                 395                 400
   Arg Phe Glu Gly Pro Thr Tyr Gly Ser Thr Ile Asn Ile Ser Arg Thr
                   405                 410                 415
   Asp Ser Ile Pro Met Asn Ser Ile Asp Val Tyr Ser Thr Thr Val Val
               420                 425                 430
   Thr Val Gly Ser Ala Trp Pro Thr Gly Gly Phe Val Leu Gly Val Ala
           435                 440                 445
   Ser Ala Arg Phe Phe Ser Lys Ser Pro Ser Thr Gly Leu Leu Gly Glu
       450                 455                 460
   Arg Val Tyr Gln Asn Pro Val Tyr Phe Ser Ser Ser Thr Leu Thr Phe
   465                 470                 475                 480
   Asn Leu Pro Gly Val Asp Gln Asp Thr Pro Thr Ala Ala Asp Tyr Ser
                   485                 490                 495
   His Lys Leu Ser Cys Ile Thr Ala Phe Arg Thr Gly Leu Asn Gly Thr
               500                 505                 510
   Val Pro Val Phe Gly Arg Tyr Ser Ala Thr Val Ser Arg Asp Asn Arg
           515                 520                 525
   Ile Glu Pro Asp Lys Ile Thr Gln Ile Pro Ala Val Lys Ser Asn Ser
       530                 535                 540
   Leu Asp Asn Cys Pro Val Val Arg Gly Thr Gly Phe Thr Gly Gly Asp
   545                 550                 555                 560
   Trp Leu Lys Thr Ser Tyr Leu Ser Val Phe Val Leu Thr Ile Thr Ser
                   565                 570                 575
   Ser Arg Ala Gly Gln Ser Tyr Arg Ile Arg Val Arg Tyr Ala Ala Ala
               580                 585                 590
   Val Asp Leu Ile Met Ser Ile Tyr Ser Asn Asp Pro Phe Ile Ser Lys
           595                 600                 605
   Gly Ile Ser Leu Thr Lys Ser Met Pro Pro Leu Thr Glu Thr Val Pro
       610                 615                 620
   Tyr Glu Ala Phe Lys Phe Ala Asp Phe Gly Val Thr Phe Thr Thr Ala
   625                 630                 635                 640
   Thr Ala Asn Lys Arg Tyr Thr Phe Gln Phe His Thr Gly Gly Ala Ala
                   645                 650                 655
   Ile Ile Asp Arg Ile Glu Phe Val Pro Ile Glu Gly Ser Leu Phe Glu
               660                 665                 670
   Tyr Glu Thr Lys Gln Gln Leu Glu Lys Ala Arg Lys Ala Val Asn His
           675                 680                 685
   Leu Phe Thr Asp Gly Ser Lys Lys Ala Leu Lys Glu Gly Thr Thr Asp
       690                 695                 700
   Tyr Glu Ile Asp Gln Ala Ala Asn Val Val Asp Cys Ile Ser Asp Glu
   705                 710                 715                 720
   Cys Gly His Glu Lys Met Ile Leu Leu Asp Glu Val Lys Tyr Ala Lys
                   725                 730                 735
   Gln Leu Ser Gln Ala Arg Asn Leu Leu Leu Asn Gly Asn Phe Asp Asp
               740                 745                 750
   Leu Tyr Pro Ala Leu Glu Arg Glu Asn Pro Trp Lys Thr Ser Pro His
           755                 760                 765
   Val Thr Ile Arg Gln Asp Asn Pro Ile Phe Lys Gly His Tyr Leu Ser
       770                 775                 780
   Met Ala Gly Ala Asn Asp Ile Glu Ala Thr Asn Asp Thr Phe Pro Thr
   785                 790                 795                 800
   Tyr Val Tyr Gln Lys Ile Asp Glu Ala Lys Leu Lys Pro Tyr Thr Arg
                   805                 810                 815
   Tyr Lys Val Arg Gly Phe Val Gly Ser Ser Lys Ala Leu Glu Leu Leu
               820                 825                 830
   Val Thr Arg Tyr Asn Glu Glu Val Asp Ala Ile Leu Asp Val Pro Asp
           835                 840                 845
   Asn Ile Pro His Ala Pro Thr Pro Val Cys Gly Glu Phe Asp Arg Cys
       850                 855                 860
   Lys Pro Tyr Ser Tyr Pro Pro Leu Leu Pro Glu Cys Asn Pro Glu Phe
   865                 870                 875                 880
   Ile Asn Gln Met Gln Pro Ser Ser Cys His His Asn Gln Met Val Asp
                   885                 890                 895
   Tyr Asn Asn Arg Lys His Arg Lys Cys His Gln Ala His Gln Phe Glu
               900                 905                 910
   Phe His Ile Asp Thr Gly Thr Ile Asp Leu Val Glu Asp Leu Gly Ile
           915                 920                 925
   Trp Val Ile Phe Lys Ile Cys Ala Thr Asp Gly Tyr Ala Ser Leu Asp
       930                 935                 940
   Asp Leu Glu Val Ile Glu Glu Gly Ala Leu Gly Val Glu Ala Leu Glu
   945                 950                 955                 960
   Leu Val Lys Lys Arg Glu Lys Lys Trp Arg His Gln Lys Glu Gln His
                   965                 970                 975
   Cys Ser Gln Thr Lys His Lys Tyr Asp Ala Ala Lys His Ala Val Met
               980                 985                 990
   Ala Leu Phe Thr Asn Thr Arg Tyr Glu Lys Leu Lys Phe Glu Thr Thr
           995                 1000                1005
   Ile Ser Asn Ile Leu Tyr Ala Asp His Leu Val Gln Ser Ile Pro Tyr
       1010                1015                1020
   Val Tyr Asn Lys Tyr Val Pro Glu Val Pro Gly Met Asn Tyr Glu Leu
   1025                1030                1035                1040
   Tyr Thr Glu Leu Asn Thr Leu Val Gln Asn Ala Phe Tyr Leu Tyr Asp
                   1045                1050                1055
   Gln Arg Asn Leu Ile Lys Asn Gly Arg Phe Ser Asn Gly Leu Met Tyr
               1060                1065                1070
   Trp Gln Ala Thr Pro His Ala Arg Val Glu Gln Glu Tyr Glu Lys Ser
           1075                1080                1085
   Val Leu Val Leu Pro Asn Trp Asp Ala Asn Val Ser Gln Asp Leu Cys
       1090                1095                1100
   Ile Glu His Asn Arg Gly Tyr Val Leu Arg Val Thr Ala Arg Lys Glu
   1105                1110                1115                1120
   Asp Pro Gly Ala Gly Asn Val Thr Phe Ser Asp Cys Ala Asn His Val
                   1125                1130                1135
   Asp Lys Leu Ser Phe Thr Ser Cys Asp Ile Ala Thr Asn Ala Val Pro
               1140                1145                1150
   Gly Ala Gln Ala Asn Asp Pro Ala Ala Gly Val Ala Tyr Gly Gln Gln
         1155                1160                1165
   Gly Cys Gln Ile Asp Arg Val Pro Tyr Gly Gln Ser Gly Tyr Arg Thr
      1170                1175                1180
   Asp Gly Thr Asn Gly Met Pro Tyr Gly Gln Ser Gly Asn Arg Ala Asp
   1185                1190                1195                1200
   Gly Val Pro Tyr Arg Gln Ser Gly Tyr Gly Thr Asp Gly Val Ala His
                   1205                1210                1215
   Asp Gln Pro Gly Tyr Arg Ala Asp Gly Val Ala Tyr Glu Gln Ser Gly
               1220                1225                1230
   Tyr Arg Ala Asp Gly Val Thr Tyr Asp Gln Ser Ala Asn Gln Thr Arg
         1235                1240                1245
   Lys Tyr His Gly Cys His Thr Val Gly Leu Pro His Pro Glu His Gly
      1250                1255                1260
   Cys Cys Tyr Pro Asp Arg Val Ser Asp Gly Gln Gln Leu Ala Tyr Val
   1265                1270                1275                1280
   Thr Lys Ser Ile Asp Leu Phe Pro Asp Thr Asp Lys Val Arg Ile Asp
                   1285                1290                1295
   Ile Gly Glu Thr Glu Gly Asn Phe Arg Val Glu Ser Val Glu Leu Ile
               1300                1305                1310
   Cys Met Glu Lys
         1315
<210> 5
   <211> 4188
   <212> DNA
   <213> Bacillus popilliae
<220>
   <221> CDS
   <222> (1)..(4158)
<400> 5
   atg ttg tgg cta aat act cat tgt tca tta ata gaa ata act ggg agg    48
   Met Leu Trp Leu Asn Thr His Cys Ser Leu Ile Glu Ile Thr Gly Arg
    1               5                  10                  15
   caa act atg aat caa tat cat aac caa aac gat aac aaa agt tac aac    96
   Gln Thr Met Asn Gln Tyr His Asn Gln Asn Asp Asn Lys Ser Tyr Asn
                   20                  25                  30
   caa agt gga aat gaa gtg caa att ata caa cct tca agt aac gct tta   144
   Gln Ser Gly Asn Glu Val Gln Ile Ile Gln Pro Ser Ser Asn Ala Leu
               35                  40                  45
   ctt tac agt ccc aac aag tat ccg tat gct acg gat ccc aat gtc ata   192
   Leu Tyr Ser Pro Asn Lys Tyr Pro Tyr Ala Thr Asp Pro Asn Val Ile
           50                  55                  60
   gca gag ggt aga agt tat aaa aat tgg ttg gat atg tgt gta ggt gta   240
   Ala Glu Gly Arg Ser Tyr Lys Asn Trp Leu Asp Met Cys Val Gly Val
       65                  70                  75                  80
   ggt gac gat aca cga agt ccc gaa gct cgg gtt act gca caa agt tct   288
   Gly Asp Asp Thr Arg Ser Pro Glu Ala Arg Val Thr Ala Gln Ser Ser
                   85                  90                  95
   att tcg act tct ctt ggt ata acc agc aca atc att ggc gct cta ggt   336
   Ile Ser Thr Ser Leu Gly Ile Thr Ser Thr Ile Ile Gly Ala Leu Gly
               100                 105                 110
   att ccg gtt gta ggc gaa gcc att gga att ttc ggc gca cta ttg gat   384
   Ile Pro Val Val Gly Glu Ala Ile Gly Ile Phe Gly Ala Leu Leu Asp
           115                 120                 125
   tgg ttg tgg cct gca ggc gct gat cca tgg gta ata ttt atg aat cat   432
   Trp Leu Trp Pro Ala Gly Ala Asp Pro Trp Val Ile Phe Met Asn His
       130                 135                 140
   gta gaa gaa ctc ata aat tca aaa ata aca gag act gta aaa aat gag   480
   Val Glu Glu Leu Ile Asn Ser Lys Ile Thr Glu Thr Val Lys Asn Glu
   145                 150                 155                 160
   gca att aca aga tta gac ggt tta ggc aat gtc cta gcg cta tat caa    528
   Ala Ile Thr Arg Leu Asp Gly Leu Gly Asn Val Leu Ala Leu Tyr Gln
                   165                 170                 175
   aag gct ttc gaa gaa tgg caa caa cat cct act ctt gaa tca gct cga    576
   Lys Ala Phe Glu Glu Trp Gln Gln His Pro Thr Leu Glu Ser Ala Arg
               180                 185                 190
   cta cgt gta aca gat gat ttt tct aat gta aat aaa ttt ttt gag gca    624
   Leu Arg Val Thr Asp Asp Phe Ser Asn Val Asn Lys Phe Phe Glu Ala
           195                 200                 205
   ttt atg cct tca ttc aga gta cca ggt tat gaa gta cca tta tta agc    672
   Phe Met Pro Ser Phe Arg Val Pro Gly Tyr Glu Val Pro Leu Leu Ser
       210                 215                 220
   gta tac gta tcc gct gca aac ctc cat tta tta tta tta aga gat agc    720
   Val Tyr Val Ser Ala Ala Asn Leu His Leu Leu Leu Leu Arg Asp Ser
   225                 230                 235                 240
   tcg att ttc ggt ttg gat tgg gga tta agt caa act cat gtt aac gat    768
   Ser Ile Phe Gly Leu Asp Trp Gly Leu Ser Gln Thr His Val Asn Asp
                   245                 250                 255
   aat tat aat ctc caa ata agg cgc tct gca gat tat gca aat cat tgt    816
   Asn Tyr Asn Leu Gln Ile Arg Arg Ser Ala Asp Tyr Ala Asn His Cys
               260                 265                 270
   aca act tgg tat cgg acg ggt tta caa aga ttg caa ggc acc aat gct    864
   Thr Thr Trp Tyr Arg Thr Gly Leu Gln Arg Leu Gln Gly Thr Asn Ala
           275                 280                 285
   agc agt tgg gtc aat tat aat cga ttt aga aga gaa atg aca cta act    912
   Ser Ser Trp Val Asn Tyr Asn Arg Phe Arg Arg Glu Met Thr Leu Thr
       290                 295                 300
   gta tta gat gtt tgt gca tta ttt tca agt tat gat tat cgt agt tac    960
   Val Leu Asp Val Cys Ala Leu Phe Ser Ser Tyr Asp Tyr Arg Ser Tyr
   305                 310                 315                 320
   cca atg gag cta agg gga gag ctt acg aga gaa att tat acg gat cca    1008
   Pro Met Glu Leu Arg Gly Glu Leu Thr Arg Glu Ile Tyr Thr Asp Pro
                   325                 330                 335
   gta gga gcc tct ttt tgg gtg aat aga gca cca aac ttc gca tca ata    1056
   Val Gly Ala Ser Phe Trp Val Asn Arg Ala Pro Asn Phe Ala Ser Ile
               340                 345                 350
   gaa aat aca gta gtt agg caa cca cac ccc ttt act tgg cta gtt act    1104
   Glu Asn Thr Val Val Arg Gln Pro His Pro Phe Thr Trp Leu Val Thr
           355                 360                 365
   tta aca gtt aat aca ggt caa gtg aga tct ggc gat gga aat tct aac    1152
   Leu Thr Val Asn Thr Gly Gln Val Arg Ser Gly Asp Gly Asn Ser Asn
       370                 375                 380
   tat tat tgg aaa tca cat agt caa acc gtg agt gaa acc gga ggg tca    1200
   Tyr Tyr Trp Lys Ser His Ser Gln Thr Val Ser Glu Thr Gly Gly Ser
   385                 390                 395                 400
   ggt cct att cag agt cct acc tgt gga agt act ggt aca att tat cgc    1248
   Gly Pro Ile Gln Ser Pro Thr Cys Gly Ser Thr Gly Thr Ile Tyr Arg
                   405                 410                 415
   acg gat aat tta ctt ttt aat cca ttt tta tta ggt gat att tat acc    1296
   Thr Asp Asn Leu Leu Phe Asn Pro Phe Leu Leu Gly Asp Ile Tyr Thr
               420                 425                 430
   att aat aca ggt tat gtt tct tat ctg gct aat ttg ttt gga atc tat    1344
   Ile Asn Thr Gly Tyr Val Ser Tyr Leu Ala Asn Leu Phe Gly Ile Tyr
           435                 440                 445
   tca gct aga ttt acg acg act cgt tct att gag ctt ctg tat gag aac    1392
   Ser Ala Arg Phe Thr Thr Thr Arg Ser Ile Glu Leu Leu Tyr Glu Asn
       450                 455                 460
   caa aga gtt ttc cca gcc tac aat cat caa att cgt gaa tta cct gga    1440
   Gln Arg Val Phe Pro Ala Tyr Asn His Gln Ile Arg Glu Leu Pro Gly
   465                 470                 475                 480
   gta aac tcg gat agg cca act gct gcc gac tat agt cat aga cta tcg    1488
   Val Asn Ser Asp Arg Pro Thr Ala Ala Asp Tyr Ser His Arg Leu Ser
                   485                 490                 495
   tat atc tca ggt ttt gca act gat gtg gga gga acg gtt cta gtt tat    1536
   Tyr Ile Ser Gly Phe Ala Thr Asp Val Gly Gly Thr Val Leu Val Tyr
               500                 505                 510
   ggg tgg aca tct tca act gct act cgt gag aat aat att acg cta gac    1584
   Gly Trp Thr Ser Ser Thr Ala Thr Arg Glu Asn Asn Ile Thr Leu Asp
           515                 520                 525
   gac aga ata gta caa ctt cca gct gtt aag gga aca agt ctc aac aat    1632
   Asp Arg Ile Val Gln Leu Pro Ala Val Lys Gly Thr Ser Leu Asn Asn
       530                 535                 540
   tgc caa gta gtt aga gga act gga ttt aca gga gga gac tgg ttg aag    1680
   Cys Gln Val Val Arg Gly Thr Gly Phe Thr Gly Gly Asp Trp Leu Lys
   545                 550                 555                 560
   cct aat aat aat ggt aca ttt tct cta gct ctt ggt ttc agg tcg act    1728
   Pro Asn Asn Asn Gly Thr Phe Ser Leu Ala Leu Gly Phe Arg Ser Thr
                   565                 570                 575
   tac act tac cgc ctc cgc att cgt tat gct gcc gca gca ggc gga tca    1776
   Tyr Thr Tyr Arg Leu Arg Ile Arg Tyr Ala Ala Ala Ala Gly Gly Ser
               580                 585                 590
   ggt ttt tct ctt gtt ata tcg gat caa tat gga gaa ttt cca acc aca    1824
   Gly Phe Ser Leu Val Ile Ser Asp Gln Tyr Gly Glu Phe Pro Thr Thr
           595                 600                 605
   aca gta tcg ctt tcc tcc aca atg tac tca ctg ccc caa aat gta cca    1872
   Thr Val Ser Leu Ser Ser Thr Met Tyr Ser Leu Pro Gln Asn Val Pro
       610                 615                 620
   tac gag gct ttt aag att gta gat tta cct tct act gtt act att aga    1920
   Tyr Glu Ala Phe Lys Ile Val Asp Leu Pro Ser Thr Val Thr Ile Arg
   625                 630                 635                 640
   aat act tct cct gct tca aca act ttt cga ctt gat ttc cgt ttc att    1968
   Asn Thr Ser Pro Ala Ser Thr Thr Phe Arg Leu Asp Phe Arg Phe Ile
                   645                 650                 655
   gtg cca tta gga ata ctc gca aat ata tta att gac cga att gaa ttt    2016
   Val Pro Leu Gly Ile Leu Ala Asn Ile Leu Ile Asp Arg Ile Glu Phe
               660                 665                 670
   gtt ccc att gag ggt tcc ttg ttc gag tac gaa acc aaa cag cag cta    2064
   Val Pro Ile Glu Gly Ser Leu Phe Glu Tyr Glu Thr Lys Gln Gln Leu
           675                 680                 685
   gaa aaa gca agg aaa gcg gtg aac cat ttg ttt aca gat gga tcg aaa    2112
   Glu Lys Ala Arg Lys Ala Val Asn His Leu Phe Thr Asp Gly Ser Lys
       690                 695                 700
   aag gcg cta aaa gaa ggc acg aca gat tat gag atc gat caa gcc gcc    2160
   Lys Ala Leu Lys Glu Gly Thr Thr Asp Tyr Glu Ile Asp Gln Ala Ala
   705                 710                 715                 720
   aac gtg gtg gat tgt ata tcg gat gag tgt gga cat gag aaa atg atc    2208
   Asn Val Val Asp Cys Ile Ser Asp Glu Cys Gly His Glu Lys Met Ile
                   725                 730                 735
   ctg ttg gat gaa gtg aaa tat gca aaa caa ctc agc caa gcc cgc aat    2256
   Leu Leu Asp Glu Val Lys Tyr Ala Lys Gln Leu Ser Gln Ala Arg Asn
               740                 745                 750
   tta ctg ctc aat ggg aat ttc gat gat cta tat cca gct ctg gag agg    2304
   Leu Leu Leu Asn Gly Asn Phe Asp Asp Leu Tyr Pro Ala Leu Glu Arg
           755                 760                 765
   gag aat cca tgg aaa aca agc ccg aat gtt acg atc cgt caa gat aac    2352
   Glu Asn Pro Trp Lys Thr Ser Pro Asn Val Thr Ile Arg Gln Asp Asn
       770                 775                 780
   ccg att ttt aaa ggc cat tat ctc agt atg gcg ggt gcg aac gat atc    2400
   Pro Ile Phe Lys Gly His Tyr Leu Ser Met Ala Gly Ala Asn Asp Ile
   785                 790                 795                 800
   gag gcc acc aat gat acc ttc ccc acg tat gcc tat caa aaa ata gac    2448
   Glu Ala Thr Asn Asp Thr Phe Pro Thr Tyr Ala Tyr Gln Lys Ile Asp
                   805                 810                 815
   gaa gcc aaa tta aag ccg tat aca cgt tat aaa gtg cgc ggg ttt gtt    2496
   Glu Ala Lys Leu Lys Pro Tyr Thr Arg Tyr Lys Val Arg Gly Phe Val
               820                 825                 830
   ggc agc agc aaa gct cta gag ctg ttg gtt aca cgc tat aat gaa gaa    2544
   Gly Ser Ser Lys Ala Leu Glu Leu Leu Val Thr Arg Tyr Asn Glu Glu
           835                 840                 845
   gtc gat gcg att tta gat gta ccg gat aat atc ccg cat gcg ccg ata    2592
   Val Asp Ala Ile Leu Asp Val Pro Asp Asn Ile Pro His Ala Pro Ile
       850                 855                 860
   cct gtc tgc ggt gaa ttt gat cga tgc aag ccc tat tcg tat cca cct    2640
   Pro Val Cys Gly Glu Phe Asp Arg Cys Lys Pro Tyr Ser Tyr Pro Pro
   865                 870                 875                 880
   tta ctt cca gaa tgt aac cct gag ttt ata aat cag atg caa cca tcc    2688
   Leu Leu Pro Glu Cys Asn Pro Glu Phe Ile Asn Gln Met Gln Pro Ser
                   885                 890                 895
   tct tgc cac cac act cag atg gtc gat tac aat aac atg aac atg agc    2736
   Ser Cys His His Thr Gln Met Val Asp Tyr Asn Asn Met Asn Met Ser
               900                 905                 910
   acg agt act acc atg aat cct acc ctt acg cct gaa ata gca tcc agc    2784
   Thr Ser Thr Thr Met Asn Pro Thr Leu Thr Pro Glu Ile Ala Ser Ser
           915                 920                 925
   caa agt gga ttc ggc aga aaa cat cgc aaa tgt cat caa gcg cat caa    2832
   Gln Ser Gly Phe Gly Arg Lys His Arg Lys Cys His Gln Ala His Gln
       930                 935                 940
   ttt gag ttc cat att gat acc ggg aca atc gat ctg gtc gaa gat ttg    2880
   Phe Glu Phe His Ile Asp Thr Gly Thr Ile Asp Leu Val Glu Asp Leu
   945                 950                 955                 960
   ggc att tgg gtg atc ttc aaa atc tgt gcc aca gat ggt tac gca agc    2928
   Gly Ile Trp Val Ile Phe Lys Ile Cys Ala Thr Asp Gly Tyr Ala Ser
                   965                 970                 975
   tta gat gat ttg gaa gtg att gaa gaa gga gcg ctg ggt gtc gaa gca    2976
   Leu Asp Asp Leu Glu Val Ile Glu Glu Gly Ala Leu Gly Val Glu Ala
               980                 985                 990
   tta gaa ctt gtc aag aaa aga gaa aag aaa tgg aga cat cag aag gag    3024
   Leu Glu Leu Val Lys Lys Arg Glu Lys Lys Trp Arg His Gln Lys Glu
           995                 1000                1005
   cag cac tgt tcg caa acg aaa cac aaa tat gat gcg gcc aaa cat gcg    3072
   Gln His Cys Ser Gln Thr Lys His Lys Tyr Asp Ala Ala Lys His Ala
       1010                1015                1020
   gtg atg gcg tta ttt aca aac acg cgc tat gaa aaa ttg aag ttc gaa    3120
   Val Met Ala Leu Phe Thr Asn Thr Arg Tyr Glu Lys Leu Lys Phe Glu
   1025                1030                1035                1040
   aca acc att tct gac att ttg tat gct gat cat ctc gtg cag tcg atc    3168
   Thr Thr Ile Ser Asp Ile Leu Tyr Ala Asp His Leu Val Gln Ser Ile
                   1045                1050                1055
   cct tat gta tat aat aaa tat gta ccg gaa gtt cca ggt atg aat tac    3216
   Pro Tyr Val Tyr Asn Lys Tyr Val Pro Glu Val Pro Gly Met Asn Tyr
               1060                1065                1070
   gaa ctc tat tca gag cta aac aca ctg gtt cag aat gcg ttc tac ctg    3264
   Glu Leu Tyr Ser Glu Leu Asn Thr Leu Val Gln Asn Ala Phe Tyr Leu
           1075                1080                1085
   tat gac cag cgg aat ctg att aaa aat ggg cgc ttt agc aat ggg ctt    3312
   Tyr Asp Gln Arg Asn Leu Ile Lys Asn Gly Arg Phe Ser Asn Gly Leu
       1090                1095                1100
   atg cat tgg caa gct act cct cat gca aga gta gag caa gaa cat gag    3360
   Met His Trp Gln Ala Thr Pro His Ala Arg Val Glu Gln Glu His Glu
   1105                1110                1115                1120
   aaa tcg gtg ctc gtg ctg cca aat tgg gat gcc aat gtg tcg caa gat    3408
   Lys Ser Val Leu Val Leu Pro Asn Trp Asp Ala Asn Val Ser Gln Asp
                   1125                1130                1135
   ctt tgt atc gaa cac aat cgc ggt tat gta ttg cgt gtc acg gcg aga    3456
   Leu Cys Ile Glu His Asn Arg Gly Tyr Val Leu Arg Val Thr Ala Arg
               1140                1145                1150
   aaa gaa gat ccg gga gcc ggc aat gtt acc ttt agt gac tgt gca aat    3504
   Lys Glu Asp Pro Gly Ala Gly Asn Val Thr Phe Ser Asp Cys Ala Asn
           1155                1160                1165
   cat gtc aac aag ctg agc ttt act tct tgc gat ata gct aca aac gca    3552
   His Val Asn Lys Leu Ser Phe Thr Ser Cys Asp Ile Ala Thr Asn Ala
       1170                1175                1180
   gtg cca ggt gcc caa gcg aat gat cca gcc gcc gga gta gcc tat gga    3600
   Val Pro Gly Ala Gln Ala Asn Asp Pro Ala Ala Gly Val Ala Tyr Gly
   1185                1190                1195                1200
   caa cag ggt tgt caa ata gat aga gtg ccg tac ggg caa tct gga tat    3648
   Gln Gln Gly Cys Gln Ile Asp Arg Val Pro Tyr Gly Gln Ser Gly Tyr
                   1205                1210                1215
   aga aca gac gga aca aat gga atg ccg tac ggg cag tct ggt aat cga    3696
   Arg Thr Asp Gly Thr Asn Gly Met Pro Tyr Gly Gln Ser Gly Asn Arg
               1220                1225                1230
   gcg gac ggg gtg cca tac aga caa tcc gga tat gga aca gat gga gta    3744
   Ala Asp Gly Val Pro Tyr Arg Gln Ser Gly Tyr Gly Thr Asp Gly Val
           1235                1240                1245
   gcg cac gac caa cct gga tat cga gca gac gga gca gcg tac gaa cag    3792
   Ala His Asp Gln Pro Gly Tyr Arg Ala Asp Gly Ala Ala Tyr Glu Gln
       1250                1255                1260
   tct ggt cat cga gca gac gga gta gcg tac gaa caa tct gga tat cga    3840
   Ser Gly His Arg Ala Asp Gly Val Ala Tyr Glu Gln Ser Gly Tyr Arg
   1265                1270                1275                1280
   gca ggt gga gta gcg tac gaa cag tct ggt cat cga gca gat gga gtg    3888
   Ala Gly Gly Val Ala Tyr Glu Gln Ser Gly His Arg Ala Asp Gly Val
                   1285                1290                1295
   ccg tac gga caa tct gga tat gga aca gac gga gta acg tac gac caa    3936
   Pro Tyr Gly Gln Ser Gly Tyr Gly Thr Asp Gly Val Thr Tyr Asp Gln
               1300                1305                1310
   tct gtc aaa caa acc cgc aaa tac cat ggt tgc cat aca gac ggg ctg    3984
   Ser Val Lys Gln Thr Arg Lys Tyr His Gly Cys His Thr Asp Gly Leu
           1315                1320                1325
   cca cat cca gag cat ggt tgt tgt tat cca gac aga gta agc gat ggc    4032
   Pro His Pro Glu His Gly Cys Cys Tyr Pro Asp Arg Val Ser Asp Gly
       1330                1335                1340
   caa cag ctt gca tat gta aca aaa tcg att gat ctg ttc ccg gat aca    4080
   Gln Gln Leu Ala Tyr Val Thr Lys Ser Ile Asp Leu Phe Pro Asp Thr
   1345                1350                1355                1360
   gat aaa gtc cgg atc gac att gga gaa acc gaa ggg aac ttt aga gtg    4128
   Asp Lys Val Arg Ile Asp Ile Gly Glu Thr Glu Gly Asn Phe Arg Val
                   1365                1370                1375
   gaa agt gtg gaa ttg att tgt atg gaa aag taaatcatca caagtaaaag      4178
   Glu Ser Val Glu Leu Ile Cys Met Glu Lys
               1380                1385
   tatcgtttac                                                         4188
<210> 6
   <211> 1386
   <212> PRT
   <213> Bacillus popilliae
<400> 6
<210> 7
   <211> 795
   <212> DNA
   <213> Bacillus popilliae
<220>
   <221> CDS
   <222> (1)..(795)
<400> 7
   caa tat cat aac caa aac gat aac aaa agt tac aac caa agt gga aat     48
   Gln Tyr His Asn Gln Asn Asp Asn Lys Ser Tyr Asn Gln Ser Gly Asn
    1               5                  10                  15
   gac atg caa att ata caa cct tca agt aac gct tta ctt tac agt ccc     96
   Asp Met Gln Ile Ile Gln Pro Ser Ser Asn Ala Leu Leu Tyr Ser Pro
               20                  25                  30
   aat aag tat ccg tat gcc acg gat ccc aat gtc ata gca gag ggg aga    144
   Asn Lys Tyr Pro Tyr Ala Thr Asp Pro Asn Val Ile Ala Glu Gly Arg
           35                  40                  45
   agt tat aat aat tgg ttg gat acg tgt gta ggt gta ggc gat ggt act    192
   Ser Tyr Asn Asn Trp Leu Asp Thr Cys Val Gly Val Gly Asp Gly Thr
       50                  55                  60
   cga agt ccc gag gct tat gct att gcc gaa gag gct gtt ggt ctt tcg    240
   Arg Ser Pro Glu Ala Tyr Ala Ile Ala Glu Glu Ala Val Gly Leu Ser
   65                  70                  75                  80
   att gat ctt ctc gcc gaa act ata tac ttt cta ggc ttt ccg att gca    288
   Ile Asp Leu Leu Ala Glu Thr Ile Tyr Phe Leu Gly Phe Pro Ile Ala
                   85                  90                  95
   tct cca atc act cgt gca cta tct gct tta ctg ggg ggg cta ttt tct    336
   Ser Pro Ile Thr Arg Ala Leu Ser Ala Leu Leu Gly Gly Leu Phe Ser
               100                 105                 110
   tct ggg gat acg ctg atg caa cat gta gaa caa ctc ata aat caa aaa    384
   Ser Gly Asp Thr Leu Met Gln His Val Glu Gln Leu Ile Asn Gln Lys
           115                 120                 125
   ata gaa att tat gca aga aat acg gca ctt gca gaa tta ttg ggt tta    432
   Ile Glu Ile Tyr Ala Arg Asn Thr Ala Leu Ala Glu Leu Leu Gly Leu
       130                 135                 140
   cgt aat gca tta gaa gtc tat agt gtg gct tta gaa tat tgg caa caa    480
   Arg Asn Ala Leu Glu Val Tyr Ser Val Ala Leu Glu Tyr Trp Gln Gln
   145                 150                 155                 160
   aat cgt aat agt gca caa gct caa gaa agt gta aga tca aga ttc cgt    528
   Asn Arg Asn Ser Ala Gln Ala Gln Glu Ser Val Arg Ser Arg Phe Arg
                   165                 170                 175
   agt cta gaa act ata ttt ata caa agg atg cct tta ttt gca ata caa    576
   Ser Leu Glu Thr Ile Phe Ile Gln Arg Met Pro Leu Phe Ala Ile Gln
               180                 185                 190
   ggt tat gag gtg ccg tta tta tcc gtg tat gca gcc gct gca aat ctc    624
   Gly Tyr Glu Val Pro Leu Leu Ser Val Tyr Ala Ala Ala Ala Asn Leu
           195                 200                 205
   cat tta cta tta tta agg gat agc tct att cac ggt tta gac tgg gga    672
   His Leu Leu Leu Leu Arg Asp Ser Ser Ile His Gly Leu Asp Trp Gly
       210                 215                 220
   ttt gat caa gga gag gtt aac agt aat tac gat cgc caa att agg ctc    720
   Phe Asp Gln Gly Glu Val Asn Ser Asn Tyr Asp Arg Gln Ile Arg Leu
   225                 230                 235                 240
   tct gca gag tat gca aat cat tgt ata act tgg tat cag gcg ggt tta    768
   Ser Ala Glu Tyr Ala Asn His Cys Ile Thr Trp Tyr Gln Ala Gly Leu
                   245                 250                 255
   caa gga ttg cag ggc acc agg ggg cga                                795
   Gln Gly Leu Gln Gly Thr Arg Gly Arg
               260                 265
<210> 8
   <211> 265
   <212> PRT
   <213> Bacillus popilliae
<400> 8
   Gln Tyr His Asn Gln Asn Asp Asn Lys Ser Tyr Asn Gln Ser Gly Asn
    1               5                  10                  15
   Asp Met Gln Ile Ile Gln Pro Ser Ser Asn Ala Leu Leu Tyr Ser Pro
               20                  25                  30
   Asn Lys Tyr Pro Tyr Ala Thr Asp Pro Asn Val Ile Ala Glu Gly Arg
           35                  40                  45
   Ser Tyr Asn Asn Trp Leu Asp Thr Cys Val Gly Val Gly Asp Gly Thr
       50                  55                  60
   Arg Ser Pro Glu Ala Tyr Ala Ile Ala Glu Glu Ala Val Gly Leu Ser
   65                  70                  75                  80
   Ile Asp Leu Leu Ala Glu Thr Ile Tyr Phe Leu Gly Phe Pro Ile Ala
                   85                  90                  95
   Ser Pro Ile Thr Arg Ala Leu Ser Ala Leu Leu Gly Gly Leu Phe Ser
               100                 105                 110
   Ser Gly Asp Thr Leu Met Gln His Val Glu Gln Leu Ile Asn Gln Lys
           115                 120                 125
   Ile Glu Ile Tyr Ala Arg Asn Thr Ala Leu Ala Glu Leu Leu Gly Leu
       130                 135                 140
   Arg Asn Ala Leu Glu Val Tyr Ser Val Ala Leu Glu Tyr Trp Gln Gln
   145                 150                 155                 160
   Asn Arg Asn Ser Ala Gln Ala Gln Glu Ser Val Arg Ser Arg Phe Arg
                   165                 170                 175
   Ser Leu Glu Thr Ile Phe Ile Gln Arg Met Pro Leu Phe Ala Ile Gln
               180                 185                 190
   Gly Tyr Glu Val Pro Leu Leu Ser Val Tyr Ala Ala Ala Ala Asn Leu
           195                 200                 205
   His Leu Leu Leu Leu Arg Asp Ser Ser Ile His Gly Leu Asp Trp Gly
       210                 215                 220
   Phe Asp Gln Gly Glu Val Asn Ser Asn Tyr Asp Arg Gln Ile Arg Leu
   225                 230                 235                 240
   Ser Ala Glu Tyr Ala Asn His Cys Ile Thr Trp Tyr Gln Ala Gly Leu
                   245                 250                 255
   Gln Gly Leu Gln Gly Thr Arg Gly Arg
               260                 265
<210> 9
   <211> 459
   <212> DNA
   <213> Bacillus popilliae
<220>
   <221> CDS
   <222> (1)..(459)
<400> 9
   tat gag gtc cca tta tta tcc gtg tat gca gac gct gca aat ctc cat      48
   Tyr Glu Val Pro Leu Leu Ser Val Tyr Ala Asp Ala Ala Asn Leu His
    1               5                  10                  15
   ttg cta ata tta agg gac agc tat att tac ggt gca ttc tgg ggg ttt      96
   Leu Leu Ile Leu Arg Asp Ser Tyr Ile Tyr Gly Ala Phe Trp Gly Phe
               20                  25                  30
   gat gaa gat gaa tat tac cgt aat tac gct cgc caa att agg ctc tcc     144
   Asp Glu Asp Glu Tyr Tyr Arg Asn Tyr Ala Arg Gln Ile Arg Leu Ser
           35                  40                  45
   gca gag tat gca aat cat tgt aca act tgg tat cag acg ggt tta cga     192
   Ala Glu Tyr Ala Asn His Cys Thr Thr Trp Tyr Gln Thr Gly Leu Arg
   aga ttg cag ggc acc cga gct aca gat tgg atc aat tat aat cga ttt     240
   Arg Leu Gln Gly Thr Arg Ala Thr Asp Trp Ile Asn Tyr Asn Arg Phe
   65                  70                  75                  80
   aga aga gaa ctg aca cta aca gta tta gat att tgt gca tta ttt tct     288
   Arg Arg Glu Leu Thr Leu Thr Val Leu Asp Ile Cys Ala Leu Phe Ser
                   85                  90                  95
   agc tat gat att cct agt tac ccg atg ggg aca aag ata cag ctt acg     336
   Ser Tyr Asp Ile Pro Ser Tyr Pro Met Gly Thr Lys Ile Gln Leu Thr
               100                 105                 110
   aga gaa att tat acc gat cca gta gta cac tct gac tgg ttg caa tca     384
   Arg Glu Ile Tyr Thr Asp Pro Val Val His Ser Asp Trp Leu Gln Ser
           115                 120                 125
   acg agt ccg gga tta ata tca ttc tca tca cta gaa aat cta gtc gtt     432
   Thr Ser Pro Gly Leu Ile Ser Phe Ser Ser Leu Glu Asn Leu Val Val
       130                 135                 140
   cgg gca cca cat ctt ttt act tgg ctt                                 459
   Arg Ala Pro His Leu Phe Thr Trp Leu
   145                 150
<210> 10
   <211> 153
   <212> PRT
<213> Bacillus popilliae
<400> 10
   Tyr Glu Val Pro Leu Leu Ser Val Tyr Ala Asp Ala Ala Asn Leu His
    1               5                  10                  15
   Leu Leu Ile Leu Arg Asp Ser Tyr Ile Tyr Gly Ala Phe Trp Gly Phe
                   20                  25                  30
   Asp Glu Asp Glu Tyr Tyr Arg Asn Tyr Ala Arg Gln Ile Arg Leu Ser
   Ala Glu Tyr Ala Asn His Cys Thr Thr Trp Tyr Gln Thr Gly Leu Arg
           50                  55                  60
   Arg Leu Gln Gly Thr Arg Ala Thr Asp Trp Ile Asn Tyr Asn Arg Phe
       65                  70                  75                  80
   Arg Arg Glu Leu Thr Leu Thr Val Leu Asp Ile Cys Ala Leu Phe Ser
                   85                  90                  95
   Ser Tyr Asp Ile Pro Ser Tyr Pro Met Gly Thr Lys Ile Gln Leu Thr
               100                 105                 110
   Arg Glu Ile Tyr Thr Asp Pro Val Val His Ser Asp Trp Leu Gln Ser
           115                 120                 125
   Thr Ser Pro Gly Leu Ile Ser Phe Ser Ser Leu Glu Asn Leu Val Val
       130                 135                 140
   Arg Ala Pro His Leu Phe Thr Trp Leu
   145                 150
<210> 11
   <211> 25
   <212> DNA
   <213> Artificial Sequence
<220>
   <223> Description of Artificial Sequence: primer
<220>
   <221> misc_feature
   <222> (5,11,12)
   <223> n=a or g or c or t
<400> 11
   atgcngaagt nnatttacac gtgtt 25
<210> 12
   <211> 17
   <212> DNA
   <213> Artificial Sequence
<220>
   <223> Description of Artificial Sequence: primer
<220>
   <221> misc_feature
   <222> (14,15)
   <223> n=a or g or c or t
<400> 12
   atgttgtggc taannac 17
<210> 13
   <211> 20
   <212> DNA
   <213> Artificial Sequence
<220>
   <223> Description of Artificial Sequence: primer
<400> 13
   gtaaacgata cttttacttg 20
<210> 14
   <211> 20
   <212> DNA
   <213> Artificial Sequence
<220>
   <223> Description of Artificial Sequence: primer
<400> 14
   tctcgatcac gaatgatcat 20
<210> 15
   <211> 20
   <212> DNA
   <213> Artificial Sequence
<220>
   <223> Description of Artificial Sequence: primer
<400> 15
   gtgtcatttc tcttctaaat 20
<210> 16
   <211> 30
   <212> DNA
   <213> Artificial Sequence
<220>
   <223> Description of Artificial Sequence: primer
<400> 16
   gtggaacaag aatatgagaa atctgtactc 30
<210> 17
   <211> 4359
   <212> DNA
   <213> Bacillus popilliae
<220>
   <221> CDS
   <222> (250)..(4245)
<400> 17
   ttgccagatg ttcatatcga ttgtcaatat gtgacggtat gtgatttaca aatgatgccc   60
   gttcatgagg gagcttgtca atttgtgaag attagcggag aatttcaatt ttattcactt  120
   taacattagc tacaaatgtt gtggctaagc accccattat ttatcaacgg gtaacaacca  180
   ggaggggtaa ctttgaatca gtatcaacat caaaacgata acaaaagtta caatcaagat  240
   ggaaatgaa atg cag atc ata caa cct tca agt aac gct tta ctt tac agt  291
             Met Gln Ile Ile Gln Pro Ser Ser Asn Ala Leu Leu Tyr Ser
             1               5                   10
   ccc aat aag tat ccg tat gcc acg gat ccc aat gtc ata gca gag ggg    339
   Pro Asn Lys Tyr Pro Tyr Ala Thr Asp Pro Asn Val Ile Ala Glu Gly
   15                  20                  25                  30
   aga agt tat aat aat tgg ttg gat acg tgt gta ggt gta ggc gat ggt    387
   Arg Ser Tyr Asn Asn Trp Leu Asp Thr Cys Val Gly Val Gly Asp Gly
                   35                  40                  45
   act cga agt ccc gag gct tat gct att gcc gaa gag gct gtc ggt ctt    435
   Thr Arg Ser Pro Glu Ala Tyr Ala Ile Ala Glu Glu Ala Val Gly Leu
               50                  55                  60
   tca att gat ata ttg gcc gaa att ata tac tat cta ggc ttc ccg att      483
   Ser Ile Asp Ile Leu Ala Glu Ile Ile Tyr Tyr Leu Gly Phe Pro Ile
           65                  70                  75
   gca tct cca ctc act cgt gca cta agt gct ata gcg gga cag cta ttt      531
   Ala Ser Pro Leu Thr Arg Ala Leu Ser Ala Ile Ala Gly Gln Leu Phe
       80                  85                  90
   tct tct ggg gat acg ctc atg caa cat att gaa caa ctc ata aat caa      579
   Ser Ser Gly Asp Thr Leu Met Gln His Ile Glu Gln Leu Ile Asn Gln
   95                  100                 105                 110
   aaa ata gcg gaa tat gcc aga aat aag gcg ctt gca gaa ttt cag ggt      627
   Lys Ile Ala Glu Tyr Ala Arg Asn Lys Ala Leu Ala Glu Phe Gln Gly
                   115                 120                 125
   tta ggt aga caa tat ggg tta tat tta gag gct tta gaa gat tgg gaa      675
   Leu Gly Arg Gln Tyr Gly Leu Tyr Leu Glu Ala Leu Glu Asp Trp Glu
               130                 135                 140
   caa aat cgt ctt agt caa cca cat aaa gag cgt gta aga caa aca ttc      723
   Gln Asn Arg Leu Ser Gln Pro His Lys Glu Arg Val Arg Gln Thr Phe
           145                 150                 155
   cgt att ctt gat aat agc ttt aca tca tct ata cct tca ttt gca gta      771
   Arg Ile Leu Asp Asn Ser Phe Thr Ser Ser Ile Pro Ser Phe Ala Val
       160                 165                 170
   cga aat tat gag gtt cca tta tta tcc gtg tat gca gac gct gca aat      819
   Arg Asn Tyr Glu Val Pro Leu Leu Ser Val Tyr Ala Asp Ala Ala Asn
   175                 180                 185                 190
   ctc cat ttg cta ata tta agg gac agc tat att tac ggt gca ttc tgg      867
   Leu His Leu Leu Ile Leu Arg Asp Ser Tyr Ile Tyr Gly Ala Phe Trp
                   195                 200                 205
   ggg ttt gat gaa gat gaa tat tac cgt aat tac gct cgc caa att agg      915
   Gly Phe Asp Glu Asp Glu Tyr Tyr Arg Asn Tyr Ala Arg Gln Ile Arg
               210                 215                 220
   ctc tcc gca gag tat gca aat cat tgt aca act tgg tat cag acg ggt      963
   Leu Ser Ala Glu Tyr Ala Asn His Cys Thr Thr Trp Tyr Gln Thr Gly
           225                 230                 235
   tta cga aga ttg cag ggc acc cga gct aca gat tgg atc aat tat aat      1011
   Leu Arg Arg Leu Gln Gly Thr Arg Ala Thr Asp Trp Ile Asn Tyr Asn
       240                 245                 250
   cga ttt aga aga gaa atg aca cta aca gta tta gat att tgt gca tta      1059
   Arg Phe Arg Arg Glu Met Thr Leu Thr Val Leu Asp Ile Cys Ala Leu
   255                 260                 265                 270
   ttt tct agc tat gat att cct agt tac ccg atg ggg aca aag ata cag      1107
   Phe Ser Ser Tyr Asp Ile Pro Ser Tyr Pro Met Gly Thr Lys Ile Gln
                   275                 280                 285
   ctt acg aga gaa att tat acc gat cca gta gta cac tct gac tgg ttg      1155
   Leu Thr Arg Glu Ile Tyr Thr Asp Pro Val Val His Ser Asp Trp Leu
               290                 295                 300
   caa tca acg agt ccg gga tta ata tca ttc tca tca cta gaa aat cta      1203
   Gln Ser Thr Ser Pro Gly Leu Ile Ser Phe Ser Ser Leu Glu Asn Leu
           305                 310                 315
   gtc gtt cgg gca cca cat ctt ttt act tgg ctt tct cga gta aca att      1251
   Val Val Arg Ala Pro His Leu Phe Thr Trp Leu Ser Arg Val Thr Ile
       320                 325                 330
   gat aca ggt ata ttg tct act gta att ggc ggg cag tat agt aat aac      1299
   Asp Thr Gly Ile Leu Ser Thr Val Ile Gly Gly Gln Tyr Ser Asn Asn
   335                 340                 345                 350
   aat ttt tgg cga aca cat tat caa act ttg cgt aca acc ggg ggc aca      1347
   Asn Phe Trp Arg Thr His Tyr Gln Thr Leu Arg Thr Thr Gly Gly Thr
                   355                 360                 365
   tct ttc caa agt cct acc tat ggc tcg aca gcg ttt cca att caa cgc      1395
   Ser Phe Gln Ser Pro Thr Tyr Gly Ser Thr Ala Phe Pro Ile Gln Arg
               370                 375                 380
   acg aat aca ttg act ttc tcc ggc gat gtt tac acc ata gag tca agt      1443
   Thr Asn Thr Leu Thr Phe Ser Gly Asp Val Tyr Thr Ile Glu Ser Ser
           385                 390                 395
   gtt gtt acg agg agt tcc ttg tat gga gct aat tcg gtt gca ttt acg      1491
   Val Val Thr Arg Ser Ser Leu Tyr Gly Ala Asn Ser Val Ala Phe Thr
       400                 405                 410
   ggt act act ggt cgg tca cta tat gag aac cca acg gtt tat ccg ttt      1539
   Gly Thr Thr Gly Arg Ser Leu Tyr Glu Asn Pro Thr Val Tyr Pro Phe
   415                 420                 425                 430
   gca caa aaa tta att cat gaa tta cct gga gta gac tca ggt aga cca      1587
   Ala Gln Lys Leu Ile His Glu Leu Pro Gly Val Asp Ser Gly Arg Pro
                   435                 440                 445
   aat gct acc aac tat agt cat aga ctg tcg tat atc tca ggt ttc agt      1635
   Asn Ala Thr Asn Tyr Ser His Arg Leu Ser Tyr Ile Ser Gly Phe Ser
               450                 455                 460
   ctt ggt tat tct cct tct gga acg ggt cta gtt tat ggg tgg aca tct      1683
   Leu Gly Tyr Ser Pro Ser Gly Thr Gly Leu Val Tyr Gly Trp Thr Ser
           465                 470                 475
   aca act gct act cgt gag aat aat att acg cta gac gac aga ata gta      1731
   Thr Thr Ala Thr Arg Glu Asn Asn Ile Thr Leu Asp Asp Arg Ile Val
       480                 485                 490
   cag ctt cca gct gtt aag gga gca agt ctc aat aat tgc caa gta gta      1779
   Gln Leu Pro Ala Val Lys Gly Ala Ser Leu Asn Asn Cys Gln Val Val
   495                 500                 505                 510
   aaa ggg act gga ttt aca gga gga gac tgg ttg aaa cct aat aat aat      1827
   Lys Gly Thr Gly Phe Thr Gly Gly Asp Trp Leu Lys Pro Asn Asn Asn
                   515                 520                 525
   ggt aca ttt tct atg tac ttt gca ttc agg tcg gct tac act tac cac      1875
   Gly Thr Phe Ser Met Tyr Phe Ala Phe Arg Ser Ala Tyr Thr Tyr His
               530                 535                 540
   ttc cgc att cgt tat gct tcc tca gca agt ttt tct ttt gtt ata tcg      1923
   Phe Arg Ile Arg Tyr Ala Ser Ser Ala Ser Phe Ser Phe Val Ile Ser
           545                 550                 555
   gaa gaa tat gga cgt ttt cca acc aca aca gtg ccg ctt ctc tcc aca      1971
   Glu Glu Tyr Gly Arg Phe Pro Thr Thr Thr Val Pro Leu Leu Ser Thr
       560                 565                 570
   atg tca cca ctg ccc caa aat aca cca ttc gaa gct ttt aag act gta      2019
   Met Ser Pro Leu Pro Gln Asn Thr Pro Phe Glu Ala Phe Lys Thr Val
   575                 580                 585                 590
   gat tta cct tct act gtt act att aga tat act tct gct gct tca aca      2067
   Asp Leu Pro Ser Thr Val Thr Ile Arg Tyr Thr Ser Ala Ala Ser Thr
                   595                 600                 605
   act ttt caa ctt aat ttc cgt ttc act gtg cca gga agc gca aat gta      2115
   Thr Phe Gln Leu Asn Phe Arg Phe Thr Val Pro Gly Ser Ala Asn Val
               610                 615                 620
   ttg att gac cga att gaa ttt gtt cca att gag ggt tcc ttg ttc gag      2163
   Leu Ile Asp Arg Ile Glu Phe Val Pro Ile Glu Gly Ser Leu Phe Glu
           625                 630                 635
   tac gaa acc aaa cag cag cta gaa aaa gca agg aaa gcg gtg aac cat      2211
   Tyr Glu Thr Lys Gln Gln Leu Glu Lys Ala Arg Lys Ala Val Asn His
       640                 645                 650
   ttg ttt aca gat gga tcg aaa aag gcg cta aaa gaa gac acg acc gat      2259
   Leu Phe Thr Asp Gly Ser Lys Lys Ala Leu Lys Glu Asp Thr Thr Asp
   655                 660                 665                 670
   tat gag att gat caa gcc gcc aac gtg gta gat tgt ata tcg gat gag      2307
   Tyr Glu Ile Asp Gln Ala Ala Asn Val Val Asp Cys Ile Ser Asp Glu
                   675                 680                 685
   tgt gga cat gag aaa atg atc ctg tta gat gaa gta aaa tat gca aaa      2355
   Cys Gly His Glu Lys Met Ile Leu Leu Asp Glu Val Lys Tyr Ala Lys
               690                 695                 700
   caa ctc agc caa gcc cgc aat tta ctg ctc aat ggg aat ttc gat gat      2403
   Gln Leu Ser Gln Ala Arg Asn Leu Leu Leu Asn Gly Asn Phe Asp Asp
           705                 710                 715
   cta tat cca gct ctg gag agg gag aat cca tgg aaa aca agt ccg aat      2451
   Leu Tyr Pro Ala Leu Glu Arg Glu Asn Pro Trp Lys Thr Ser Pro Asn
       720                 725                 730
   gtt acg atc cgt caa gat aac ccg att ttt aaa ggc cat tat ctc agt      2499
   Val Thr Ile Arg Gln Asp Asn Pro Ile Phe Lys Gly His Tyr Leu Ser
   735                 740                 745                 750
   atg gcg ggt gcg aac gat atc gag gcc acc aat gat acg ttc ccc acg      2547
   Met Ala Gly Ala Asn Asp Ile Glu Ala Thr Asn Asp Thr Phe Pro Thr
                   755                 760                 765
   tat gtc tat caa aaa ata gat gaa gcc aaa tta aag cca tat aca cgg      2595
   Tyr Val Tyr Gln Lys Ile Asp Glu Ala Lys Leu Lys Pro Tyr Thr Arg
               770                 775                 780
   tat aaa gtg cgc ggg ttt gtt ggc agc agc aaa gat ctg gag ctg ttg      2643
   Tyr Lys Val Arg Gly Phe Val Gly Ser Ser Lys Asp Leu Glu Leu Leu
       785                 790                 795
   gtt aca cgc tat aat gaa gaa gtt gat gcg att tta gat gta ccg gat      2691
   Val Thr Arg Tyr Asn Glu Glu Val Asp Ala Ile Leu Asp Val Pro Asp
       800                 805                 810
   aat atc ccg cat gcg ccg act cct gtc tgc ggt gaa ttt gat cga tgc      2739
   Asn Ile Pro His Ala Pro Thr Pro Val Cys Gly Glu Phe Asp Arg Cys
   815                 820                 825                 830
   aag ccc tat tcg tat cca cct tta ctt cca gaa tgt aac cct gag ttt      2787
   Lys Pro Tyr Ser Tyr Pro Pro Leu Leu Pro Glu Cys Asn Pro Glu Phe
                   835                 840                 845
   ata aat cag atg caa cca tcc tct tgc cac cac aat cag atg gtc gat      2835
   Ile Asn Gln Met Gln Pro Ser Ser Cys His His Asn Gln Met Val Asp
               850                 855                 860
   tac aat aac atg aac acg agc acg agt act acc atg aat cct agc atg      2883
   Tyr Asn Asn Met Asn Thr Ser Thr Ser Thr Thr Met Asn Pro Ser Met
       865                 870                 875
   aat cct ccc ctt acg cct gaa ata gca tcc agc caa agt gga ttc ggc      2931
   Asn Pro Pro Leu Thr Pro Glu Ile Ala Ser Ser Gln Ser Gly Phe Gly
       880                 885                 890
   aga aaa cat cgc aaa tgt cat caa gcg cat caa ttt gag ttc cac att      2979
   Arg Lys His Arg Lys Cys His Gln Ala His Gln Phe Glu Phe His Ile
   895                 900                 905                 910
   gat acc ggg aca atc gat ttg gtg gaa gat ttg ggc att tgg gtg atc      3027
   Asp Thr Gly Thr Ile Asp Leu Val Glu Asp Leu Gly Ile Trp Val Ile
                   915                 920                 925
   ttc aaa atc tgt gcc aca gat gga tac gca agc tta gat gat ctg gaa      3075
   Phe Lys Ile Cys Ala Thr Asp Gly Tyr Ala Ser Leu Asp Asp Leu Glu
               930                 935                 940
   gtg att gaa gaa gga gcg ctg ggg gtc gaa gcc tta gaa ctt gtc aag      3123
   Val Ile Glu Glu Gly Ala Leu Gly Val Glu Ala Leu Glu Leu Val Lys
       945                 950                 955
   aaa aga gaa aag aaa tgg aga cat cag aag gag cag cac tgt tcg caa      3171
   Lys Arg Glu Lys Lys Trp Arg His Gln Lys Glu Gln His Cys Ser Gln
       960                 965                 970
   acg aaa cac aaa tat gat gcg gcc aaa cat gcg gtg atg gcg tta ttt      3219
   Thr Lys His Lys Tyr Asp Ala Ala Lys His Ala Val Met Ala Leu Phe
   975                 980                 985                 990
   aca aac acg cgc tat gaa aaa ttg aag ttc gaa aca acc att tct gac      3267
   Thr Asn Thr Arg Tyr Glu Lys Leu Lys Phe Glu Thr Thr Ile Ser Asp
                   995                 1000                1005
   att ttg tat gct gat cat ctc gtg cag tcg att cct tat gta tat aat      3315
   Ile Leu Tyr Ala Asp His Leu Val Gln Ser Ile Pro Tyr Val Tyr Asn
               1010                1015                1020
   aaa tat gta ccg gaa gtt tca ggt atg aat tac gaa ctc tat aca gag      3363
   Lys Tyr Val Pro Glu Val Ser Gly Met Asn Tyr Glu Leu Tyr Thr Glu
           1025                1030                1035
   cta aac act ctc gtt cag aat gcg ttc tac ctg tat gac cag cgg aat      3411
   Leu Asn Thr Leu Val Gln Asn Ala Phe Tyr Leu Tyr Asp Gln Arg Asn
       1040                1045                1050
   ctg att aaa aat ggg cgc ttt agc aat ggg ctt atg tat tgg caa gct      3459
   Leu Ile Lys Asn Gly Arg Phe Ser Asn Gly Leu Met Tyr Trp Gln Ala
   1055                1060                1065                1070
   acc ccg cat gca cga gtg gag caa gaa tat gat aga tca gtg ctg gtg      3507
   Thr Pro His Ala Arg Val Glu Gln Glu Tyr Asp Arg Ser Val Leu Val
                   1075                1080                1085
   ctg ccg aat tgg gat gcc aat gtg tcg caa cag ctg tgt atc gaa cac      3555
   Leu Pro Asn Trp Asp Ala Asn Val Ser Gln Gln Leu Cys Ile Glu His
               1090                1095                1100
   aat cgc ggt tat gta ttg cgt gtc acg gcg aga aaa gaa gat ccg gga      3603
   Asn Arg Gly Tyr Val Leu Arg Val Thr Ala Arg Lys Glu Asp Pro Gly
           1105                1110                1115
   gcc ggc aat gtt acc ttt agt gac tgt gca aat cat gtc gac aag ctg      3651
   Ala Gly Asn Val Thr Phe Ser Asp Cys Ala Asn His Val Asp Lys Leu
       1120                1125                1130
   agc ttt act tct tgc gat ata gct aca aac gca gtg cca ggt gcc caa      3699
   Ser Phe Thr Ser Cys Asp Ile Ala Thr Asn Ala Val Pro Gly Ala Gln
   1135                1140                1145                1150
   gcg aat gat cca gcc gcc gga gta gcc tat gga caa cag ggt tgt caa      3747
   Ala Asn Asp Pro Ala Ala Gly Val Ala Tyr Gly Gln Gln Gly Cys Gln
                   1155                1160                1165
   ata gat aga gtg ccg tac gga cca tct gga tat cga gca gac gga gta      3795
   Ile Asp Arg Val Pro Tyr Gly Pro Ser Gly Tyr Arg Ala Asp Gly Val
               1170                1175                1180
   gcg tac gaa cag tct ggt cat cga aca gat gga gtg ccg tac aga caa      3843
   Ala Tyr Glu Gln Ser Gly His Arg Thr Asp Gly Val Pro Tyr Arg Gln
           1185                1190                1195
   tct gga tat cga gca gac gga gta gcg cac gac caa cct gga tat cga      3891
   Ser Gly Tyr Arg Ala Asp Gly Val Ala His Asp Gln Pro Gly Tyr Arg
       1200                1205                1210
   gca gac gga gta gcg tac gaa caa tct gga tat cga gca gat gga gta      3939
   Ala Asp Gly Val Ala Tyr Glu Gln Ser Gly Tyr Arg Ala Asp Gly Val
   1215                1220                1225                1230
   gcg tac gaa cag tct ggt cat cga gca gat gga gtg ccg tac gga caa      3987
   Ala Tyr Glu Gln Ser Gly His Arg Ala Asp Gly Val Pro Tyr Gly Gln
                   1235                1240                1245
   tct gga tat gga aca gac gga gta acg tac gac caa tct gcc aaa caa      4035
   Ser Gly Tyr Gly Thr Asp Gly Val Thr Tyr Asp Gln Ser Ala Lys Gln
               1250                1255                1260
   acc cgc aaa tac cat ggt tgc cat aca gac gga ctg cca cat cca gag      4083
   Thr Arg Lys Tyr His Gly Cys His Thr Asp Gly Leu Pro His Pro Glu
           1265                1270                1275
   cat ggt tgt tgt tat cca gac aga gta agc gat ggc caa cag ctt gca      4131
   His Gly Cys Cys Tyr Pro Asp Arg Val Ser Asp Gly Gln Gln Leu Ala
       1280                1285                1290
   tat gta aca aaa tcg att gat ctg ttc ccg gat aca gat aaa gtc cgg      4179
   Tyr Val Thr Lys Ser Ile Asp Leu Phe Pro Asp Thr Asp Lys Val Arg
   1295                1300                1305                1310
   atc gac att gga gaa acc gaa ggg aac ttt aga gtg gaa agt gtg gaa      4227
   Ile Asp Ile Gly Glu Thr Glu Gly Asn Phe Arg Val Glu Ser Val Glu
                   1315                1320                1325
   ttg att tgt atg gaa aag taaatcatca caagtaaaag tatcgtttac             4275
   Leu Ile Cys Met Glu Lys
               1330
   taaaaattta ttttccaagc aacaggggag aggggggctg tccagaaggt cagtgaaaac     4335
   tggacgcccc gttttagtag aata                                           4359
<210> 18
   <211> 1332
   <212> PRT
   <213> Bacillus popilliae
<400> 18
<210> 19
   <211> 4366
   <212> DNA
   <213> Bacillus popilliae
<220>
   <221> CDS
   <222> (224)..(4255)
<400> 19
   gtagatcatg tactcaaatg cagtgtcgga agtttgccag atgttcatat cgattgtcaa 60
   tatgtgacgg tatgtgattt acagatgagg cccgttcatg agggagcttg tcaatttgtg 120
   aagattagcg gagaatttca attttattca ctttaacatt agctacaaat gttgtggcta 180
   agcaccccat tatataaatg gatgacaacc aggaggggta act atg aat cag tat   235
                                                   Met Asn Gln Tyr
                                                    1
   cat aac caa aac gat aac aaa agt tac aac caa agt gga aat gaa atg   283
   His Asn Gln Asn Asp Asn Lys Ser Tyr Asn Gln Ser Gly Asn Glu Met
     5                  10                  15                  20
   caa atc att caa cct tca agt aat tct tta ctt tac agt ccc aat aag    331
   Gln Ile Ile Gln Pro Ser Ser Asn Ser Leu Leu Tyr Ser Pro Asn Lys
                   25                  30                  35
   tat ccg tat gcc acg gat ccc aat gtc ata gca gag ggt aga agt tat    379
   Tyr Pro Tyr Ala Thr Asp Pro Asn Val Ile Ala Glu Gly Arg Ser Tyr
               40                  45                  50
   aaa aat tgg ctt gat atg tgt gta ggt gaa ggc gac ggt aca cga agt    427
   Lys Asn Trp Leu Asp Met Cys Val Gly Glu Gly Asp Gly Thr Arg Ser
           55                  60                  65
   ctc gag gct att gct gtt gct gtc gga gtt cga ata agc cac aca att    475
   Leu Glu Ala Ile Ala Val Ala Val Gly Val Arg Ile Ser His Thr Ile
       70                  75                  80
   ttc cgc ctt tta ggt gtt cca tat tca gct caa ggc gag caa tta ttt    523
   Phe Arg Leu Leu Gly Val Pro Tyr Ser Ala Gln Gly Glu Gln Leu Phe
   85                  90                  95                  100
   agc ttc cta ttg gat acg tta tgg ctt gaa ggc aat act caa tgg gaa    571
   Ser Phe Leu Leu Asp Thr Leu Trp Leu Glu Gly Asn Thr Gln Trp Glu
                   105                 110                 115
   gag ttg atg aga cat gca gaa gaa ctc ata aat gaa cag gta ccg gat    619
   Glu Leu Met Arg His Ala Glu Glu Leu Ile Asn Glu Gln Val Pro Asp
               120                 125                 130
   tat gta aga acc aag gca ctt gca gaa tta acg gat tta ggt aac aac    667
   Tyr Val Arg Thr Lys Ala Leu Ala Glu Leu Thr Asp Leu Gly Asn Asn
           135                 140                 145
   tta aat tta tat ata gca gct ttt gaa gat tgg aaa cga aat ccg agc    715
   Leu Asn Leu Tyr Ile Ala Ala Phe Glu Asp Trp Lys Arg Asn Pro Ser
       150                 155                 160
   agt caa gaa gtt aga acc cgg gta ata gat aga ttc aat ata ctc gac    763
   Ser Gln Glu Val Arg Thr Arg Val Ile Asp Arg Phe Asn Ile Leu Asp
   165                 170                 175                 180
   ggt tta ttt gaa gcc tat ctg cct tca ttt gca gta cct ggt tat gaa    811
   Gly Leu Phe Glu Ala Tyr Leu Pro Ser Phe Ala Val Pro Gly Tyr Glu
                   185                 190                 195
   gta cca cta tta tcc gtg tat gca aat gtt gta aat atc cac tta ttg    859
   Val Pro Leu Leu Ser Val Tyr Ala Asn Val Val Asn Ile His Leu Leu
               200                 205                 210
   gta ctg agg gac agc tcg att tat ggt ctg gat tgg gga tta agt tca    907
   Val Leu Arg Asp Ser Ser Ile Tyr Gly Leu Asp Trp Gly Leu Ser Ser
           215                 220                 225
   act agt gtt gac aat aat tac aat cgc caa caa agg aac tcc gca acg    955
   Thr Ser Val Asp Asn Asn Tyr Asn Arg Gln Gln Arg Asn Ser Ala Thr
       230                 235                 240
   tat gca aat cat tgt aca act tgg tat cag acg ggt tta caa aga ttg    1003
   Tyr Ala Asn His Cys Thr Thr Trp Tyr Gln Thr Gly Leu Gln Arg Leu
   245                 250                 255                 260
   caa ggc agc gat gct agc agt tgg gtc aat tat aat cga ttt aga aga    1051
   Gln Gly Ser Asp Ala Ser Ser Trp Val Asn Tyr Asn Arg Phe Arg Arg
                   265                 270                 275
   gaa ata acg tta ata gta ttg gat att tgt gca ttg ttt tca aat tat    1099
   Glu Ile Thr Leu Ile Val Leu Asp Ile Cys Ala Leu Phe Ser Asn Tyr
               280                 285                 290
   gat gtt cgt agt tat cca ata cag tta cgg gga gag ctt acg aga gga    1147
   Asp Val Arg Ser Tyr Pro Ile Gln Leu Arg Gly Glu Leu Thr Arg Gly
           295                 300                 305
   att tat acg gat cca gca gta tat agc ggt aca ggt tcc tat tcc tgg    1195
   Ile Tyr Thr Asp Pro Ala Val Tyr Ser Gly Thr Gly Ser Tyr Ser Trp
       310                 315                 320
   ttg agt caa gca cca tca ttt gca gaa ata gaa aat atc gca att agg    1243
   Leu Ser Gln Ala Pro Ser Phe Ala Glu Ile Glu Asn Ile Ala Ile Arg
   325                 330                 335                 340
   gaa cca agc aat ttt act tgg gca tct tat gcg aga gta aca aca ggt    1291
   Glu Pro Ser Asn Phe Thr Trp Ala Ser Tyr Ala Arg Val Thr Thr Gly
                   345                 350                 355
   aca ctg gaa tat ctc agc tct aag aat gat ttt tgg aaa tca cac tat    1339
   Thr Leu Glu Tyr Leu Ser Ser Lys Asn Asp Phe Trp Lys Ser His Tyr
               360                 365                 370
   atg aac tat act gaa acc aat tcg ggt ata ttg att caa gga cct acc    1387
   Met Asn Tyr Thr Glu Thr Asn Ser Gly Ile Leu Ile Gln Gly Pro Thr
           375                 380                 385
   tat gga atg acg acg ggt aca aat att cgt ata gag tcc gta tca atg    1435
   Tyr Gly Met Thr Thr Gly Thr Asn Ile Arg Ile Glu Ser Val Ser Met
       390                 395                 400
   caa gaa att tat tcc gtt aga tta gaa gct gtt gct cat gct gga gct    1483
   Gln Glu Ile Tyr Ser Val Arg Leu Glu Ala Val Ala His Ala Gly Ala
   405                 410                 415                 420
   ggg ggt cct ttt ttg gga atc tct acg tct gaa ttt ttc tgg agt ttg    1531
   Gly Gly Pro Phe Leu Gly Ile Ser Thr Ser Glu Phe Phe Trp Ser Leu
                   425                 430                 435
   ggt gtt aga agg tat cag aac tca cgt agt cct caa ttt gcg tct caa    1579
   Gly Val Arg Arg Tyr Gln Asn Ser Arg Ser Pro Gln Phe Ala Ser Gln
               440                 445                 450
   ata ata act agg caa tta cct gga gta aac tca gcg gtt cca tct gcc    1627
   Ile Ile Thr Arg Gln Leu Pro Gly Val Asn Ser Ala Val Pro Ser Ala
           455                 460                 465
   ctc gac cat agt cat gaa cta tcg tat atc aca gcg ttt cca gtt aga    1675
   Leu Asp His Ser His Glu Leu Ser Tyr Ile Thr Ala Phe Pro Val Arg
       470                 475                 480
   tcg gtg gga acg att ctc gtt cat gaa tgg aca tct aca aca gtt agt    1723
   Ser Val Gly Thr Ile Leu Val His Glu Trp Thr Ser Thr Thr Val Ser
   485                 490                 495                 500
   cgt aac aat aga att gag cca gat aaa ata aca caa atc ccg gct gtt    1771
   Arg Asn Asn Arg Ile Glu Pro Asp Lys Ile Thr Gln Ile Pro Ala Val
                   505                 510                 515
   aag tca cac aca ctc tcc aat tgt caa gta gtt agt ggg act ggg ttt    1819
   Lys Ser His Thr Leu Ser Asn Cys Gln Val Val Ser Gly Thr Gly Phe
               520                 525                 530
   acg gga gga aac tgg ttg aga cct tct gat aat ggt tca ttt aga cta    1867
   Thr Gly Gly Asn Trp Leu Arg Pro Ser Asp Asn Gly Ser Phe Arg Leu
           535                 540                 545
   acg att act tca ttc tca agc caa tct tac cgc att cgc att cat tat    1915
   Thr Ile Thr Ser Phe Ser Ser Gln Ser Tyr Arg Ile Arg Ile His Tyr
       550                 555                 560
   gct tcc gca aca ttt ttt tat ttg gat att cgt acg ggt gat act tct    1963
   Ala Ser Ala Thr Phe Phe Tyr Leu Asp Ile Arg Thr Gly Asp Thr Ser
   565                 570                 575                 580
   aac aca ttt gcg gtt acc cca aca aca tta tca tca gga tcc caa act    2011
   Asn Thr Phe Ala Val Thr Pro Thr Thr Leu Ser Ser Gly Ser Gln Thr
                   585                 590                 595
   gta ccc tac gaa tct ttt ggg ttt ata aat ata cct tat act ttt aca    2059
   Val Pro Tyr Glu Ser Phe Gly Phe Ile Asn Ile Pro Tyr Thr Phe Thr
               600                 605                 610
   aca gca cct act gaa agt aga tat act ttt gat ttc atg ttc tac tca    2107
   Thr Ala Pro Thr Glu Ser Arg Tyr Thr Phe Asp Phe Met Phe Tyr Ser
           615                 620                 625
   ata gga agc gca aat gta tta att gac cga att gaa att gtt cca atc    2155
   Ile Gly Ser Ala Asn Val Leu Ile Asp Arg Ile Glu Ile Val Pro Ile
       630                 635                 640
   gga gtt cct ttg ttc gag tac gaa acc aaa cag cag cta gaa aaa gca    2203
   Gly Val Pro Leu Phe Glu Tyr Glu Thr Lys Gln Gln Leu Glu Lys Ala
   645                 650                 655                 660
   agg aaa gcg gtg aac cat ttg ttt aca gat gga tcg aaa aag gcg cta    2251
   Arg Lys Ala Val Asn His Leu Phe Thr Asp Gly Ser Lys Lys Ala Leu
                   665                 670                 675
   aaa gaa gac acg acc gat tat gag att gat caa gcc gcc aac gtg gta    2299
   Lys Glu Asp Thr Thr Asp Tyr Glu Ile Asp Gln Ala Ala Asn Val Val
               680                 685                 690
   gat tgt ata tcg gat gag tgt gga cat gat aaa atg atc ctg tta gat    2347
   Asp Cys Ile Ser Asp Glu Cys Gly His Asp Lys Met Ile Leu Leu Asp
           695                 700                 705
   gaa gta aaa tat gca aaa caa ctc agc caa gcc cgc aat tta ctg ctc    2395
   Glu Val Lys Tyr Ala Lys Gln Leu Ser Gln Ala Arg Asn Leu Leu Leu
       710                 715                 720
   aat ggg aat ttc gat gat cta tat tca gct ctg gag aag gag aat cca    2443
   Asn Gly Asn Phe Asp Asp Leu Tyr Ser Ala Leu Glu Lys Glu Asn Pro
   725                 730                 735                 740
   tgg aaa aca agt ccg aat gtt acg atc cga caa gat aac ccg att ttt    2491
   Trp Lys Thr Ser Pro Asn Val Thr Ile Arg Gln Asp Asn Pro Ile Phe
                   745                 750                 755
   aaa ggc cat tat ctc agt atg gcg ggt gcg aac gat atc gag gcc acc    2539
   Lys Gly His Tyr Leu Ser Met Ala Gly Ala Asn Asp Ile Glu Ala Thr
               760                 765                 770
   aat gat acc ttc ccc acg tat gtc tat caa aaa ata gac gaa gcc aaa    2587
   Asn Asp Thr Phe Pro Thr Tyr Val Tyr Gln Lys Ile Asp Glu Ala Lys
           775                 780                 785
   tta aag ccg tat aca cgt tat aaa gtg cgc ggg ttt gtt ggc agc agc    2635
   Leu Lys Pro Tyr Thr Arg Tyr Lys Val Arg Gly Phe Val Gly Ser Ser
       790                 795                 800
   aaa gct cta gag ctg ttg gtt aca cgc tat aat gaa gaa gtt gat gcg    2683
   Lys Ala Leu Glu Leu Leu Val Thr Arg Tyr Asn Glu Glu Val Asp Ala
   805                 810                 815                 820
   att tta gat gta ccg gat aat atc ccg cat gcg ccg act cct gtc tgc    2731
   Ile Leu Asp Val Pro Asp Asn Ile Pro His Ala Pro Thr Pro Val Cys
                   825                 830                 835
   ggt gaa ttt gat cga tgc aag ccc tat tcg tat cca cct tta ctt cca    2779
   Gly Glu Phe Asp Arg Cys Lys Pro Tyr Ser Tyr Pro Pro Leu Leu Pro
               840                 845                 850
   gaa tgt aac cct gag ttt ata aat cag atg caa cca tcc tct tgc cac    2827
   Glu Cys Asn Pro Glu Phe Ile Asn Gln Met Gln Pro Ser Ser Cys His
           855                 860                 865
   cac aat cag atg gtc gat tac aat aac atg aac acg agc acg agt act    2875
   His Asn Gln Met Val Asp Tyr Asn Asn Met Asn Thr Ser Thr Ser Thr
       870                 875                 880
   acc atg aat cct agc atg aat cct ccc ctt acg cct gaa ata gca tcc    2923
   Thr Met Asn Pro Ser Met Asn Pro Pro Leu Thr Pro Glu Ile Ala Ser
   885                 890                 895                 900
   agc caa agt gga ttc ggc aga aaa cat cgc aaa tgt cat caa gcg cat    2971
   Ser Gln Ser Gly Phe Gly Arg Lys His Arg Lys Cys His Gln Ala His
                   905                 910                 915
   caa ttt gag ttc cac att gat acc ggg aca atc gat ttg gtc gaa gat    3019
   Gln Phe Glu Phe His Ile Asp Thr Gly Thr Ile Asp Leu Val Glu Asp
               920                 925                 930
   ttg ggc att tgg gtg atc ttc aaa atc tgt gcc aca gat gga tac gca    3067
   Leu Gly Ile Trp Val Ile Phe Lys Ile Cys Ala Thr Asp Gly Tyr Ala
           935                 940                 945
   agc tta gat gat ctg gaa gtg att gaa gaa gga gcg ctg ggt gtc gaa    3115
   Ser Leu Asp Asp Leu Glu Val Ile Glu Glu Gly Ala Leu Gly Val Glu
       950                 955                 960
   gca tta gaa ctt gtc aaa aaa aga gaa aag aaa tgg aga cat cag aag    3163
   Ala Leu Glu Leu Val Lys Lys Arg Glu Lys Lys Trp Arg His Gln Lys
   965                 970                 975                 980
   gag cag cac tgt tcg caa acg aaa cac aaa tat gat gcg gcc aaa cat    3211
   Glu Gln His Cys Ser Gln Thr Lys His Lys Tyr Asp Ala Ala Lys His
                   985                 990                 995
   gcg gtg atg gcg tta ttt aca aac aag cgc tat gaa aaa ttg aag ttc    3259
   Ala Val Met Ala Leu Phe Thr Asn Lys Arg Tyr Glu Lys Leu Lys Phe
               1000                1005                1010
   gaa aca acc att tct gac att ttg tat gct gat cat ctc gtg cag tcg    3307
   Glu Thr Thr Ile Ser Asp Ile Leu Tyr Ala Asp His Leu Val Gln Ser
           1015                1020                1025
   att cct tat gta tat aat aaa tat gta ccg gaa gtt cca ggt atg aat  3355
   Ile Pro Tyr Val Tyr Asn Lys Tyr Val Pro Glu Val Pro Gly Met Asn
      1030                1035                1040
   tac gaa ctc tat tca gag cta aac aca ctg gtt cag aat gcg ttc tac   3403
   Tyr Glu Leu Tyr Ser Glu Leu Asn Thr Leu Val Gln Asn Ala Phe Tyr
   1045                1050                1055                1060
   ctg tat gac cag cgg aat ctg att aaa aat ggg cgc ttt agc aat ggg   3451
   Leu Tyr Asp Gln Arg Asn Leu Ile Lys Asn Gly Arg Phe Ser Asn Gly
                   1065                1070                1075
   ctt atg cat tgg caa gct act cct cat gca aga gta gag caa gaa tat   3499
   Leu Met His Trp Gln Ala Thr Pro His Ala Arg Val Glu Gln Glu Tyr
               1080                1085                1090
   gag aaa tcg gtg ctc gtg ctg cca aat tgg gat gcc aat gtg tcg caa   3547
   Glu Lys Ser Val Leu Val Leu Pro Asn Trp Asp Ala Asn Val Ser Gln
           1095                1100                1105
   gat ctt tgt atc gaa cac aat cgc ggt tat gta ttg cgt gtc acg gcg   3595
   Asp Leu Cys Ile Glu His Asn Arg Gly Tyr Val Leu Arg Val Thr Ala
       1110                1115                1120
   aga aaa gaa gat ccg gga gct ggc aat gtt acc ttt agt gac tgt gaa   3643
   Arg Lys Glu Asp Pro Gly Ala Gly Asn Val Thr Phe Ser Asp Cys Glu
   1125                1130                1135                1140
   aat cat gtc gac aag ctg agc ttt act tct tgc gat ata gct aca aac   3691
   Asn His Val Asp Lys Leu Ser Phe Thr Ser Cys Asp Ile Ala Thr Asn
                   1145                1150                1155
   gca gtg cca ggt gcc caa gcg aat gat cca gcc gcc gga gta gcc tat   3739
   Ala Val Pro Gly Ala Gln Ala Asn Asp Pro Ala Ala Gly Val Ala Tyr
               1160                1165                1170
   gga caa cag ggt tgt caa ata gat aga gtg ccg tac ggg caa tct gga   3787
   Gly Gln Gln Gly Cys Gln Ile Asp Arg Val Pro Tyr Gly Gln Ser Gly
           1175                1180                1185
   tat cga gca gac gga gta gcg tac gaa cag tct ggt cat cga aca gat  3835
   Tyr Arg Ala Asp Gly Val Ala Tyr Glu Gln Ser Gly His Arg Thr Asp
       1190                1195                1200
   gga gtg ccg tac aga caa tct gga tat gga aca gac gga gta acg tac   3883
   Gly Val Pro Tyr Arg Gln Ser Gly Tyr Gly Thr Asp Gly Val Thr Tyr
   1205                1210                1215                1220
   gaa caa tct ggt cat cga gca gat gga gtg ccg tac gga caa tct gga   3931
   Glu Gln Ser Gly His Arg Ala Asp Gly Val Pro Tyr Gly Gln Ser Gly
                   1225                1230                1235
   tat cga gca gat gga gta gcg tac gaa cag tct ggt cat cga gca gat   3979
   Tyr Arg Ala Asp Gly Val Ala Tyr Glu Gln Ser Gly His Arg Ala Asp
               1240                1245                1250
   gga gtg ccg tac gga caa tct gga tat gga aca gac gga gta acg tac   4027
   Gly Val Pro Tyr Gly Gln Ser Gly Tyr Gly Thr Asp Gly Val Thr Tyr
           1255                1260                1265
   gac caa tct gcc aat caa acc cgc aaa tat cat ggt tgc cat aca gac   4075
   Asp Gln Ser Ala Asn Gln Thr Arg Lys Tyr His Gly Cys His Thr Asp
       1270                1275                1280
   gga ctg cca cat cca gag cat ggt tgt tgt tat cca gac aga gta agc   4123
   Gly Leu Pro His Pro Glu His Gly Cys Cys Tyr Pro Asp Arg Val Ser
   1285                1290                1295                1300
   gat ggc caa cag ctt gca tat gta aca aaa tcg att gat ctg ttc ccg   4171
   Asp Gly Gln Gln Leu Ala Tyr Val Thr Lys Ser Ile Asp Leu Phe Pro
                   1305                1310                1315
   gat aca gat aaa gtc cgg atc gac att gga gaa acc gaa ggg aac ttt   4219
   Asp Thr Asp Lys Val Arg Ile Asp Ile Gly Glu Thr Glu Gly Asn Phe
               1320                1325                1330
   aga gtg gaa agt gtg gaa ttg att tgt atg gaa aag taaatcatca        4265
   Arg Val Glu Ser Val Glu Leu Ile Cys Met Glu Lys
           1335                1340
   caagtaaaag tatcgtttac taaaaattta ttttccaagc aacaggggag aagatgattt 4325
   gggtgtaata ctcaaatcat cttttcttat aagccacttt a                     4366
<210> 20
   <211> 1344
   <212> PRT
   <213> Bacillus popilliae
<400> 20
   Met Asn Gln Tyr His Asn Gln Asn Asp Asn Lys Ser Tyr Asn Gln Ser
   1               5                   10                  15
   Gly Asn Glu Met Gln Ile Ile Gln Pro Ser Ser Asn Ser Leu Leu Tyr
               20                  25                  30
   Ser Pro Asn Lys Tyr Pro Tyr Ala Thr Asp Pro Asn Val Ile Ala Glu
           35                  40                  45
   Gly Arg Ser Tyr Lys Asn Trp Leu Asp Met Cys Val Gly Glu Gly Asp
       50                  55                  60
   Gly Thr Arg Ser Leu Glu Ala Ile Ala Val Ala Val Gly Val Arg Ile
   65                  70                  75                  80
   Ser His Thr Ile Phe Arg Leu Leu Gly Val Pro Tyr Ser Ala Gln Gly
                   85                  90                  95
   Glu Gln Leu Phe Ser Phe Leu Leu Asp Thr Leu Trp Leu Glu Gly Asn
               100                 105                 110
   Thr Gln Trp Glu Glu Leu Met Arg His Ala Glu Glu Leu Ile Asn Glu
           115                 120                 125
   Gln Val Pro Asp Tyr Val Arg Thr Lys Ala Leu Ala Glu Leu Thr Asp
       130                 135                 140
   Leu Gly Asn Asn Leu Asn Leu Tyr Ile Ala Ala Phe Glu Asp Trp Lys
   145                 150                 155                 160
   Arg Asn Pro Ser Ser Gln Glu Val Arg Thr Arg Val Ile Asp Arg Phe
                   165                 170                 175
   Asn Ile Leu Asp Gly Leu Phe Glu Ala Tyr Leu Pro Ser Phe Ala Val
               180                 185                 190
   Pro Gly Tyr Glu Val Pro Leu Leu Ser Val Tyr Ala Asn Val Val Asn
           195                 200                 205
   Ile His Leu Leu Val Leu Arg Asp Ser Ser Ile Tyr Gly Leu Asp Trp
       210                 215                 220
   Gly Leu Ser Ser Thr Ser Val Asp Asn Asn Tyr Asn Arg Gln Gln Arg
   225                 230                 235                 240
   Asn Ser Ala Thr Tyr Ala Asn His Cys Thr Thr Trp Tyr Gln Thr Gly
                   245                 250                 255
   Leu Gln Arg Leu Gln Gly Ser Asp Ala Ser Ser Trp Val Asn Tyr Asn
               260                 265                 270
   Arg Phe Arg Arg Glu Ile Thr Leu Ile Val Leu Asp Ile Cys Ala Leu
           275                 280                 285
   Phe Ser Asn Tyr Asp Val Arg Ser Tyr Pro Ile Gln Leu Arg Gly Glu
       290                 295                 300
   Leu Thr Arg Gly Ile Tyr Thr Asp Pro Ala Val Tyr Ser Gly Thr Gly
   305                 310                 315                 320
   Ser Tyr Ser Trp Leu Ser Gln Ala Pro Ser Phe Ala Glu Ile Glu Asn
                   325                 330                 335
   Ile Ala Ile Arg Glu Pro Ser Asn Phe Thr Trp Ala Ser Tyr Ala Arg
               340                 345                 350
   Val Thr Thr Gly Thr Leu Glu Tyr Leu Ser Ser Lys Asn Asp Phe Trp
           355                 360                 365
   Lys Ser His Tyr Met Asn Tyr Thr Glu Thr Asn Ser Gly Ile Leu Ile
       370                 375                 380
   Gln Gly Pro Thr Tyr Gly Met Thr Thr Gly Thr Asn Ile Arg Ile Glu
   385                 390                 395                 400
   Ser Val Ser Met Gln Glu Ile Tyr Ser Val Arg Leu Glu Ala Val Ala
                   405                 410                 415
   His Ala Gly Ala Gly Gly Pro Phe Leu Gly Ile Ser Thr Ser Glu Phe
               420                 425                 430
   Phe Trp Ser Leu Gly Val Arg Arg Tyr Gln Asn Ser Arg Ser Pro Gln
           435                 440                 445
   Phe Ala Ser Gln Ile Ile Thr Arg Gln Leu Pro Gly Val Asn Ser Ala
       450                 455                 460
   Val Pro Ser Ala Leu Asp His Ser His Glu Leu Ser Tyr Ile Thr Ala
   465                 470                 475                 480
   Phe Pro Val Arg Ser Val Gly Thr Ile Leu Val His Glu Trp Thr Ser
                   485                 490                 495
   Thr Thr Val Ser Arg Asn Asn Arg Ile Glu Pro Asp Lys Ile Thr Gln
               500                 505                 510
   Ile Pro Ala Val Lys Ser His Thr Leu Ser Asn Cys Gln Val Val Ser
           515                 520                 525
   Gly Thr Gly Phe Thr Gly Gly Asn Trp Leu Arg Pro Ser Asp Asn Gly
       530                 535                 540
   Ser Phe Arg Leu Thr Ile Thr Ser Phe Ser Ser Gln Ser Tyr Arg Ile
   545                 550                 555                 560
   Arg Ile His Tyr Ala Ser Ala Thr Phe Phe Tyr Leu Asp Ile Arg Thr
                   565                 570                 575
   Gly Asp Thr Ser Asn Thr Phe Ala Val Thr Pro Thr Thr Leu Ser Ser
               580                 585                 590
   Gly Ser Gln Thr Val Pro Tyr Glu Ser Phe Gly Phe Ile Asn Ile Pro
           595                 600                 605
   Tyr Thr Phe Thr Thr Ala Pro Thr Glu Ser Arg Tyr Thr Phe Asp Phe
       610                 615                 620
   Met Phe Tyr Ser Ile Gly Ser Ala Asn Val Leu Ile Asp Arg Ile Glu
   625                 630                 635                 640
   Ile Val Pro Ile Gly Val Pro Leu Phe Glu Tyr Glu Thr Lys Gln Gln
                   645                 650                 655
   Leu Glu Lys Ala Arg Lys Ala Val Asn His Leu Phe Thr Asp Gly Ser
               660                 665                 670
   Lys Lys Ala Leu Lys Glu Asp Thr Thr Asp Tyr Glu Ile Asp Gln Ala
           675                 680                 685
   Ala Asn Val Val Asp Cys Ile Ser Asp Glu Cys Gly His Asp Lys Met
       690                 695                 700
   Ile Leu Leu Asp Glu Val Lys Tyr Ala Lys Gln Leu Ser Gln Ala Arg
   705                 710                 715                 720
   Asn Leu Leu Leu Asn Gly Asn Phe Asp Asp Leu Tyr Ser Ala Leu Glu
                   725                 730                 735
   Lys Glu Asn Pro Trp Lys Thr Ser Pro Asn Val Thr Ile Arg Gln Asp
               740                 745                 750
   Asn Pro Ile Phe Lys Gly His Tyr Leu Ser Met Ala Gly Ala Asn Asp
           755                 760                 765
   Ile Glu Ala Thr Asn Asp Thr Phe Pro Thr Tyr Val Tyr Gln Lys Ile
       770                 775                 780
   Asp Glu Ala Lys Leu Lys Pro Tyr Thr Arg Tyr Lys Val Arg Gly Phe
   785                 790                 795                 800
   Val Gly Ser Ser Lys Ala Leu Glu Leu Leu Val Thr Arg Tyr Asn Glu
                   805                 810                 815
   Glu Val Asp Ala Ile Leu Asp Val Pro Asp Asn Ile Pro His Ala Pro
               820                 825                 830
   Thr Pro Val Cys Gly Glu Phe Asp Arg Cys Lys Pro Tyr Ser Tyr Pro
           835                 840                 845
   Pro Leu Leu Pro Glu Cys Asn Pro Glu Phe Ile Asn Gln Met Gln Pro
       850                 855                 860
   Ser Ser Cys His His Asn Gln Met Val Asp Tyr Asn Asn Met Asn Thr
   865                 870                 875                 880
   Ser Thr Ser Thr Thr Met Asn Pro Ser Met Asn Pro Pro Leu Thr Pro
                   885                 890                 895
   Glu Ile Ala Ser Ser Gln Ser Gly Phe Gly Arg Lys His Arg Lys Cys
               900                 905                 910
   His Gln Ala His Gln Phe Glu Phe His Ile Asp Thr Gly Thr Ile Asp
           915                 920                 925
   Leu Val Glu Asp Leu Gly Ile Trp Val Ile Phe Lys Ile Cys Ala Thr
       930                 935                 940
   Asp Gly Tyr Ala Ser Leu Asp Asp Leu Glu Val Ile Glu Glu Gly Ala
   945                 950                 955                 960
   Leu Gly Val Glu Ala Leu Glu Leu Val Lys Lys Arg Glu Lys Lys Trp
                   965                 970                 975
   Arg His Gln Lys Glu Gln His Cys Ser Gln Thr Lys His Lys Tyr Asp
               980                 985                 990
   Ala Ala Lys His Ala Val Met Ala Leu Phe Thr Asn Lys Arg Tyr Glu
           995                 1000                1005
   Lys Leu Lys Phe Glu Thr Thr Ile Ser Asp Ile Leu Tyr Ala Asp His
      1010                1015                1020
   Leu Val Gln Ser Ile Pro Tyr Val Tyr Asn Lys Tyr Val Pro Glu Val
   1025                1030                1035                1040
   Pro Gly Met Asn Tyr Glu Leu Tyr Ser Glu Leu Asn Thr Leu Val Gln
                   1045                1050                1055
   Asn Ala Phe Tyr Leu Tyr Asp Gln Arg Asn Leu Ile Lys Asn Gly Arg
               1060                1065                1070
   Phe Ser Asn Gly Leu Met His Trp Gln Ala Thr Pro His Ala Arg Val
           1075                1080                1085
   Glu Gln Glu Tyr Glu Lys Ser Val Leu Val Leu Pro Asn Trp Asp Ala
      1090                1095                1100
   Asn Val Ser Gln Asp Leu Cys Ile Glu His Asn Arg Gly Tyr Val Leu
   1105                1110                1115                1120
   Arg Val Thr Ala Arg Lys Glu Asp Pro Gly Ala Gly Asn Val Thr Phe
                   1125                1130                1135
   Ser Asp Cys Glu Asn His Val Asp Lys Leu Ser Phe Thr Ser Cys Asp
               1140                1145                1150
   Ile Ala Thr Asn Ala Val Pro Gly Ala Gln Ala Asn Asp Pro Ala Ala
           1155                1160                1165
   Gly Val Ala Tyr Gly Gln Gln Gly Cys Gln Ile Asp Arg Val Pro Tyr
      1170                1175                1180
   Gly Gln Ser Gly Tyr Arg Ala Asp Gly Val Ala Tyr Glu Gln Ser Gly
   1185                1190                1195                1200
   His Arg Thr Asp Gly Val Pro Tyr Arg Gln Ser Gly Tyr Gly Thr Asp
                   1205                1210                1215
   Gly Val Thr Tyr Glu Gln Ser Gly His Arg Ala Asp Gly Val Pro Tyr
               1220                1225                1230
   Gly Gln Ser Gly Tyr Arg Ala Asp Gly Val Ala Tyr Glu Gln Ser Gly
           1235                1240                1245
   His Arg Ala Asp Gly Val Pro Tyr Gly Gln Ser Gly Tyr Gly Thr Asp
      1250                1255                1260
   Gly Val Thr Tyr Asp Gln Ser Ala Asn Gln Thr Arg Lys Tyr His Gly
   1265                1270                1275                1280
   Cys His Thr Asp Gly Leu Pro His Pro Glu His Gly Cys Cys Tyr Pro
                   1285                1290                1295
   Asp Arg Val Ser Asp Gly Gln Gln Leu Ala Tyr Val Thr Lys Ser Ile
               1300                1305                1310
   Asp Leu Phe Pro Asp Thr Asp Lys Val Arg Ile Asp Ile Gly Glu Thr
           1315                1320                1325
   Glu Gly Asn Phe Arg Val Glu Ser Val Glu Leu Ile Cys Met Glu Lys
      1330                1335                1340
<210> 21
   <211> 34
   <212> DNA
   <213> Artificial Sequence
<220>
   <223> Description of Artificial Sequence: synthetic oligonucleotide
   <400> 21
   ctggaattca tgaatcagta tcataaccaa aacg 34
<210> 22
   <211> 34
   <212> DNA
   <213> Artificial Sequence
<220>
   <223> Description of Artificial Sequence: synthetic oligonucleotide
   <400> 22
   gagctgcagt tacttttcca tacaaatcaa ttcc 34

### SEQUENCE LISTING

<110> Dainippon Ink and Chemicals, Inc.
<120> Polypeptide having larvae growth inhibiting or insecticidal effect on scarabaeidae insects and polynucleotide encoding the same
<130> 12290EP
<140>
   <141>
<150> JP 2001-115754
   <151> 2001-04-13
   <150> JP 2001-203463
   <151> 2001-07-04
<160> 22
<170> PatentIn Ver. 2.0
<210> 1
   <211> 402
   <212> DNA
   <213> Bacillus popilliae
<220>
   <221> CDS
   <222> (1) .. (402)
<400> 1
<210> 2
   <211> 134
   <212> PRT
   <213> Bacillus popilliae
<210> 3
   <211> 4359
   <212> DNA
   <213> Bacillus popilliae
<220>
   <221> CDS
   <222> (282)..(4229)
<400> 3
<210> 4
   <211> 1316
   <212> PRT
   <213> Bacillus popilliae
<400> 4
   Met Leu Trp Leu Asn Thr His Cys Ser Leu Ile Gly Ile Thr Gly Arg
    1               5                  10                  15
   Gln Thr Met Asn Gln Tyr His Asn Gln Asn Asp Asn Lys Ser Tyr Asn
                   20                  25                  30
   Gln Ser Gly Asn Glu Met Gln Ile Ile Gln Pro Ser Ser Asn Ala Leu
               35                  40                  45
   Leu Tyr Ser Pro Asn Lys Tyr Pro Tyr Ala Thr Asp Pro Asn Val Ile
           50                  55                  60
   Ala Glu Gly Gly Ser Tyr Lys Asn Trp Leu Asp Met Cys Thr Gly Thr
       65                  70                  75                  80
   Gly Asp Thr Arg Ser Pro Glu Thr Ala Ala Ile Ser Lys Gly Ala Val
                   85                  90                  95
   Ser Ala Ala Ile Thr Ile Ser Thr Gly Leu Leu Gly Leu Leu Gly Val
               100                 105                 110
   Pro Phe Ala Ser Gln Ile Gly Ala Phe Tyr Thr Phe Leu Leu Asn Thr
           115                 120                 125
   Leu Trp Pro Ala Ser Asn Thr Gln Trp Glu Gln Phe Ile Ala His Val
       130                 135                 140
   Glu Glu Leu Ile Asn Ala Lys Leu Thr Asp His Val Arg Asn Ser Ala
   145                 150                 155                 160
   Leu Thr Lys Leu Asn Gly Leu Arg Asn Asn Ile Glu Ile Tyr Asn Glu
                   165                 170                 175
   Ala Leu Ile Val Trp Lys Gln Asp Pro Asn Asn Ser Lys Leu Lys Asp
               180                 185                 190
   Asp Val Arg Ser Lys Phe Val Gly Leu Asn Ser Gln Phe Glu Glu Tyr
           195                 200                 205
   Ile Pro Gln Phe Lys Glu Glu Gly Phe Glu Val Gln Leu Leu Thr Ile
       210                 215                 220
   Tyr Ala Gln Ser Ala Asn Leu His Leu Leu Leu Leu Arg Asp Ser Ser
   225                 230                 235                 240
   Leu Tyr Gly Ala Ser Trp Gly Phe Ala Gln Ala Thr Ile Asp Asn Asn
                   245                 250                 255
   Tyr Asn Arg Gln Ile Arg Lys Thr Ala Glu Tyr Ala Asn His Cys Thr
               260                 265                 270
   Thr Trp Tyr Gln Thr Gly Leu Gln Arg Leu Gln Gly Thr Thr Ala Ser
           275                 280                 285
   Ser Trp Leu Ser Tyr His Arg Phe Arg Arg Glu Met Thr Leu Thr Val
       290                 295                 300
   Leu Asp Ile Cys Ala Leu Phe Ser Asn Tyr Asp Ala Arg Ser Tyr Pro
   305                 310                 315                 320
   Leu Glu Val Arg Gly Glu Leu Thr Arg Glu Ile Tyr Thr Asp Pro Val
                   325                 330                 335
   Ala Pro Gly Thr Asn Trp Ile Asp Arg Ala Pro Ser Phe Ala Glu Ile
               340                 345                 350
   Glu Asn Leu Val Ile Arg Ala Pro Arg Thr Val Thr Trp Ile Ser Gly
           355                 360                 365
   Asp Leu Ile Val Tyr Thr Gly Arg Leu Tyr Gly Tyr Thr Gly Asn Asn
       370                 375                 380
   Asp Tyr Trp Ala Ala His Arg Leu Asp Phe Leu Glu Thr Asn Gly Tyr
   385                 390                 395                 400
   Arg Phe Glu Gly Pro Thr Tyr Gly Ser Thr Ile Asn Ile Ser Arg Thr
                   405                 410                 415
   Asp Ser Ile Pro Met Asn Ser Ile Asp Val Tyr Ser Thr Thr Val Val
               420                 425                 430
   Thr Val Gly Ser Ala Trp Pro Thr Gly Gly Phe Val Leu Gly Val Ala
           435                 440                 445
   Ser Ala Arg Phe Phe Ser Lys Ser Pro Ser Thr Gly Leu Leu Gly Glu
       450                 455                 460
   Arg Val Tyr Gln Asn Pro Val Tyr Phe Ser Ser Ser Thr Leu Thr Phe
   465                 470                 475                 480
   Asn Leu Pro Gly Val Asp Gln Asp Thr Pro Thr Ala Ala Asp Tyr Ser
                   485                 490                 495
   His Lys Leu Ser Cys Ile Thr Ala Phe Arg Thr Gly Leu Asn Gly Thr
               500                 505                 510
   Val Pro Val Phe Gly Arg Tyr Ser Ala Thr Val Ser Arg Asp Asn Arg
           515                 520                 525
   Ile Glu Pro Asp Lys Ile Thr Gln Ile Pro Ala Val Lys Ser Asn Ser
       530                 535                 540
   Leu Asp Asn Cys Pro Val Val Arg Gly Thr Gly Phe Thr Gly Gly Asp
   545                 550                 555                 560
   Trp Leu Lys Thr Ser Tyr Leu Ser Val Phe Val Leu Thr Ile Thr Ser
                   565                 570                 575
   Ser Arg Ala Gly Gln Ser Tyr Arg Ile Arg Val Arg Tyr Ala Ala Ala
               580                 585                 590
   Val Asp Leu Ile Met Ser Ile Tyr Ser Asn Asp Pro Phe Ile Ser Lys
           595                 600                 605
   Gly Ile Ser Leu Thr Lys Ser Met Pro Pro Leu Thr Glu Thr Val Pro
       610                 615                 620
   Tyr Glu Ala Phe Lys Phe Ala Asp Phe Gly Val Thr Phe Thr Thr Ala
   625                 630                 635                 640
   Thr Ala Asn Lys Arg Tyr Thr Phe Gln Phe His Thr Gly Gly Ala Ala
                   645                 650                 655
   Ile Ile Asp Arg Ile Glu Phe Val Pro Ile Glu Gly Ser Leu Phe Glu
               660                 665                 670
   Tyr Glu Thr Lys Gln Gln Leu Glu Lys Ala Arg Lys Ala Val Asn His
           675                 680                 685
   Leu Phe Thr Asp Gly Ser Lys Lys Ala Leu Lys Glu Gly Thr Thr Asp
       690                 695                 700
   Tyr Glu Ile Asp Gln Ala Ala Asn Val Val Asp Cys Ile Ser Asp Glu
   705                 710                 715                 720
   Cys Gly His Glu Lys Met Ile Leu Leu Asp Glu Val Lys Tyr Ala Lys
                   725                 730                 735
   Gln Leu Ser Gln Ala Arg Asn Leu Leu Leu Asn Gly Asn Phe Asp Asp
               740                 745                 750
   Leu Tyr Pro Ala Leu Glu Arg Glu Asn Pro Trp Lys Thr Ser Pro His
           755                 760                 765
   Val Thr Ile Arg Gln Asp Asn Pro Ile Phe Lys Gly His Tyr Leu Ser
       770                 775                 780
   Met Ala Gly Ala Asn Asp Ile Glu Ala Thr Asn Asp Thr Phe Pro Thr
   785                 790                 795                 800
   Tyr Val Tyr Gln Lys Ile Asp Glu Ala Lys Leu Lys Pro Tyr Thr Arg
                   805                 810                 815
   Tyr Lys Val Arg Gly Phe Val Gly Ser Ser Lys Ala Leu Glu Leu Leu
               820                 825                 830
   Val Thr Arg Tyr Asn Glu Glu Val Asp Ala Ile Leu Asp Val Pro Asp
           835                 840                 845
   Asn Ile Pro His Ala Pro Thr Pro Val Cys Gly Glu Phe Asp Arg Cys
       850                 855                 860
   Lys Pro Tyr Ser Tyr Pro Pro Leu Leu Pro Glu Cys Asn Pro Glu Phe
   865                 870                 875                 880
   Ile Asn Gln Met Gln Pro Ser Ser Cys His His Asn Gln Met Val Asp
                   885                 890                 895
   Tyr Asn Asn Arg Lys His Arg Lys Cys His Gln Ala His Gln Phe Glu
               900                 905                 910
   Phe His Ile Asp Thr Gly Thr Ile Asp Leu Val Glu Asp Leu Gly Ile
           915                 920                 925
   Trp Val Ile Phe Lys Ile Cys Ala Thr Asp Gly Tyr Ala Ser Leu Asp
       930                 935                 940
   Asp Leu Glu Val Ile Glu Glu Gly Ala Leu Gly Val Glu Ala Leu Glu
   945                 950                 955                 960
   Leu Val Lys Lys Arg Glu Lys Lys Trp Arg His Gln Lys Glu Gln His
                   965                 970                 975
   Cys Ser Gln Thr Lys His Lys Tyr Asp Ala Ala Lys His Ala Val Met
               980                 985                 990
   Ala Leu Phe Thr Asn Thr Arg Tyr Glu Lys Leu Lys Phe Glu Thr Thr
           995                 1000                1005
   Ile Ser Asn Ile Leu Tyr Ala Asp His Leu Val Gln Ser Ile Pro Tyr
       1010                1015                1020
   Val Tyr Asn Lys Tyr Val Pro Glu Val Pro Gly Met Asn Tyr Glu Leu
   1025                1030                1035                1040
   Tyr Thr Glu Leu Asn Thr Leu Val Gln Asn Ala Phe Tyr Leu Tyr Asp
                   1045                1050                1055
   Gln Arg Asn Leu Ile Lys Asn Gly Arg Phe Ser Asn Gly Leu Met Tyr
               1060                1065                1070
   Trp Gln Ala Thr Pro His Ala Arg Val Glu Gln Glu Tyr Glu Lys Ser
           1075                1080                1085
   Val Leu Val Leu Pro Asn Trp Asp Ala Asn Val Ser Gln Asp Leu Cys
       1090                1095                1100
   Ile Glu His Asn Arg Gly Tyr Val Leu Arg Val Thr Ala Arg Lys Glu
   1105                1110                1115                1120
   Asp Pro Gly Ala Gly Asn Val Thr Phe Ser Asp Cys Ala Asn His Val
                   1125                1130                1135
   Asp Lys Leu Ser Phe Thr Ser Cys Asp Ile Ala Thr Asn Ala Val Pro
               1140                1145                1150
   Gly Ala Gln Ala Asn Asp Pro Ala Ala Gly Val Ala Tyr Gly Gln Gln
        1155                1160                1165
   Gly Cys Gln Ile Asp Arg Val Pro Tyr Gly Gln Ser Gly Tyr Arg Thr
      1170                1175                1180
   Asp Gly Thr Asn Gly Met Pro Tyr Gly Gln Ser Gly Asn Arg Ala Asp
   1185                1190                1195                1200
   Gly Val Pro Tyr Arg Gln Ser Gly Tyr Gly Thr Asp Gly Val Ala His
                  1205                1210                1215
   Asp Gln Pro Gly Tyr Arg Ala Asp Gly Val Ala Tyr Glu Gln Ser Gly
             1220                1225                1230
   Tyr Arg Ala Asp Gly Val Thr Tyr Asp Gln Ser Ala Asn Gln Thr Arg
        1235                1240                1245
   Lys Tyr His Gly Cys His Thr Val Gly Leu Pro His Pro Glu His Gly
      1250                1255                1260
   Cys Cys Tyr Pro Asp Arg Val Ser Asp Gly Gln Gln Leu Ala Tyr Val
   1265                1270                1275                1280
   Thr Lys Ser Ile Asp Leu Phe Pro Asp Thr Asp Lys Val Arg Ile Asp
                  1285                1290                1295
   Ile Gly Glu Thr Glu Gly Asn Phe Arg Val Glu Ser Val Glu Leu Ile
             1300                1305                1310
   Cys Met Glu Lys
        1315
<210> 5
   <211> 4188
   <212> DNA
   <213> Bacillus popilliae
<220>
   <221> CDS
   <222> (1)..(4158)
<400> 5
   atg ttg tgg cta aat act cat tgt tca tta ata gaa ata act ggg agg   48
   Met Leu Trp Leu Asn Thr His Cys Ser Leu Ile Glu Ile Thr Gly Arg
    1               5                  10                  15
   caa act atg aat caa tat cat aac caa aac gat aac aaa agt tac aac   96
   Gln Thr Met Asn Gln Tyr His Asn Gln Asn Asp Asn Lys Ser Tyr Asn
                   20                  25                  30
   caa agt gga aat gaa gtg caa att ata caa cct tca agt aac gct tta   144
   Gln Ser Gly Asn Glu Val Gln Ile Ile Gln Pro Ser Ser Asn Ala Leu
               35                  40                  45
   ctt tac agt ccc aac aag tat ccg tat gct acg gat ccc aat gtc ata   192
   Leu Tyr Ser Pro Asn Lys Tyr Pro Tyr Ala Thr Asp Pro Asn Val Ile
           50                  55                  60
   gca gag ggt aga agt tat aaa aat tgg ttg gat atg tgt gta ggt gta   240
   Ala Glu Gly Arg Ser Tyr Lys Asn Trp Leu Asp Met Cys Val Gly Val
       65                  70                  75                  80
   ggt gac gat aca cga agt ccc gaa gct cgg gtt act gca caa agt tct   288
   Gly Asp Asp Thr Arg Ser Pro Glu Ala Arg Val Thr Ala Gln Ser Ser
                   85                  90                  95
   att tcg act tct ctt ggt ata acc agc aca atc att ggc gct cta ggt   336
   Ile Ser Thr Ser Leu Gly Ile Thr Ser Thr Ile Ile Gly Ala Leu Gly
               100                 105                 110
   att ccg gtt gta ggc gaa gcc att gga att ttc ggc gca cta ttg gat   384
   Ile Pro Val Val Gly Glu Ala Ile Gly Ile Phe Gly Ala Leu Leu Asp
           115                 120                 125
   tgg ttg tgg cct gca ggc gct gat cca tgg gta ata ttt atg aat cat   432
   Trp Leu Trp Pro Ala Gly Ala Asp Pro Trp Val Ile Phe Met Asn His
       130                 135                 140
   gta gaa gaa ctc ata aat tca aaa ata aca gag act gta aaa aat gag   480
   Val Glu Glu Leu Ile Asn Ser Lys Ile Thr Glu Thr Val Lys Asn Glu
   145                 150                 155                 160
   gca att aca aga tta gac ggt tta ggc aat gtc cta gcg cta tat caa   528
   Ala Ile Thr Arg Leu Asp Gly Leu Gly Asn Val Leu Ala Leu Tyr Gln
                   165                 170                 175
   aag gct ttc gaa gaa tgg caa caa cat cct act ctt gaa tca gct cga   576
   Lys Ala Phe Glu Glu Trp Gln Gln His Pro Thr Leu Glu Ser Ala Arg
               180                 185                 190
   cta cgt gta aca gat gat ttt tct aat gta aat aaa ttt ttt gag gca   624
   Leu Arg Val Thr Asp Asp Phe Ser Asn Val Asn Lys Phe Phe Glu Ala
           195                 200                 205
   ttt atg cct tca ttc aga gta cca ggt tat gaa gta cca tta tta agc   672
   Phe Met Pro Ser Phe Arg Val Pro Gly Tyr Glu Val Pro Leu Leu Ser
       210                 215                 220
   gta tac gta tcc gct gca aac ctc cat tta tta tta tta aga gat agc   720
   Val Tyr Val Ser Ala Ala Asn Leu His Leu Leu Leu Leu Arg Asp Ser
   225                 230                 235                 240
   tcg att ttc ggt ttg gat tgg gga tta agt caa act cat gtt aac gat   768
   Ser Ile Phe Gly Leu Asp Trp Gly Leu Ser Gln Thr His Val Asn Asp
                   245                 250                 255
   aat tat aat ctc caa ata agg cgc tct gca gat tat gca aat cat tgt   816
   Asn Tyr Asn Leu Gln Ile Arg Arg Ser Ala Asp Tyr Ala Asn His Cys
               260                 265                 270
   aca act tgg tat cgg acg ggt tta caa aga ttg caa ggc acc aat gct   864
   Thr Thr Trp Tyr Arg Thr Gly Leu Gln Arg Leu Gln Gly Thr Asn Ala
           275                 280                 285
   agc agt tgg gtc aat tat aat cga ttt aga aga gaa atg aca cta act   912
   Ser Ser Trp Val Asn Tyr Asn Arg Phe Arg Arg Glu Met Thr Leu Thr
       290                 295                 300
   gta tta gat gtt tgt gca tta ttt tca agt tat gat tat cgt agt tac   960
   Val Leu Asp Val Cys Ala Leu Phe Ser Ser Tyr Asp Tyr Arg Ser Tyr
   305                 310                 315                 320
   cca atg gag cta agg gga gag ctt acg aga gaa att tat acg gat cca   1008
   Pro Met Glu Leu Arg Gly Glu Leu Thr Arg Glu Ile Tyr Thr Asp Pro
                   325                 330                 335
   gta gga gcc tct ttt tgg gtg aat aga gca cca aac ttc gca tca ata   1056
   Val Gly Ala Ser Phe Trp Val Asn Arg Ala Pro Asn Phe Ala Ser Ile
               340                 345                 350
   gaa aat aca gta gtt agg caa cca cac ccc ttt act tgg cta gtt act   1104
   Glu Asn Thr Val Val Arg Gln Pro His Pro Phe Thr Trp Leu Val Thr
           355                 360                 365
   tta aca gtt aat aca ggt caa gtg aga tct ggc gat gga aat tct aac   1152
   Leu Thr Val Asn Thr Gly Gln Val Arg Ser Gly Asp Gly Asn Ser Asn
       370                 375                 380
   tat tat tgg aaa tca cat agt caa acc gtg agt gaa acc gga ggg tca   1200
   Tyr Tyr Trp Lys Ser His Ser Gln Thr Val Ser Glu Thr Gly Gly Ser
   385                 390                 395                 400
   ggt cct att cag agt cct acc tgt gga agt act ggt aca att tat cgc   1248
   Gly Pro Ile Gln Ser Pro Thr Cys Gly Ser Thr Gly Thr Ile Tyr Arg
                   405                 410                 415
   acg gat aat tta ctt ttt aat cca ttt tta tta ggt gat att tat acc   1296
   Thr Asp Asn Leu Leu Phe Asn Pro Phe Leu Leu Gly Asp Ile Tyr Thr
               420                 425                 430
   att aat aca ggt tat gtt tct tat ctg gct aat ttg ttt gga atc tat   1344
   Ile Asn Thr Gly Tyr Val Ser Tyr Leu Ala Asn Leu Phe Gly Ile Tyr
           435                 440                 445
   tca gct aga ttt acg acg act cgt tct att gag ctt ctg tat gag aac   1392
   Ser Ala Arg Phe Thr Thr Thr Arg Ser Ile Glu Leu Leu Tyr Glu Asn
       450                 455                 460
   caa aga gtt ttc cca gcc tac aat cat caa att cgt gaa tta cct gga   1440
   Gln Arg Val Phe Pro Ala Tyr Asn His Gln Ile Arg Glu Leu Pro Gly
   465                 470                 475                 480
   gta aac tcg gat agg cca act gct gcc gac tat agt cat aga cta tcg   1488
   Val Asn Ser Asp Arg Pro Thr Ala Ala Asp Tyr Ser His Arg Leu Ser
                   485                 490                 495
   tat atc tca ggt ttt gca act gat gtg gga gga acg gtt cta gtt tat 1536
   Tyr Ile Ser Gly Phe Ala Thr Asp Val Gly Gly Thr Val Leu Val Tyr
   500 505 510
   ggg tgg aca tct tca act gct act cgt gag aat aat att acg cta gac 1584
   Gly Trp Thr Ser Ser Thr Ala Thr Arg Glu Asn Asn Ile Thr Leu Asp
   515 520 525
   gac aga ata gta caa ctt cca gct gtt aag gga aca agt ctc aac aat 1632
   Asp Arg Ile Val Gln Leu Pro Ala Val Lys Gly Thr Ser Leu Asn Asn
   530 535 540
   tgc caa gta gtt aga gga act gga ttt aca gga gga gac tgg ttg aag 1680
   Cys Gln Val Val Arg Gly Thr Gly Phe Thr Gly Gly Asp Trp Leu Lys
   545 550 555 560
   cct aat aat aat ggt aca ttt tct cta gct ctt ggt ttc agg tcg act 1728
   Pro Asn Asn Asn Gly Thr Phe Ser Leu Ala Leu Gly Phe Arg Ser Thr
   565 570 575
   tac act tac cgc ctc cgc att cgt tat gct gcc gca gca ggc gga tca 1776
   Tyr Thr Tyr Arg Leu Arg Ile Arg Tyr Ala Ala Ala Ala Gly Gly Ser
   580 585 590
   ggt ttt tct ctt gtt ata tcg gat caa tat gga gaa ttt cca acc aca 1824
   Gly Phe Ser Leu Val Ile Ser Asp Gln Tyr Gly Glu Phe Pro Thr Thr
   595 600 605
   aca gta tcg ctt tcc tcc aca atg tac tca ctg ccc caa aat gta cca 1872
   Thr Val Ser Leu Ser Ser Thr Met Tyr Ser Leu Pro Gln Asn Val Pro
   610 615 620
   tac gag gct ttt aag att gta gat tta cct tct act gtt act att aga 1920
   Tyr Glu Ala Phe Lys Ile Val Asp Leu Pro Ser Thr Val Thr Ile Arg
   625 630 635 640
   aat act tct cct gct tca aca act ttt cga ctt gat ttc cgt ttc att 1968
   Asn Thr Ser Pro Ala Ser Thr Thr Phe Arg Leu Asp Phe Arg Phe Ile
   645 650 655
   gtg cca tta gga ata ctc gca aat ata tta att gac cga att gaa ttt 2016
   Val Pro Leu Gly Ile Leu Ala Asn Ile Leu Ile Asp Arg Ile Glu Phe
   660 665 670
   gtt ccc att gag ggt tcc ttg ttc gag tac gaa acc aaa cag cag cta 2064
   Val Pro Ile Glu Gly Ser Leu Phe Glu Tyr Glu Thr Lys Gln Gln Leu
   675 680 685
   gaa aaa gca agg aaa gcg gtg aac cat ttg ttt aca gat gga tcg aaa 2112
   Glu Lys Ala Arg Lys Ala Val Asn His Leu Phe Thr Asp Gly Ser Lys
   690 695 700
   aag gcg cta aaa gaa ggc acg aca gat tat gag atc gat caa gcc gcc 2160
   Lys Ala Leu Lys Glu Gly Thr Thr Asp Tyr Glu Ile Asp Gln Ala Ala
   705 710 715 720
   aac gtg gtg gat tgt ata tcg gat gag tgt gga cat gag aaa atg atc 2208
   Asn Val Val Asp Cys Ile Ser Asp Glu Cys Gly His Glu Lys Met Ile
   725 730 735
   ctg ttg gat gaa gtg aaa tat gca aaa caa ctc agc caa gcc cgc aat 2256
   Leu Leu Asp Glu Val Lys Tyr Ala Lys Gln Leu Ser Gln Ala Arg Asn
   740 745 750
   tta ctg ctc aat ggg aat ttc gat gat cta tat cca gct ctg gag agg 2304
   Leu Leu Leu Asn Gly Asn Phe Asp Asp Leu Tyr Pro Ala Leu Glu Arg
   755 760 765
   gag aat cca tgg aaa aca agc ccg aat gtt acg atc cgt caa gat aac 2352
   Glu Asn Pro Trp Lys Thr Ser Pro Asn Val Thr Ile Arg Gln Asp Asn
   770 775 780
   ccg att ttt aaa ggc cat tat ctc agt atg gcg ggt gcg aac gat atc 2400
   Pro Ile Phe Lys Gly His Tyr Leu Ser Met Ala Gly Ala Asn Asp Ile
   785 790 795 800
   gag gcc acc aat gat acc ttc ccc acg tat gcc tat caa aaa ata gac 2448
   Glu Ala Thr Asn Asp Thr Phe Pro Thr Tyr Ala Tyr Gln Lys Ile Asp
   805 810 815
   gaa gcc aaa tta aag ccg tat aca cgt tat aaa gtg cgc ggg ttt gtt 2496
   Glu Ala Lys Leu Lys Pro Tyr Thr Arg Tyr Lys Val Arg Gly Phe Val
   820 825 830
   ggc agc agc aaa gct cta gag ctg ttg gtt aca cgc tat aat gaa gaa     2544
   Gly Ser Ser Lys Ala Leu Glu Leu Leu Val Thr Arg Tyr Asn Glu Glu
               835                 840                 845
   gtc gat gcg att tta gat gta ccg gat aat atc ccg cat gcg ccg ata     2592
   Val Asp Ala Ile Leu Asp Val Pro Asp Asn Ile Pro His Ala Pro Ile
           850                 855                 860
   cct gtc tgc ggt gaa ttt gat cga tgc aag ccc tat tcg tat cca cct     2640
   Pro Val Cys Gly Glu Phe Asp Arg Cys Lys Pro Tyr Ser Tyr Pro Pro
   865                 870                 875                 880
   tta ctt cca gaa tgt aac cct gag ttt ata aat cag atg caa cca tcc     2688
   Leu Leu Pro Glu Cys Asn Pro Glu Phe Ile Asn Gln Met Gln Pro Ser
                   885                 890                 895
   tct tgc cac cac act cag atg gtc gat tac aat aac atg aac atg agc     2736
   Ser Cys His His Thr Gln Met Val Asp Tyr Asn Asn Met Asn Met Ser
               900                 905                 910
   acg agt act acc atg aat cct acc ctt acg cct gaa ata gca tcc agc     2784
   Thr Ser Thr Thr Met Asn Pro Thr Leu Thr Pro Glu Ile Ala Ser Ser
           915                 920                 925
   caa agt gga ttc ggc aga aaa cat cgc aaa tgt cat caa gcg cat caa     2832
   Gln Ser Gly Phe Gly Arg Lys His Arg Lys Cys His Gln Ala His Gln
       930                 935                 940
   ttt gag ttc cat att gat acc ggg aca atc gat ctg gtc gaa gat ttg     2880
   Phe Glu Phe His Ile Asp Thr Gly Thr Ile Asp Leu Val Glu Asp Leu
   945                 950                 955                 960
   ggc att tgg gtg atc ttc aaa atc tgt gcc aca gat ggt tac gca agc     2928
   Gly Ile Trp Val Ile Phe Lys Ile Cys Ala Thr Asp Gly Tyr Ala Ser
                   965                 970                 975
   tta gat gat ttg gaa gtg att gaa gaa gga gcg ctg ggt gtc gaa gca     2976
   Leu Asp Asp Leu Glu Val Ile Glu Glu Gly Ala Leu Gly Val Glu Ala
               980                 985                 990
   tta gaa ctt gtc aag aaa aga gaa aag aaa tgg aga cat cag aag gag     3024
   Leu Glu Leu Val Lys Lys Arg Glu Lys Lys Trp Arg His Gln Lys Glu
           995                 1000                1005
   cag cac tgt tcg caa acg aaa cac aaa tat gat gcg gcc aaa cat gcg     3072
   Gln His Cys Ser Gln Thr Lys His Lys Tyr Asp Ala Ala Lys His Ala
       1010                1015                1020
   gtg atg gcg tta ttt aca aac acg cgc tat gaa aaa ttg aag ttc gaa     3120
   Val Met Ala Leu Phe Thr Asn Thr Arg Tyr Glu Lys Leu Lys Phe Glu
   1025                1030                1035                1040
   aca acc att tct gac att ttg tat gct gat cat ctc gtg cag tcg atc     3168
   Thr Thr Ile Ser Asp Ile Leu Tyr Ala Asp His Leu Val Gln Ser Ile
                   1045                1050                1055
   cct tat gta tat aat aaa tat gta ccg gaa gtt cca ggt atg aat tac     3216
   Pro Tyr Val Tyr Asn Lys Tyr Val Pro Glu Val Pro Gly Met Asn Tyr
               1060                1065                1070
   gaa ctc tat tca gag cta aac aca ctg gtt cag aat gcg ttc tac ctg     3264
   Glu Leu Tyr Ser Glu Leu Asn Thr Leu Val Gln Asn Ala Phe Tyr Leu
           1075                1080                1085
   tat gac cag cgg aat ctg att aaa aat ggg cgc ttt agc aat ggg ctt     3312
   Tyr Asp Gln Arg Asn Leu Ile Lys Asn Gly Arg Phe Ser Asn Gly Leu
       1090                1095                1100
   atg cat tgg caa gct act cct cat gca aga gta gag caa gaa cat gag     3360
   Met His Trp Gln Ala Thr Pro His Ala Arg Val Glu Gln Glu His Glu
   1105                1110                1115                1120
   aaa tcg gtg ctc gtg ctg cca aat tgg gat gcc aat gtg tcg caa gat     3408
   Lys Ser Val Leu Val Leu Pro Asn Trp Asp Ala Asn Val Ser Gln Asp
                   1125                1130                1135
   ctt tgt atc gaa cac aat cgc ggt tat gta ttg cgt gtc acg gcg aga     3456
   Leu Cys Ile Glu His Asn Arg Gly Tyr Val Leu Arg Val Thr Ala Arg
               1140                1145                1150
   aaa gaa gat ccg gga gcc ggc aat gtt acc ttt agt gac tgt gca aat     3504
   Lys Glu Asp Pro Gly Ala Gly Asn Val Thr Phe Ser Asp Cys Ala Asn
           1155                1160                1165
   cat gtc aac aag ctg agc ttt act tct tgc gat ata gct aca aac gca    3552
   His Val Asn Lys Leu Ser Phe Thr Ser Cys Asp Ile Ala Thr Asn Ala
       1170                1175                1180
   gtg cca ggt gcc caa gcg aat gat cca gcc gcc gga gta gcc tat gga    3600
   Val Pro Gly Ala Gln Ala Asn Asp Pro Ala Ala Gly Val Ala Tyr Gly
   1185                1190                1195                1200
   caa cag ggt tgt caa ata gat aga gtg ccg tac ggg caa tct gga tat    3648
   Gln Gln Gly Cys Gln Ile Asp Arg Val Pro Tyr Gly Gln Ser Gly Tyr
                   1205                1210                1215
   aga aca gac gga aca aat gga atg ccg tac ggg cag tct ggt aat cga    3696
   Arg Thr Asp Gly Thr Asn Gly Met Pro Tyr Gly Gln Ser Gly Asn Arg
               1220                1225                1230
   gcg gac ggg gtg cca tac aga caa tcc gga tat gga aca gat gga gta    3744
   Ala Asp Gly Val Pro Tyr Arg Gln Ser Gly Tyr Gly Thr Asp Gly Val
           1235                1240                1245
   gcg cac gac caa cct gga tat cga gca gac gga gca gcg tac gaa cag    3792
   Ala His Asp Gln Pro Gly Tyr Arg Ala Asp Gly Ala Ala Tyr Glu Gln
       1250                1255                1260
   tct ggt cat cga gca gac gga gta gcg tac gaa caa tct gga tat cga    3840
   Ser Gly His Arg Ala Asp Gly Val Ala Tyr Glu Gln Ser Gly Tyr Arg
   1265                1270                1275                1280
   gca ggt gga gta gcg tac gaa cag tct ggt cat cga gca gat gga gtg    3888
   Ala Gly Gly Val Ala Tyr Glu Gln Ser Gly His Arg Ala Asp Gly Val
                   1285                1290                1295
   ccg tac gga caa tct gga tat gga aca gac gga gta acg tac gac caa    3936
   Pro Tyr Gly Gln Ser Gly Tyr Gly Thr Asp Gly Val Thr Tyr Asp Gln
               1300                1305                1310
   tct gtc aaa caa acc cgc aaa tac cat ggt tgc cat aca gac ggg ctg    3984
   Ser Val Lys Gln Thr Arg Lys Tyr His Gly Cys His Thr Asp Gly Leu
           1315                1320                1325
   cca cat cca gag cat ggt tgt tgt tat cca gac aga gta agc gat ggc    4032
   Pro His Pro Glu His Gly Cys Cys Tyr Pro Asp Arg Val Ser Asp Gly
       1330                1335                1340
   caa cag ctt gca tat gta aca aaa tcg att gat ctg ttc ccg gat aca    4080
   Gln Gln Leu Ala Tyr Val Thr Lys Ser Ile Asp Leu Phe Pro Asp Thr
   1345                1350                1355                1360
   gat aaa gtc cgg atc gac att gga gaa acc gaa ggg aac ttt aga gtg    4128
   Asp Lys Val Arg Ile Asp Ile Gly Glu Thr Glu Gly Asn Phe Arg Val
                   1365                1370                1375
   gaa agt gtg gaa ttg att tgt atg gaa aag taaatcatca caagtaaaag      4178
   Glu Ser Val Glu Leu Ile Cys Met Glu Lys
               1380                1385
   tatcgtttac                                                         4188
<210> 6
   <211> 1386
   <212> PRT
   <213> Bacillus popilliae
<400> 6
<210> 7
   <211> 795
   <212> DNA
   <213> Bacillus popilliae
<220>
   <221> CDS
   <222> (1)..(795)
<400> 7
   caa tat cat aac caa aac gat aac aaa agt tac aac caa agt gga aat    48
   Gln Tyr His Asn Gln Asn Asp Asn Lys Ser Tyr Asn Gln Ser Gly Asn
    1               5                  10                  15
   gac atg caa att ata caa cct tca agt aac gct tta ctt tac agt ccc    96
   Asp Met Gln Ile Ile Gln Pro Ser Ser Asn Ala Leu Leu Tyr Ser Pro
               20                  25                  30
   aat aag tat ccg tat gcc acg gat ccc aat gtc ata gca gag ggg aga    144
   Asn Lys Tyr Pro Tyr Ala Thr Asp Pro Asn Val Ile Ala Glu Gly Arg
           35                  40                  45
   agt tat aat aat tgg ttg gat acg tgt gta ggt gta ggc gat ggt act    192
   Ser Tyr Asn Asn Trp Leu Asp Thr Cys Val Gly Val Gly Asp Gly Thr
       50                  55                  60
   cga agt ccc gag gct tat gct att gcc gaa gag gct gtt ggt ctt tcg    240
   Arg Ser Pro Glu Ala Tyr Ala Ile Ala Glu Glu Ala Val Gly Leu Ser
   65                  70                  75                  80
   att gat ctt ctc gcc gaa act ata tac ttt cta ggc ttt ccg att gca    288
   Ile Asp Leu Leu Ala Glu Thr Ile Tyr Phe Leu Gly Phe Pro Ile Ala
                   85                  90                  95
   tct cca atc act cgt gca cta tct gct tta ctg ggg ggg cta ttt tct    336
   Ser Pro Ile Thr Arg Ala Leu Ser Ala Leu Leu Gly Gly Leu Phe Ser
               100                 105                 110
   tct ggg gat acg ctg atg caa cat gta gaa caa ctc ata aat caa aaa    384
   Ser Gly Asp Thr Leu Met Gln His Val Glu Gln Leu Ile Asn Gln Lys
           115                 120                 125
   ata gaa att tat gca aga aat acg gca ctt gca gaa tta ttg ggt tta    432
   Ile Glu Ile Tyr Ala Arg Asn Thr Ala Leu Ala Glu Leu Leu Gly Leu
       130                 135                 140
   cgt aat gca tta gaa gtc tat agt gtg gct tta gaa tat tgg caa caa    480
   Arg Asn Ala Leu Glu Val Tyr Ser Val Ala Leu Glu Tyr Trp Gln Gln
   145                 150                 155                 160
   aat cgt aat agt gca caa gct caa gaa agt gta aga tca aga ttc cgt    528
   Asn Arg Asn Ser Ala Gln Ala Gln Glu Ser Val Arg Ser Arg Phe Arg
                   165                 170                 175
   agt cta gaa act ata ttt ata caa agg atg cct tta ttt gca ata caa    576
   Ser Leu Glu Thr Ile Phe Ile Gln Arg Met Pro Leu Phe Ala Ile Gln
               180                 185                 190
   ggt tat gag gtg ccg tta tta tcc gtg tat gca gcc gct gca aat ctc    624
   Gly Tyr Glu Val Pro Leu Leu Ser Val Tyr Ala Ala Ala Ala Asn Leu
           195                 200                 205
   cat tta cta tta tta agg gat agc tct att cac ggt tta gac tgg gga    672
   His Leu Leu Leu Leu Arg Asp Ser Ser Ile His Gly Leu Asp Trp Gly
       210                 215                 220
   ttt gat caa gga gag gtt aac agt aat tac gat cgc caa att agg ctc    720
   Phe Asp Gln Gly Glu Val Asn Ser Asn Tyr Asp Arg Gln Ile Arg Leu
   225                 230                 235                 240
   tct gca gag tat gca aat cat tgt ata act tgg tat cag gcg ggt tta    768
   Ser Ala Glu Tyr Ala Asn His Cys Ile Thr Trp Tyr Gln Ala Gly Leu
                   245                 250                 255
   caa gga ttg cag ggc acc agg ggg cga                                795
   Gln Gly Leu Gln Gly Thr Arg Gly Arg
               260                 265
<210> 8
   <211> 265
   <212> PRT
   <213> Bacillus popilliae
<400> 8
   Gln Tyr His Asn Gln Asn Asp Asn Lys Ser Tyr Asn Gln Ser Gly Asn
    1               5                  10                  15
   Asp Met Gln Ile Ile Gln Pro Ser Ser Asn Ala Leu Leu Tyr Ser Pro
               20                  25                  30
   Asn Lys Tyr Pro Tyr Ala Thr Asp Pro Asn Val Ile Ala Glu Gly Arg
           35                  40                  45
   Ser Tyr Asn Asn Trp Leu Asp Thr Cys Val Gly Val Gly Asp Gly Thr
       50                  55                  60
   Arg Ser Pro Glu Ala Tyr Ala Ile Ala Glu Glu Ala Val Gly Leu Ser
   65                  70                  75                  80
   Ile Asp Leu Leu Ala Glu Thr Ile Tyr Phe Leu Gly Phe Pro Ile Ala
                   85                  90                  95
   Ser Pro Ile Thr Arg Ala Leu Ser Ala Leu Leu Gly Gly Leu Phe Ser
               100                 105                 110
   Ser Gly Asp Thr Leu Met Gln His Val Glu Gln Leu Ile Asn Gln Lys
           115                 120                 125
   Ile Glu Ile Tyr Ala Arg Asn Thr Ala Leu Ala Glu Leu Leu Gly Leu
       130                 135                 140
   Arg Asn Ala Leu Glu Val Tyr Ser Val Ala Leu Glu Tyr Trp Gln Gln
   145                 150                 155                 160
   Asn Arg Asn Ser Ala Gln Ala Gln Glu Ser Val Arg Ser Arg Phe Arg
                   165                 170                 175
   Ser Leu Glu Thr Ile Phe Ile Gln Arg Met Pro Leu Phe Ala Ile Gln
               180                 185                 190
   Gly Tyr Glu Val Pro Leu Leu Ser Val Tyr Ala Ala Ala Ala Asn Leu
           195                 200                 205
   His Leu Leu Leu Leu Arg Asp Ser Ser Ile His Gly Leu Asp Trp Gly
       210                 215                 220
   Phe Asp Gln Gly Glu Val Asn Ser Asn Tyr Asp Arg Gln Ile Arg Leu
   225                 230                 235                 240
   Ser Ala Glu Tyr Ala Asn His Cys Ile Thr Trp Tyr Gln Ala Gly Leu
                   245                 250                 255
   Gln Gly Leu Gln Gly Thr Arg Gly Arg
               260                 265
<210> 9
   <211> 459
   <212> DNA
   <213> Bacillus popilliae
<220>
   <221> CDS
   <222> (1)..(459)
<400> 9
   tat gag gtc cca tta tta tcc gtg tat gca gac gct gca aat ctc cat    48
   Tyr Glu Val Pro Leu Leu Ser Val Tyr Ala Asp Ala Ala Asn Leu His
    1               5                   10                  15
   ttg cta ata tta agg gac agc tat att tac ggt gca ttc tgg ggg ttt    96
   Leu Leu Ile Leu Arg Asp Ser Tyr Ile Tyr Gly Ala Phe Trp Gly Phe
               20                  25                  30
   gat gaa gat gaa tat tac cgt aat tac gct cgc caa att agg ctc tcc   144
   Asp Glu Asp Glu Tyr Tyr Arg Asn Tyr Ala Arg Gln Ile Arg Leu Ser
           35                  40                  45
   gca gag tat gca aat cat tgt aca act tgg tat cag acg ggt tta cga   192
   Ala Glu Tyr Ala Asn His Cys Thr Thr Trp Tyr Gln Thr Gly Leu Arg
   aga ttg cag ggc acc cga gct aca gat tgg atc aat tat aat cga ttt   240
   Arg Leu Gln Gly Thr Arg Ala Thr Asp Trp Ile Asn Tyr Asn Arg Phe
   65                  70                  75                  80
   aga aga gaa ctg aca cta aca gta tta gat att tgt gca tta ttt tct   288
   Arg Arg Glu Leu Thr Leu Thr Val Leu Asp Ile Cys Ala Leu Phe Ser
                   85                  90                  95
   agc tat gat att cct agt tac ccg atg ggg aca aag ata cag ctt acg   336
   Ser Tyr Asp Ile Pro Ser Tyr Pro Met Gly Thr Lys Ile Gln Leu Thr
               100                 105                 110
   aga gaa att tat acc gat cca gta gta cac tct gac tgg ttg caa tca   384
   Arg Glu Ile Tyr Thr Asp Pro Val Val His Ser Asp Trp Leu Gln Ser
           115                 120                 125
   acg agt ccg gga tta ata tca ttc tca tca cta gaa aat cta gtc gtt   432
   Thr Ser Pro Gly Leu Ile Ser Phe Ser Ser Leu Glu Asn Leu Val Val
       130                 135                 140
   cgg gca cca cat ctt ttt act tgg ctt                               459
   Arg Ala Pro His Leu Phe Thr Trp Leu
   145                 150
<210> 10
   <211> 153
   <212> PRT
<213> Bacillus popilliae
<400> 10
   Tyr Glu Val Pro Leu Leu Ser Val Tyr Ala Asp Ala Ala Asn Leu His
     1             5                  10                  15
   Leu Leu Ile Leu Arg Asp Ser Tyr Ile Tyr Gly Ala Phe Trp Gly Phe
                   20                 25                  30
   Asp Glu Asp Glu Tyr Tyr Arg Asn Tyr Ala Arg Gln Ile Arg Leu Ser
   Ala Glu Tyr Ala Asn His Cys Thr Thr Trp Tyr Gln Thr Gly Leu Arg
           50                  55                 60
   Arg Leu Gln Gly Thr Arg Ala Thr Asp Trp Ile Asn Tyr Asn Arg Phe
       65                  70                  75                  80
   Arg Arg Glu Leu Thr Leu Thr Val Leu Asp Ile Cys Ala Leu Phe Ser
                   85                  90                  95
   Ser Tyr Asp Ile Pro Ser Tyr Pro Met Gly Thr Lys Ile Gln Leu Thr
               100                 105                 110
   Arg Glu Ile Tyr Thr Asp Pro Val Val His Ser Asp Trp Leu Gln Ser
           115                 120                 125
   Thr Ser Pro Gly Leu Ile Ser Phe Ser Ser Leu Glu Asn Leu Val Val
       130                 135                 140
   Arg Ala Pro His Leu Phe Thr Trp Leu
   145                 150
<210> 11
   <211> 25
   <212> DNA
   <213> Artificial Sequence
<220>
   <223> Description of Artificial Sequence: primer
<220>
   <221> misc_feature
   <222> (5,11,12)
   <223> n=a or g or c or t
<400> 11
   atgcngaagt nnatttacac gtgtt 25
<210> 12.
   <211> 17
   <212> DNA
   <213> Artificial Sequence
<220>
   <223> Description of Artificial Sequence: primer
<220>
   <221> misc_feature
   <222> (14,15)
   <223> n=a or g or c or t
<400> 12
   atgttgtggc taannac 17
<210> 13
   <211> 20
   <212> DNA
   <213> Artificial Sequence
<220>
   <223> Description of Artificial Sequence: primer
<400> 13
   gtaaacgata cttttacttg 20
<210> 14
   <211> 20
   <212> DNA
   <213> Artificial Sequence
<220>
   <223> Description of Artificial Sequence: primer
<400> 14
   tctcgatcac gaatgatcat 20
<210> 15
   <211> 20
   <212> DNA
   <213> Artificial Sequence
<220>
   <223> Description of Artificial Sequence: primer
<400> 15
   gtgtcatttc tcttctaaat 20
<210> 16
   <211> 30
   <212> DNA
   <213> Artificial Sequence
<220>
   <223> Description of Artificial Sequence: primer
<400> 16
   gtggaacaag aatatgagaa atctgtactc 30
<210> 17
   <211> 4359
   <212> DNA
   <213> Bacillus popilliae
<220>
   <221> CDS
   <222> (250)..(4245)
<400> 17
   ttgccagatg ttcatatcga ttgtcaatat gtgacggtat gtgatttaca aatgatgccc   60
   gttcatgagg gagcttgtca atttgtgaag attagcggag aatttcaatt ttattcactt  120
   taacattagc tacaaatgtt gtggctaagc accccattat ttatcaacgg gtaacaacca  180
   ggaggggtaa ctttgaatca gtatcaacat caaaacgata acaaaagtta caatcaagat  240
   ggaaatgaa atg cag atc ata caa cct tca agt aac gct tta ctt tac agt  291
             Met Gln Ile Ile Gln Pro Ser Ser Asn Ala Leu Leu Tyr Ser
               1               5                  10
   ccc aat aag tat ccg tat gcc acg gat ccc aat gtc ata gca gag ggg    339
   Pro Asn Lys Tyr Pro Tyr Ala Thr Asp Pro Asn Val Ile Ala Glu Gly
    15                  20                  25                  30
   aga agt tat aat aat tgg ttg gat acg tgt gta ggt gta ggc gat ggt    387
   Arg Ser Tyr Asn Asn Trp Leu Asp Thr Cys Val Gly Val Gly Asp Gly
                    35                  40                  45
   act cga agt ccc gag gct tat gct att gcc gaa gag gct gtc ggt ctt    435
   Thr Arg Ser Pro Glu Ala Tyr Ala Ile Ala Glu Glu Ala Val Gly Leu
                50                  55                  60
   tca att gat ata ttg gcc gaa att ata tac tat cta ggc ttc ccg att      483
   Ser Ile Asp Ile Leu Ala Glu Ile Ile Tyr Tyr Leu Gly Phe Pro Ile
           65                  70                  75
   gca tct cca ctc act cgt gca cta agt gct ata gcg gga cag cta ttt      531
   Ala Ser Pro Leu Thr Arg Ala Leu Ser Ala Ile Ala Gly Gln Leu Phe
       80                  85                  90
   tct tct ggg gat acg ctc atg caa cat att gaa caa ctc ata aat caa      579
   Ser Ser Gly Asp Thr Leu Met Gln His Ile Glu Gln Leu Ile Asn Gln
   95                  100                 105                 110
   aaa ata gcg gaa tat gcc aga aat aag gcg ctt gca gaa ttt cag ggt      627
   Lys Ile Ala Glu Tyr Ala Arg Asn Lys Ala Leu Ala Glu Phe Gln Gly
                   115                 120                 125
   tta ggt aga caa tat ggg tta tat tta gag gct tta gaa gat tgg gaa      675
   Leu Gly Arg Gln Tyr Gly Leu Tyr Leu Glu Ala Leu Glu Asp Trp Glu
               130                 135                 140
   caa aat cgt ctt agt caa cca cat aaa gag cgt gta aga caa aca ttc      723
   Gln Asn Arg Leu Ser Gln Pro His Lys Glu Arg Val Arg Gln Thr Phe
           145                 150                 155
   cgt att ctt gat aat agc ttt aca tca tct ata cct tca ttt gca gta      771
   Arg Ile Leu Asp Asn Ser Phe Thr Ser Ser Ile Pro Ser Phe Ala Val
       160                 165                 170
   cga aat tat gag gtt cca tta tta tcc gtg tat gca gac gct gca aat      819
   Arg Asn Tyr Glu Val Pro Leu Leu Ser Val Tyr Ala Asp Ala Ala Asn
   175                 180                 185                 190
   ctc cat ttg cta ata tta agg gac agc tat att tac ggt gca ttc tgg      867
   Leu His Leu Leu Ile Leu Arg Asp Ser Tyr Ile Tyr Gly Ala Phe Trp
                   195                 200                 205
   ggg ttt gat gaa gat gaa tat tac cgt aat tac gct cgc caa att agg      915
   Gly Phe Asp Glu Asp Glu Tyr Tyr Arg Asn Tyr Ala Arg Gln Ile Arg
               210                 215                 220
   ctc tcc gca gag tat gca aat cat tgt aca act tgg tat cag acg ggt      963
   Leu Ser Ala Glu Tyr Ala Asn His Cys Thr Thr Trp Tyr Gln Thr Gly
           225                 230                 235
   tta cga aga ttg cag ggc acc cga gct aca gat tgg atc aat tat aat      1011
   Leu Arg Arg Leu Gln Gly Thr Arg Ala Thr Asp Trp Ile Asn Tyr Asn
       240                 245                 250
   cga ttt aga aga gaa atg aca cta aca gta tta gat att tgt gca tta      1059
   Arg Phe Arg Arg Glu Met Thr Leu Thr Val Leu Asp Ile Cys Ala Leu
   255                 260                 265                 270
   ttt tct agc tat gat att cct agt tac ccg atg ggg aca aag ata cag      1107
   Phe Ser Ser Tyr Asp Ile Pro Ser Tyr Pro Met Gly Thr Lys Ile Gln
                   275                 280                 285
   ctt acg aga gaa att tat acc gat cca gta gta cac tct gac tgg ttg      1155
   Leu Thr Arg Glu Ile Tyr Thr Asp Pro Val Val His Ser Asp Trp Leu
               290                 295                 300
   caa tca acg agt ccg gga tta ata tca ttc tca tca cta gaa aat cta      1203
   Gln Ser Thr Ser Pro Gly Leu Ile Ser Phe Ser Ser Leu Glu Asn Leu
           305                 310                 315
   gtc gtt cgg gca cca cat ctt ttt act tgg ctt tct cga gta aca att      1251
   Val Val Arg Ala Pro His Leu Phe Thr Trp Leu Ser Arg Val Thr Ile
       320                 325                 330
   gat aca ggt ata ttg tct act gta att ggc ggg cag tat agt aat aac      1299
   Asp Thr Gly Ile Leu Ser Thr Val Ile Gly Gly Gln Tyr Ser Asn Asn
   335                 340                 345                 350
   aat ttt tgg cga aca cat tat caa act ttg cgt aca acc ggg ggc aca      1347
   Asn Phe Trp Arg Thr His Tyr Gln Thr Leu Arg Thr Thr Gly Gly Thr
                   355                 360                 365
   tct ttc caa agt cct acc tat ggc tcg aca gcg ttt cca att caa cgc      1395
   Ser Phe Gln Ser Pro Thr Tyr Gly Ser Thr Ala Phe Pro Ile Gln Arg
               370                 375                 380
   acg aat aca ttg act ttc tcc ggc gat gtt tac acc ata gag tca agt      1443
   Thr Asn Thr Leu Thr Phe Ser Gly Asp Val Tyr Thr Ile Glu Ser Ser
           385                 390                 395
   gtt gtt acg agg agt tcc ttg tat gga gct aat tcg gtt gca ttt acg      1491
   Val Val Thr Arg Ser Ser Leu Tyr Gly Ala Asn Ser Val Ala Phe Thr
       400                 405                 410
   ggt act act ggt cgg tca cta tat gag aac cca acg gtt tat ccg ttt      1539
   Gly Thr Thr Gly Arg Ser Leu Tyr Glu Asn Pro Thr Val Tyr Pro Phe
   415                 420                 425                 430
   gca caa aaa tta att cat gaa tta cct gga gta gac tca ggt aga cca      1587
   Ala Gln Lys Leu Ile His Glu Leu Pro Gly Val Asp Ser Gly Arg Pro
                   435                 440                 445
   aat gct acc aac tat agt cat aga ctg tcg tat atc tca ggt ttc agt      1635
   Asn Ala Thr Asn Tyr Ser His Arg Leu Ser Tyr Ile Ser Gly Phe Ser
               450                 455                 460
   ctt ggt tat tct cct tct gga acg ggt cta gtt tat ggg tgg aca tct      1683
   Leu Gly Tyr Ser Pro Ser Gly Thr Gly Leu Val Tyr Gly Trp Thr Ser
           465                 470                 475
   aca act gct act cgt gag aat aat att acg cta gac gac aga ata gta      1731
   Thr Thr Ala Thr Arg Glu Asn Asn Ile Thr Leu Asp Asp Arg Ile Val
       480                 485                 490
   cag ctt cca gct gtt aag gga gca agt ctc aat aat tgc caa gta gta      1779
   Gln Leu Pro Ala Val Lys Gly Ala Ser Leu Asn Asn Cys Gln Val Val
   495                 500                 505                 510
   aaa ggg act gga ttt aca gga gga gac tgg ttg aaa cct aat aat aat      1827
   Lys Gly Thr Gly Phe Thr Gly Gly Asp Trp Leu Lys Pro Asn Asn Asn
                   515                 520                 525
   ggt aca ttt tct atg tac ttt gca ttc agg tcg gct tac act tac cac      1875
   Gly Thr Phe Ser Met Tyr Phe Ala Phe Arg Ser Ala Tyr Thr Tyr His
               530                 535                 540
   ttc cgc att cgt tat gct tcc tca gca agt ttt tct ttt gtt ata tcg      1923
   Phe Arg Ile Arg Tyr Ala Ser Ser Ala Ser Phe Ser Phe Val Ile Ser
           545                 550                 555
   gaa gaa tat gga cgt ttt cca acc aca aca gtg ccg ctt ctc tcc aca      1971
   Glu Glu Tyr Gly Arg Phe Pro Thr Thr Thr Val Pro Leu Leu Ser Thr
       560                 565                 570
   atg tca cca ctg ccc caa aat aca cca ttc gaa gct ttt aag act gta      2019
   Met Ser Pro Leu Pro Gln Asn Thr Pro Phe Glu Ala Phe Lys Thr Val
   575                 580                 585                 590
   gat tta cct tct act gtt act att aga tat act tct gct gct tca aca      2067
   Asp Leu Pro Ser Thr Val Thr Ile Arg Tyr Thr Ser Ala Ala Ser Thr
                   595                 600                 605
   act ttt caa ctt aat ttc cgt ttc act gtg cca gga agc gca aat gta      2115
   Thr Phe Gln Leu Asn Phe Arg Phe Thr Val Pro Gly Ser Ala Asn Val
               610                 615                 620
   ttg att gac cga att gaa ttt gtt cca att gag ggt tcc ttg ttc gag      2163
   Leu Ile Asp Arg Ile Glu Phe Val Pro Ile Glu Gly Ser Leu Phe Glu
           625                 630                 635
   tac gaa acc aaa cag cag cta gaa aaa gca agg aaa gcg gtg aac cat      2211
   Tyr Glu Thr Lys Gln Gln Leu Glu Lys Ala Arg Lys Ala Val Asn His
       640                 645                 650
   ttg ttt aca gat gga tcg aaa aag gcg cta aaa gaa gac acg acc gat      2259
   Leu Phe Thr Asp Gly Ser Lys Lys Ala Leu Lys Glu Asp Thr Thr Asp
   655                 660                 665                 670
   tat gag att gat caa gcc gcc aac gtg gta gat tgt ata tcg gat gag      2307
   Tyr Glu Ile Asp Gln Ala Ala Asn Val Val Asp Cys Ile Ser Asp Glu
                   675                 680                 685
   tgt gga cat gag aaa atg atc ctg tta gat gaa gta aaa tat gca aaa      2355
   Cys Gly His Glu Lys Met Ile Leu Leu Asp Glu Val Lys Tyr Ala Lys
               690                 695                 700
   caa ctc agc caa gcc cgc aat tta ctg ctc aat ggg aat ttc gat gat      2403
   Gln Leu Ser Gln Ala Arg Asn Leu Leu Leu Asn Gly Asn Phe Asp Asp
           705                 710                 715
   cta tat cca gct ctg gag agg gag aat cca tgg aaa aca agt ccg aat      2451
   Leu Tyr Pro Ala Leu Glu Arg Glu Asn Pro Trp Lys Thr Ser Pro Asn
       720                 725                 730
   gtt acg atc cgt caa gat aac ccg att ttt aaa ggc cat tat ctc agt      2499
   Val Thr Ile Arg Gln Asp Asn Pro Ile Phe Lys Gly His Tyr Leu Ser
   735                 740                 745                 750
   atg gcg ggt gcg aac gat atc gag gcc acc aat gat acg ttc ccc acg      2547
   Met Ala Gly Ala Asn Asp Ile Glu Ala Thr Asn Asp Thr Phe Pro Thr
                   755                 760                 765
   tat gtc tat caa aaa ata gat gaa gcc aaa tta aag cca tat aca cgg      2595
   Tyr Val Tyr Gln Lys Ile Asp Glu Ala Lys Leu Lys Pro Tyr Thr Arg
               770                 775                 780
   tat aaa gtg cgc ggg ttt gtt ggc agc agc aaa gat ctg gag ctg ttg      2643
   Tyr Lys Val Arg Gly Phe Val Gly Ser Ser Lys Asp Leu Glu Leu Leu
           785                 790                 795
   gtt aca cgc tat aat gaa gaa gtt gat gcg att tta gat gta ccg gat      2691
   Val Thr Arg Tyr Asn Glu Glu Val Asp Ala Ile Leu Asp Val Pro Asp
       800                 805                 810
   aat atc ccg cat gcg ccg act cct gtc tgc ggt gaa ttt gat cga tgc      2739
   Asn Ile Pro His Ala Pro Thr Pro Val Cys Gly Glu Phe Asp Arg Cys
   815                 820                 825                 830
   aag ccc tat tcg tat cca cct tta ctt cca gaa tgt aac cct gag ttt      2787
   Lys Pro Tyr Ser Tyr Pro Pro Leu Leu Pro Glu Cys Asn Pro Glu Phe
                   835                 840                 845
   ata aat cag atg caa cca tcc tct tgc cac cac aat cag atg gtc gat      2835
   Ile Asn Gln Met Gln Pro Ser Ser Cys His His Asn Gln Met Val Asp
               850                 855                 860
   tac aat aac atg aac acg agc acg agt act acc atg aat cct agc atg      2883
   Tyr Asn Asn Met Asn Thr Ser Thr Ser Thr Thr Met Asn Pro Ser Met
           865                 870                 875
   aat cct ccc ctt acg cct gaa ata gca tcc agc caa agt gga ttc ggc      2931
   Asn Pro Pro Leu Thr Pro Glu Ile Ala Ser Ser Gln Ser Gly Phe Gly
       880                 885                 890
   aga aaa cat cgc aaa tgt cat caa gcg cat caa ttt gag ttc cac att      2979
   Arg Lys His Arg Lys Cys His Gln Ala His Gln Phe Glu Phe His Ile
   895                 900                 905                 910
   gat acc ggg aca atc gat ttg gtg gaa gat ttg ggc att tgg gtg atc      3027
   Asp Thr Gly Thr Ile Asp Leu Val Glu Asp Leu Gly Ile Trp Val Ile
                   915                 920                 925
   ttc aaa atc tgt gcc aca gat gga tac gca agc tta gat gat ctg gaa      3075
   Phe Lys Ile Cys Ala Thr Asp Gly Tyr Ala Ser Leu Asp Asp Leu Glu
               930                 935                 940
   gtg att gaa gaa gga gcg ctg ggg gtc gaa gcc tta gaa ctt gtc aag      3123
   Val Ile Glu Glu Gly Ala Leu Gly Val Glu Ala Leu Glu Leu Val Lys
           945                 950                 955
   aaa aga gaa aag aaa tgg aga cat cag aag gag cag cac tgt tcg caa      3171
   Lys Arg Glu Lys Lys Trp Arg His Gln Lys Glu Gln His Cys Ser Gln
       960                 965                 970
   acg aaa cac aaa tat gat gcg gcc aaa cat gcg gtg atg gcg tta ttt      3219
   Thr Lys His Lys Tyr Asp Ala Ala Lys His Ala Val Met Ala Leu Phe
   975                 980                 985                 990
   aca aac acg cgc tat gaa aaa ttg aag ttc gaa aca acc att tct gac      3267
   Thr Asn Thr Arg Tyr Glu Lys Leu Lys Phe Glu Thr Thr Ile Ser Asp
                   995                 1000                1005
   att ttg tat gct gat cat ctc gtg cag tcg att cct tat gta tat aat      3315
   Ile Leu Tyr Ala Asp His Leu Val Gln Ser Ile Pro Tyr Val Tyr Asn
               1010                1015                1020
   aaa tat gta ccg gaa gtt tca ggt atg aat tac gaa ctc tat aca gag      3363
   Lys Tyr Val Pro Glu Val Ser Gly Met Asn Tyr Glu Leu Tyr Thr Glu
           1025                1030                1035
   cta aac act ctc gtt cag aat gcg ttc tac ctg tat gac cag cgg aat      3411
   Leu Asn Thr Leu Val Gln Asn Ala Phe Tyr Leu Tyr Asp Gln Arg Asn
       1040                1045                1050
   ctg att aaa aat ggg cgc ttt agc aat ggg ctt atg tat tgg caa gct      3459
   Leu Ile Lys Asn Gly Arg Phe Ser Asn Gly Leu Met Tyr Trp Gln Ala
   1055                1060                1065                1070
   acc ccg cat gca cga gtg gag caa gaa tat gat aga tca gtg ctg gtg      3507
   Thr Pro His Ala Arg Val Glu Gln Glu Tyr Asp Arg Ser Val Leu Val
                   1075                1080                1085
   ctg ccg aat tgg gat gcc aat gtg tcg caa cag ctg tgt atc gaa cac      3555
   Leu Pro Asn Trp Asp Ala Asn Val Ser Gln Gln Leu Cys Ile Glu His
               1090                1095                1100
   aat cgc ggt tat gta ttg cgt gtc acg gcg aga aaa gaa gat ccg gga      3603
   Asn Arg Gly Tyr Val Leu Arg Val Thr Ala Arg Lys Glu Asp Pro Gly
           1105                1110                1115
   gcc ggc aat gtt acc ttt agt gac tgt gca aat cat gtc gac aag ctg      3651
   Ala Gly Asn Val Thr Phe Ser Asp Cys Ala Asn His Val Asp Lys Leu
       1120                1125                1130
   agc ttt act tct tgc gat ata gct aca aac gca gtg cca ggt gcc caa      3699
   Ser Phe Thr Ser Cys Asp Ile Ala Thr Asn Ala Val Pro Gly Ala Gln
   1135                1140                1145                1150
   gcg aat gat cca gcc gcc gga gta gcc tat gga caa cag ggt tgt caa      3747
   Ala Asn Asp Pro Ala Ala Gly Val Ala Tyr Gly Gln Gln Gly Cys Gln
                   1155                1160                1165
   ata gat aga gtg ccg tac gga cca tct gga tat cga gca gac gga gta      3795
   Ile Asp Arg Val Pro Tyr Gly Pro Ser Gly Tyr Arg Ala Asp Gly Val
               1170                1175                1180
   gcg tac gaa cag tct ggt cat cga aca gat gga gtg ccg tac aga caa      3843
   Ala Tyr Glu Gln Ser Gly His Arg Thr Asp Gly Val Pro Tyr Arg Gln
           1185                1190                1195
   tct gga tat cga gca gac gga gta gcg cac gac caa cct gga tat cga      3891
   Ser Gly Tyr Arg Ala Asp Gly Val Ala His Asp Gln Pro Gly Tyr Arg
       1200                1205                1210
   gca gac gga gta gcg tac gaa caa tct gga tat cga gca gat gga gta      3939
   Ala Asp Gly Val Ala Tyr Glu Gln Ser Gly Tyr Arg Ala Asp Gly Val
   1215                1220                1225                1230
   gcg tac gaa cag tct ggt cat cga gca gat gga gtg ccg tac gga caa      3987
   Ala Tyr Glu Gln Ser Gly His Arg Ala Asp Gly Val Pro Tyr Gly Gln
                   1235                1240                1245
   tct gga tat gga aca gac gga gta acg tac gac caa tct gcc aaa caa      4035
   Ser Gly Tyr Gly Thr Asp Gly Val Thr Tyr Asp Gln Ser Ala Lys Gln
               1250                1255                1260
   acc cgc aaa tac cat ggt tgc cat aca gac gga ctg cca cat cca gag      4083
   Thr Arg Lys Tyr His Gly Cys His Thr Asp Gly Leu Pro His Pro Glu
           1265                1270                1275
   cat ggt tgt tgt tat cca gac aga gta agc gat ggc caa cag ctt gca      4131
   His Gly Cys Cys Tyr Pro Asp Arg Val Ser Asp Gly Gln Gln Leu Ala
       1280                1285                1290
   tat gta aca aaa tcg att gat ctg ttc ccg gat aca gat aaa gtc cgg      4179
   Tyr Val Thr Lys Ser Ile Asp Leu Phe Pro Asp Thr Asp Lys Val Arg
   1295                1300                1305                1310
   atc gac att gga gaa acc gaa ggg aac ttt aga gtg gaa agt gtg gaa      4227
   Ile Asp Ile Gly Glu Thr Glu Gly Asn Phe Arg Val Glu Ser Val Glu
                   1315                1320                1325
   ttg att tgt atg gaa aag taaatcatca caagtaaaag tatcgtttac             4275
   Leu Ile Cys Met Glu Lys
               1330
   taaaaattta ttttccaagc aacaggggag aggggggctg tccagaaggt cagtgaaaac    4335
   tggacgcccc gttttagtag aata                                           4359
<210> 18
   <211> 1332
   <212> PRT
   <213> Bacillus popilliae
<400> 18
<210> 19
   <211> 4366
   <212> DNA
   <213> Bacillus popilliae
<220>
   <221> CDS
   <222> (224)..(4255)
<400> 19
   gtagatcatg tactcaaatg cagtgtcgga agtttgccag atgttcatat cgattgtcaa 60
   tatgtgacgg tatgtgattt acagatgagg cccgttcatg agggagcttg tcaatttgtg 120
   aagattagcg gagaatttca attttattca ctttaacatt agctacaaat gttgtggcta 180
   agcaccccat tatataaatg gatgacaacc aggaggggta act atg aat cag tat   235
                                                   Met Asn Gln Tyr
                                                     1
   cat aac caa aac gat aac aaa agt tac aac caa agt gga aat gaa atg   283
   His Asn Gln Asn Asp Asn Lys Ser Tyr Asn Gln Ser Gly Asn Glu Met
     5                  10                  15                  20
   caa atc att caa cct tca agt aat tct tta ctt tac agt ccc aat aag   331
   Gln Ile Ile Gln Pro Ser Ser Asn Ser Leu Leu Tyr Ser Pro Asn Lys
                   25                  30                  35
   tat ccg tat gcc acg gat ccc aat gtc ata gca gag ggt aga agt tat   379
   Tyr Pro Tyr Ala Thr Asp Pro Asn Val Ile Ala Glu Gly Arg Ser Tyr
               40                  45                  50
   aaa aat tgg ctt gat atg tgt gta ggt gaa ggc gac ggt aca cga agt   427
   Lys Asn Trp Leu Asp Met Cys Val Gly Glu Gly Asp Gly Thr Arg Ser
           55                  60                  65
   ctc gag gct att gct gtt gct gtc gga gtt cga ata agc cac aca att   475
   Leu Glu Ala Ile Ala Val Ala Val Gly Val Arg Ile Ser His Thr Ile
       70                  75                  80
   ttc cgc ctt tta ggt gtt cca tat tca gct caa ggc gag caa tta ttt   523
   Phe Arg Leu Leu Gly Val Pro Tyr Ser Ala Gln Gly Glu Gln Leu Phe
   85                  90                  95                  100
   agc ttc cta ttg gat acg tta tgg ctt gaa ggc aat act caa tgg gaa   571
   Ser Phe Leu Leu Asp Thr Leu Trp Leu Glu Gly Asn Thr Gln Trp Glu
                   105                 110                 115
   gag ttg atg aga cat gca gaa gaa ctc ata aat gaa cag gta ccg gat   619
   Glu Leu Met Arg His Ala Glu Glu Leu Ile Asn Glu Gln Val Pro Asp
               120                 125                 130
   tat gta aga acc aag gca ctt gca gaa tta acg gat tta ggt aac aac   667
   Tyr Val Arg Thr Lys Ala Leu Ala Glu Leu Thr Asp Leu Gly Asn Asn
           135                 140                 145
   tta aat tta tat ata gca gct ttt gaa gat tgg aaa cga aat ccg agc   715
   Leu Asn Leu Tyr Ile Ala Ala Phe Glu Asp Trp Lys Arg Asn Pro Ser
       150                 155                 160
   agt caa gaa gtt aga acc cgg gta ata gat aga ttc aat ata ctc gac   763
   Ser Gln Glu Val Arg Thr Arg Val Ile Asp Arg Phe Asn Ile Leu Asp
   165                 170                 175                 180
   ggt tta ttt gaa gcc tat ctg cct tca ttt gca gta cct ggt tat gaa   811
   Gly Leu Phe Glu Ala Tyr Leu Pro Ser Phe Ala Val Pro Gly Tyr Glu
                   185                 190                 195
   gta cca cta tta tcc gtg tat gca aat gtt gta aat atc cac tta ttg   859
   Val Pro Leu Leu Ser Val Tyr Ala Asn Val Val Asn Ile His Leu Leu
               200                 205                 210
   gta ctg agg gac agc tcg att tat ggt ctg gat tgg gga tta agt tca   907
   Val Leu Arg Asp Ser Ser Ile Tyr Gly Leu Asp Trp Gly Leu Ser Ser
           215                 220                 225
   act agt gtt gac aat aat tac aat cgc caa caa agg aac tcc gca acg   955
   Thr Ser Val Asp Asn Asn Tyr Asn Arg Gln Gln Arg Asn Ser Ala Thr
       230                 235                 240
   tat gca aat cat tgt aca act tgg tat cag acg ggt tta caa aga ttg   1003
   Tyr Ala Asn His Cys Thr Thr Trp Tyr Gln Thr Gly Leu Gln Arg Leu
   245                 250                 255                 260
   caa ggc agc gat gct agc agt tgg gtc aat tat aat cga ttt aga aga   1051
   Gln Gly Ser Asp Ala Ser Ser Trp Val Asn Tyr Asn Arg Phe Arg Arg
                   265                 270                 275
   gaa ata acg tta ata gta ttg gat att tgt gca ttg ttt tca aat tat   1099
   Glu Ile Thr Leu Ile Val Leu Asp Ile Cys Ala Leu Phe Ser Asn Tyr
               280                 285                 290
   gat gtt cgt agt tat cca ata cag tta cgg gga gag ctt acg aga gga   1147
   Asp Val Arg Ser Tyr Pro Ile Gln Leu Arg Gly Glu Leu Thr Arg Gly
           295                 300                 305
   att tat acg gat cca gca gta tat agc ggt aca ggt tcc tat tcc tgg   1195
   Ile Tyr Thr Asp Pro Ala Val Tyr Ser Gly Thr Gly Ser Tyr Ser Trp
       310                 315                 320
   ttg agt caa gca cca tca ttt gca gaa ata gaa aat atc gca att agg   1243
   Leu Ser Gln Ala Pro Ser Phe Ala Glu Ile Glu Asn Ile Ala Ile Arg
   325                 330                 335                 340
   gaa cca agc aat ttt act tgg gca tct tat gcg aga gta aca aca ggt   1291
   Glu Pro Ser Asn Phe Thr Trp Ala Ser Tyr Ala Arg Val Thr Thr Gly
                   345                 350                 355
   aca ctg gaa tat ctc agc tct aag aat gat ttt tgg aaa tca cac tat    1339
   Thr Leu Glu Tyr Leu Ser Ser Lys Asn Asp Phe Trp Lys Ser His Tyr
               360                 365                 370
   atg aac tat act gaa acc aat tcg ggt ata ttg att caa gga cct acc    1387
   Met Asn Tyr Thr Glu Thr Asn Ser Gly Ile Leu Ile Gln Gly Pro Thr
           375                 380                 385
   tat gga atg acg acg ggt aca aat att cgt ata gag tcc gta tca atg    1435
   Tyr Gly Met Thr Thr Gly Thr Asn Ile Arg Ile Glu Ser Val Ser Met
       390                 395                 400
   caa gaa att tat tcc gtt aga tta gaa gct gtt gct cat gct gga gct    1483
   Gln Glu Ile Tyr Ser Val Arg Leu Glu Ala Val Ala His Ala Gly Ala
   405                 410                 415                 420
   ggg ggt cct ttt ttg gga atc tct acg tct gaa ttt ttc tgg agt ttg    1531
   Gly Gly Pro Phe Leu Gly Ile Ser Thr Ser Glu Phe Phe Trp Ser Leu
                   425                 430                 435
   ggt gtt aga agg tat cag aac tca cgt agt cct caa ttt gcg tct caa    1579
   Gly Val Arg Arg Tyr Gln Asn Ser Arg Ser Pro Gln Phe Ala Ser Gln
               440                 445                 450
   ata ata act agg caa tta cct gga gta aac tca gcg gtt cca tct gcc    1627
   Ile Ile Thr Arg Gln Leu Pro Gly Val Asn Ser Ala Val Pro Ser Ala
           455                 460                 465
   ctc gac cat agt cat gaa cta tcg tat atc aca gcg ttt cca gtt aga    1675
   Leu Asp His Ser His Glu Leu Ser Tyr Ile Thr Ala Phe Pro Val Arg
       470                 475                 480
   tcg gtg gga acg att ctc gtt cat gaa tgg aca tct aca aca gtt agt    1723
   Ser Val Gly Thr Ile Leu Val His Glu Trp Thr Ser Thr Thr Val Ser
   485                 490                 495                 500
   cgt aac aat aga att gag cca gat aaa ata aca caa atc ccg gct gtt    1771
   Arg Asn Asn Arg Ile Glu Pro Asp Lys Ile Thr Gln Ile Pro Ala Val
                   505                 510                 515
   aag tca cac aca ctc tcc aat tgt caa gta gtt agt ggg act ggg ttt    1819
   Lys Ser His Thr Leu Ser Asn Cys Gln Val Val Ser Gly Thr Gly Phe
               520                 525                 530
   acg gga gga aac tgg ttg aga cct tct gat aat ggt tca ttt aga cta    1867
   Thr Gly Gly Asn Trp Leu Arg Pro Ser Asp Asn Gly Ser Phe Arg Leu
           535                 540                 545
   acg att act tca ttc tca agc caa tct tac cgc att cgc att cat tat    1915
   Thr Ile Thr Ser Phe Ser Ser Gln Ser Tyr Arg Ile Arg Ile His Tyr
       550                 555                 560
   gct tcc gca aca ttt ttt tat ttg gat att cgt acg ggt gat act tct    1963
   Ala Ser Ala Thr Phe Phe Tyr Leu Asp Ile Arg Thr Gly Asp Thr Ser
   565                 570                 575                 580
   aac aca ttt gcg gtt acc cca aca aca tta tca tca gga tcc caa act    2011
   Asn Thr Phe Ala Val Thr Pro Thr Thr Leu Ser Ser Gly Ser Gln Thr
                   585                 590                 595
   gta ccc tac gaa tct ttt ggg ttt ata aat ata cct tat act ttt aca    2059
   Val Pro Tyr Glu Ser Phe Gly Phe Ile Asn Ile Pro Tyr Thr Phe Thr
               600                 605                 610
   aca gca cct act gaa agt aga tat act ttt gat ttc atg ttc tac tca    2107
   Thr Ala Pro Thr Glu Ser Arg Tyr Thr Phe Asp Phe Met Phe Tyr Ser
           615                 620                 625
   ata gga agc gca aat gta tta att gac cga att gaa att gtt cca atc    2155
   Ile Gly Ser Ala Asn Val Leu Ile Asp Arg Ile Glu Ile Val Pro Ile
       630                 635                 640
   gga gtt cct ttg ttc gag tac gaa acc aaa cag cag cta gaa aaa gca    2203
   Gly Val Pro Leu Phe Glu Tyr Glu Thr Lys Gln Gln Leu Glu Lys Ala
   645                 650                 655                 660
   agg aaa gcg gtg aac cat ttg ttt aca gat gga tcg aaa aag gcg cta    2251
   Arg Lys Ala Val Asn His Leu Phe Thr Asp Gly Ser Lys Lys Ala Leu
                   665                 670                 675
   aaa gaa gac acg acc gat tat gag att gat caa gcc gcc aac gtg gta    2299
   Lys Glu Asp Thr Thr Asp Tyr Glu Ile Asp Gln Ala Ala Asn Val Val
               680                 685                 690
   gat tgt ata tcg gat gag tgt gga cat gat aaa atg atc ctg tta gat   2347
   Asp Cys Ile Ser Asp Glu Cys Gly His Asp Lys Met Ile Leu Leu Asp
           695                 700                 705
   gaa gta aaa tat gca aaa caa ctc agc caa gcc cgc aat tta ctg ctc   2395
   Glu Val Lys Tyr Ala Lys Gln Leu Ser Gln Ala Arg Asn Leu Leu Leu
       710                 715                 720
   aat ggg aat ttc gat gat cta tat tca gct ctg gag aag gag aat cca   2443
   Asn Gly Asn Phe Asp Asp Leu Tyr Ser Ala Leu Glu Lys Glu Asn Pro
   725                 730                 735                 740
   tgg aaa aca agt ccg aat gtt acg atc cga caa gat aac ccg att ttt   2491
   Trp Lys Thr Ser Pro Asn Val Thr Ile Arg Gln Asp Asn Pro Ile Phe
                   745                 750                 755
   aaa ggc cat tat ctc agt atg gcg ggt gcg aac gat atc gag gcc acc   2539
   Lys Gly His Tyr Leu Ser Met Ala Gly Ala Asn Asp Ile Glu Ala Thr
               760                 765                 770
   aat gat acc ttc ccc acg tat gtc tat caa aaa ata gac gaa gcc aaa   2587
   Asn Asp Thr Phe Pro Thr Tyr Val Tyr Gln Lys Ile Asp Glu Ala Lys
           775                 780                 785
   tta aag ccg tat aca cgt tat aaa gtg cgc ggg ttt gtt ggc agc agc   2635
   Leu Lys Pro Tyr Thr Arg Tyr Lys Val Arg Gly Phe Val Gly Ser Ser
       790                 795                 800
   aaa gct cta gag ctg ttg gtt aca cgc tat aat gaa gaa gtt gat gcg   2683
   Lys Ala Leu Glu Leu Leu Val Thr Arg Tyr Asn Glu Glu Val Asp Ala
   805                 810                 815                 820
   att tta gat gta ccg gat aat atc ccg cat gcg ccg act cct gtc tgc   2731
   Ile Leu Asp Val Pro Asp Asn Ile Pro His Ala Pro Thr Pro Val Cys
                   825                 830                 835
   ggt gaa ttt gat cga tgc aag ccc tat tcg tat cca cct tta ctt cca   2779
   Gly Glu Phe Asp Arg Cys Lys Pro Tyr Ser Tyr Pro Pro Leu Leu Pro
               840                 845                 850
   gaa tgt aac cct gag ttt ata aat cag atg caa cca tcc tct tgc cac   2827
   Glu Cys Asn Pro Glu Phe Ile Asn Gln Met Gln Pro Ser Ser Cys His
           855                 860                 865
   cac aat cag atg gtc gat tac aat aac atg aac acg agc acg agt act   2875
   His Asn Gln Met Val Asp Tyr Asn Asn Met Asn Thr Ser Thr Ser Thr
       870                 875                 880
   acc atg aat cct agc atg aat cct ccc ctt acg cct gaa ata gca tcc   2923
   Thr Met Asn Pro Ser Met Asn Pro Pro Leu Thr Pro Glu Ile Ala Ser
   885                 890                 895                 900
   agc caa agt gga ttc ggc aga aaa cat cgc aaa tgt cat caa gcg cat   2971
   Ser Gln Ser Gly Phe Gly Arg Lys His Arg Lys Cys His Gln Ala His
                   905                 910                 915
   caa ttt gag ttc cac att gat acc ggg aca atc gat ttg gtc gaa gat   3019
   Gln Phe Glu Phe His Ile Asp Thr Gly Thr Ile Asp Leu Val Glu Asp
               920                 925                 930
   ttg ggc att tgg gtg atc ttc aaa atc tgt gcc aca gat gga tac gca   3067
   Leu Gly Ile Trp Val Ile Phe Lys Ile Cys Ala Thr Asp Gly Tyr Ala
           935                 940                 945
   agc tta gat gat ctg gaa gtg att gaa gaa gga gcg ctg ggt gtc gaa   3115
   Ser Leu Asp Asp Leu Glu Val Ile Glu Glu Gly Ala Leu Gly Val Glu
       950                 955                 960
   gca tta gaa ctt gtc aaa aaa aga gaa aag aaa tgg aga cat cag aag   3163
   Ala Leu Glu Leu Val Lys Lys Arg Glu Lys Lys Trp Arg His Gln Lys
   965                 970                 975                 980
   gag cag cac tgt tcg caa acg aaa cac aaa tat gat gcg gcc aaa cat   3211
   Glu Gln His Cys Ser Gln Thr Lys His Lys Tyr Asp Ala Ala Lys His
                   985                 990                 995
   gcg gtg atg gcg tta ttt aca aac aag cgc tat gaa aaa ttg aag ttc   3259
   Ala Val Met Ala Leu Phe Thr Asn Lys Arg Tyr Glu Lys Leu Lys Phe
               1000                1005                1010
   gaa aca acc att tct gac att ttg tat gct gat cat ctc gtg cag tcg   3307
   Glu Thr Thr Ile Ser Asp Ile Leu Tyr Ala Asp His Leu Val Gln Ser
           1015                1020                1025
   att cct tat gta tat aat aaa tat gta ccg gaa gtt cca ggt atg aat  3355
   Ile Pro Tyr Val Tyr Asn Lys Tyr Val Pro Glu Val Pro Gly Met Asn
      1030                1035                1040
   tac gaa ctc tat tca gag cta aac aca ctg gtt cag aat gcg ttc tac   3403
   Tyr Glu Leu Tyr Ser Glu Leu Asn Thr Leu Val Gln Asn Ala Phe Tyr
   1045                1050                1055                1060
   ctg tat gac cag cgg aat ctg att aaa aat ggg cgc ttt agc aat ggg   3451
   Leu Tyr Asp Gln Arg Asn Leu Ile Lys Asn Gly Arg Phe Ser Asn Gly
                   1065                1070                1075
   ctt atg cat tgg caa gct act cct cat gca aga gta gag caa gaa tat   3499
   Leu Met His Trp Gln Ala Thr Pro His Ala Arg Val Glu Gln Glu Tyr
               1080                1085                1090
   gag aaa tcg gtg ctc gtg ctg cca aat tgg gat gcc aat gtg tcg caa   3547
   Glu Lys Ser Val Leu Val Leu Pro Asn Trp Asp Ala Asn Val Ser Gln
           1095                1100                1105
   gat ctt tgt atc gaa cac aat cgc ggt tat gta ttg cgt gtc acg gcg   3595
   Asp Leu Cys Ile Glu His Asn Arg Gly Tyr Val Leu Arg Val Thr Ala
       1110                1115                1120
   aga aaa gaa gat ccg gga gct ggc aat gtt acc ttt agt gac tgt gaa   3643
   Arg Lys Glu Asp Pro Gly Ala Gly Asn Val Thr Phe Ser Asp Cys Glu
   1125                1130                1135                1140
   aat cat gtc gac aag ctg agc ttt act tct tgc gat ata gct aca aac   3691
   Asn His Val Asp Lys Leu Ser Phe Thr Ser Cys Asp Ile Ala Thr Asn
                   1145                1150                1155
   gca gtg cca ggt gcc caa gcg aat gat cca gcc gcc gga gta gcc tat   3739
   Ala Val Pro Gly Ala Gln Ala Asn Asp Pro Ala Ala Gly Val Ala Tyr
               1160                1165                1170
   gga caa cag ggt tgt caa ata gat aga gtg ccg tac ggg caa tct gga   3787
   Gly Gln Gln Gly Cys Gln Ile Asp Arg Val Pro Tyr Gly Gln Ser Gly
           1175                1180                1185
   tat cga gca gac gga gta gcg tac gaa cag tct ggt cat cga aca gat  3835
   Tyr Arg Ala Asp Gly Val Ala Tyr Glu Gln Ser Gly His Arg Thr Asp
       1190                1195                1200
   gga gtg ccg tac aga caa tct gga tat gga aca gac gga gta acg tac   3883
   Gly Val Pro Tyr Arg Gln Ser Gly Tyr Gly Thr Asp Gly Val Thr Tyr
   1205                1210                1215                1220
   gaa caa tct ggt cat cga gca gat gga gtg ccg tac gga caa tct gga   3931
   Glu Gln Ser Gly His Arg Ala Asp Gly Val Pro Tyr Gly Gln Ser Gly
                   1225                1230                1235
   tat cga gca gat gga gta gcg tac gaa cag tct ggt cat cga gca gat   3979
   Tyr Arg Ala Asp Gly Val Ala Tyr Glu Gln Ser Gly His Arg Ala Asp
               1240                1245                1250
   gga gtg ccg tac gga caa tct gga tat gga aca gac gga gta acg tac   4027
   Gly Val Pro Tyr Gly Gln Ser Gly Tyr Gly Thr Asp Gly Val Thr Tyr
           1255                1260                1265
   gac caa tct gcc aat caa acc cgc aaa tat cat ggt tgc cat aca gac   4075
   Asp Gln Ser Ala Asn Gln Thr Arg Lys Tyr His Gly Cys His Thr Asp
       1270                1275                1280
   gga ctg cca cat cca gag cat ggt tgt tgt tat cca gac aga gta agc   4123
   Gly Leu Pro His Pro Glu His Gly Cys Cys Tyr Pro Asp Arg Val Ser
   1285                1290                1295                1300
   gat ggc caa cag ctt gca tat gta aca aaa tcg att gat ctg ttc ccg   4171
   Asp Gly Gln Gln Leu Ala Tyr Val Thr Lys Ser Ile Asp Leu Phe Pro
                   1305                1310                1315
   gat aca gat aaa gtc cgg atc gac att gga gaa acc gaa ggg aac ttt   4219
   Asp Thr Asp Lys Val Arg Ile Asp Ile Gly Glu Thr Glu Gly Asn Phe
               1320                1325                1330
   aga gtg gaa agt gtg gaa ttg att tgt atg gaa aag taaatcatca        4265
   Arg Val Glu Ser Val Glu Leu Ile Cys Met Glu Lys
           1335                1340
   caagtaaaag tatcgtttac taaaaattta ttttccaagc aacaggggag aagatgattt 4325
   gggtgtaata ctcaaatcat cttttcttat aagccacttt a                     4366
<210> 20
   <211> 1344
   <212> PRT
   <213> Bacillus popilliae
<400> 20
   Met Asn Gln Tyr His Asn Gln Asn Asp Asn Lys Ser Tyr Asn Gln Ser
    1               5                  10                  15
   Gly Asn Glu Met Gln Ile Ile Gln Pro Ser Ser Asn Ser Leu Leu Tyr
               20                  25                  30
   Ser Pro Asn Lys Tyr Pro Tyr Ala Thr Asp Pro Asn Val Ile Ala Glu
           35                  40                  45
   Gly Arg Ser Tyr Lys Asn Trp Leu Asp Met Cys Val Gly Glu Gly Asp
       50                  55                  60
   Gly Thr Arg Ser Leu Glu Ala Ile Ala Val Ala Val Gly Val Arg Ile
   65                  70                  75                  80
   Ser His Thr Ile Phe Arg Leu Leu Gly Val Pro Tyr Ser Ala Gln Gly
                   85                  90                  95
   Glu Gln Leu Phe Ser Phe Leu Leu Asp Thr Leu Trp Leu Glu Gly Asn
               100                 105                 110
   Thr Gln Trp Glu Glu Leu Met Arg His Ala Glu Glu Leu Ile Asn Glu
           115                 120                 125
   Gln Val Pro Asp Tyr Val Arg Thr Lys Ala Leu Ala Glu Leu Thr Asp
       130                 135                 140
   Leu Gly Asn Asn Leu Asn Leu Tyr Ile Ala Ala Phe Glu Asp Trp Lys
   145                 150                 155                 160
   Arg Asn Pro Ser Ser Gln Glu Val Arg Thr Arg Val Ile Asp Arg Phe
                   165                 170                 175
   Asn Ile Leu Asp Gly Leu Phe Glu Ala Tyr Leu Pro Ser Phe Ala Val
               180                 185                 190
   Pro Gly Tyr Glu Val Pro Leu Leu Ser Val Tyr Ala Asn Val Val Asn
           195                 200                 205
   Ile His Leu Leu Val Leu Arg Asp Ser Ser Ile Tyr Gly Leu Asp Trp
       210                 215                 220
   Gly Leu Ser Ser Thr Ser Val Asp Asn Asn Tyr Asn Arg Gln Gln Arg
   225                 230                 235                 240
   Asn Ser Ala Thr Tyr Ala Asn His Cys Thr Thr Trp Tyr Gln Thr Gly
                   245                 250                 255
   Leu Gln Arg Leu Gln Gly Ser Asp Ala Ser Ser Trp Val Asn Tyr Asn
               260                 265                 270
   Arg Phe Arg Arg Glu Ile Thr Leu Ile Val Leu Asp Ile Cys Ala Leu
           275                 280                 285
   Phe Ser Asn Tyr Asp Val Arg Ser Tyr Pro Ile Gln Leu Arg Gly Glu
       290                 295                 300
   Leu Thr Arg Gly Ile Tyr Thr Asp Pro Ala Val Tyr Ser Gly Thr Gly
   305                 310                 315                 320
   Ser Tyr Ser Trp Leu Ser Gln Ala Pro Ser Phe Ala Glu Ile Glu Asn
                   325                 330                 335
   Ile Ala Ile Arg Glu Pro Ser Asn Phe Thr Trp Ala Ser Tyr Ala Arg
               340                 345                 350
   Val Thr Thr Gly Thr Leu Glu Tyr Leu Ser Ser Lys Asn Asp Phe Trp
           355                 360                 365
   Lys Ser His Tyr Met Asn Tyr Thr Glu Thr Asn Ser Gly Ile Leu Ile
       370                 375                 380
   Gln Gly Pro Thr Tyr Gly Met Thr Thr Gly Thr Asn Ile Arg Ile Glu
   385                 390                 395                 400
   Ser Val Ser Met Gln Glu Ile Tyr Ser Val Arg Leu Glu Ala Val Ala
                   405                 410                 415
   His Ala Gly Ala Gly Gly Pro Phe Leu Gly Ile Ser Thr Ser Glu Phe
               420                 425                 430
   Phe Trp Ser Leu Gly Val Arg Arg Tyr Gln Asn Ser Arg Ser Pro Gln
           435                 440                 445
   Phe Ala Ser Gln Ile Ile Thr Arg Gln Leu Pro Gly Val Asn Ser Ala
       450                 455                 460
   Val Pro Ser Ala Leu Asp His Ser His Glu Leu Ser Tyr Ile Thr Ala
   465                 470                 475                 480
   Phe Pro Val Arg Ser Val Gly Thr Ile Leu Val His Glu Trp Thr Ser
                   485                 490                 495
   Thr Thr Val Ser Arg Asn Asn Arg Ile Glu Pro Asp Lys Ile Thr Gln
               500                 505                 510
   Ile Pro Ala Val Lys Ser His Thr Leu Ser Asn Cys Gln Val Val Ser
           515                 520                 525
   Gly Thr Gly Phe Thr Gly Gly Asn Trp Leu Arg Pro Ser Asp Asn Gly
       530                 535                 540
   Ser Phe Arg Leu Thr Ile Thr Ser Phe Ser Ser Gln Ser Tyr Arg Ile
   545                 550                 555                 560
   Arg Ile His Tyr Ala Ser Ala Thr Phe Phe Tyr Leu Asp Ile Arg Thr
                   565                 570                 575
   Gly Asp Thr Ser Asn Thr Phe Ala Val Thr Pro Thr Thr Leu Ser Ser
               580                 585                 590
   Gly Ser Gln Thr Val Pro Tyr Glu Ser Phe Gly Phe Ile Asn Ile Pro
           595                 600                 605
   Tyr Thr Phe Thr Thr Ala Pro Thr Glu Ser Arg Tyr Thr Phe Asp Phe
       610                 615                 620
   Met Phe Tyr Ser Ile Gly Ser Ala Asn Val Leu Ile Asp Arg Ile Glu
   625                 630                 635                 640
   Ile Val Pro Ile Gly Val Pro Leu Phe Glu Tyr Glu Thr Lys Gln Gln
                   645                 650                 655
   Leu Glu Lys Ala Arg Lys Ala Val Asn His Leu Phe Thr Asp Gly Ser
               660                 665                 670
   Lys Lys Ala Leu Lys Glu Asp Thr Thr Asp Tyr Glu Ile Asp Gln Ala
           675                 680                 685
   Ala Asn Val Val Asp Cys Ile Ser Asp Glu Cys Gly His Asp Lys Met
       690                 695                 700
   Ile Leu Leu Asp Glu Val Lys Tyr Ala Lys Gln Leu Ser Gln Ala Arg
   705                 710                 715                 720
   Asn Leu Leu Leu Asn Gly Asn Phe Asp Asp Leu Tyr Ser Ala Leu Glu
                   725                 730                 735
   Lys Glu Asn Pro Trp Lys Thr Ser Pro Asn Val Thr Ile Arg Gln Asp
               740                 745                 750
   Asn Pro Ile Phe Lys Gly His Tyr Leu Ser Met Ala Gly Ala Asn Asp
           755                 760                 765
   Ile Glu Ala Thr Asn Asp Thr Phe Pro Thr Tyr Val Tyr Gln Lys Ile
       770                 775                 780
   Asp Glu Ala Lys Leu Lys Pro Tyr Thr Arg Tyr Lys Val Arg Gly Phe
   785                 790                 795                 800
   Val Gly Ser Ser Lys Ala Leu Glu Leu Leu Val Thr Arg Tyr Asn Glu
                   805                 810                 815
   Glu Val Asp Ala Ile Leu Asp Val Pro Asp Asn Ile Pro His Ala Pro
               820                 825                 830
   Thr Pro Val Cys Gly Glu Phe Asp Arg Cys Lys Pro Tyr Ser Tyr Pro
           835                 840                 845
   Pro Leu Leu Pro Glu Cys Asn Pro Glu Phe Ile Asn Gln Met Gln Pro
       850                 855                 860
   Ser Ser Cys His His Asn Gln Met Val Asp Tyr Asn Asn Met Asn Thr
   865                 870                 875                 880
   Ser Thr Ser Thr Thr Met Asn Pro Ser Met Asn Pro Pro Leu Thr Pro
                   885                 890                 895
   Glu Ile Ala Ser Ser Gln Ser Gly Phe Gly Arg Lys His Arg Lys Cys
               900                 905                 910
   His Gln Ala His Gln Phe Glu Phe His Ile Asp Thr Gly Thr Ile Asp
           915                 920                 925
   Leu Val Glu Asp Leu Gly Ile Trp Val Ile Phe Lys Ile Cys Ala Thr
       930                 935                 940
   Asp Gly Tyr Ala Ser Leu Asp Asp Leu Glu Val Ile Glu Glu Gly Ala
   945                 950                 955                 960
   Leu Gly Val Glu Ala Leu Glu Leu Val Lys Lys Arg Glu Lys Lys Trp
                   965                 970                 975
   Arg His Gln Lys Glu Gln His Cys Ser Gln Thr Lys His Lys Tyr Asp
               980                 985                 990
   Ala Ala Lys His Ala Val Met Ala Leu Phe Thr Asn Lys Arg Tyr Glu
           995                 1000                1005
   Lys Leu Lys Phe Glu Thr Thr Ile Ser Asp Ile Leu Tyr Ala Asp His
      1010                1015                1020
   Leu Val Gln Ser Ile Pro Tyr Val Tyr Asn Lys Tyr Val Pro Glu Val
   1025                1030                1035                1040
   Pro Gly Met Asn Tyr Glu Leu Tyr Ser Glu Leu Asn Thr Leu Val Gln
                   1045                1050                1055
   Asn Ala Phe Tyr Leu Tyr Asp Gln Arg Asn Leu Ile Lys Asn Gly Arg
               1060                1065                1070
   Phe Ser Asn Gly Leu Met His Trp Gln Ala Thr Pro His Ala Arg Val
           1075                1080                1085
   Glu Gln Glu Tyr Glu Lys Ser Val Leu Val Leu Pro Asn Trp Asp Ala
      1090                1095                1100
   Asn Val Ser Gln Asp Leu Cys Ile Glu His Asn Arg Gly Tyr Val Leu
   1105                1110                1115                1120
   Arg Val Thr Ala Arg Lys Glu Asp Pro Gly Ala Gly Asn Val Thr Phe
                   1125                1130                1135
   Ser Asp Cys Glu Asn His Val Asp Lys Leu Ser Phe Thr Ser Cys Asp
               1140                1145                1150
   Ile Ala Thr Asn Ala Val Pro Gly Ala Gln Ala Asn Asp Pro Ala Ala
           1155                1160                1165
   Gly Val Ala Tyr Gly Gln Gln Gly Cys Gln Ile Asp Arg Val Pro Tyr
      1170                1175                1180
   Gly Gln Ser Gly Tyr Arg Ala Asp Gly Val Ala Tyr Glu Gln Ser Gly
   1185                1190                1195                1200
   His Arg Thr Asp Gly Val Pro Tyr Arg Gln Ser Gly Tyr Gly Thr Asp
                   1205                1210                1215
   Gly Val Thr Tyr Glu Gln Ser Gly His Arg Ala Asp Gly Val Pro Tyr
               1220                1225                1230
   Gly Gln Ser Gly Tyr Arg Ala Asp Gly Val Ala Tyr Glu Gln Ser Gly
           1235                1240                1245
   His Arg Ala Asp Gly Val Pro Tyr Gly Gln Ser Gly Tyr Gly Thr Asp
      1250                1255                1260
   Gly Val Thr Tyr Asp Gln Ser Ala Asn Gln Thr Arg Lys Tyr His Gly
   1265                1270                1275                1280
   Cys His Thr Asp Gly Leu Pro His Pro Glu His Gly Cys Cys Tyr Pro
                   1285                1290                1295
   Asp Arg Val Ser Asp Gly Gln Gln Leu Ala Tyr Val Thr Lys Ser Ile
               1300                1305                1310
   Asp Leu Phe Pro Asp Thr Asp Lys Val Arg Ile Asp Ile Gly Glu Thr
           1315                1320                1325
   Glu Gly Asn Phe Arg Val Glu Ser Val Glu Leu Ile Cys Met Glu Lys
      1330                1335                1340
<210> 21
   <211> 34
   <212> DNA
   <213> Artificial Sequence
<220>
   <223> Description of Artificial Sequence: synthetic oligonucleotide
<400> 21
   ctggaattca tgaatcagta tcataaccaa aacg 34
<210> 22
   <211> 34
   <212> DNA
   <213> Artificial Sequence
<220>
<223> Description of Artificial Sequence: synthetic oligonucleotide
<400> 22
   gagctgcagt tacttttcca tacaaatcaa ttcc 34